# EUROPEAN PATENT APPLICATION

(11) **EP 2 145 886 A1**
(43) Date of publication of application: **20.01.2010**
(21) Application number: 08740270.7
(22) Date of filing: 11.04.2008
(51) Int. Cl.: C07D 405/06, A61K 31/395, A61P 35/00, A61P 35/02

(54) **TWELVE-MEMBERED CYCLOMACROLACTAM DERIVATIVE**

(30) Priority: 12.04.2007 US 911410 P; 12.12.2007 JP 2007321169
(71) Applicant: Eisai R&D Management Co., Ltd., Tokyo 112-8088 (JP)
(72) Inventor: MIYANO, Masayuki, Tsukuba-shi Ibaraki 300-2635 (JP); ITO, Daisuke, Tsukuba-shi Ibaraki 300-2635 (JP); MURAI, Norio, Tsukuba-shi Ibaraki 300-2635 (JP)
(74) Representative: HOFFMANN EITLE
(86) International application number: PCT/JP2008/057173
(87) International publication number: WO 2008/126918

(57) **Abstract**

There provided a 12-membered-ring macrolactam derivative having antitumor activity: A compound represented by Formula (1) or a salt thereof. In this Formula, R₁ is a hydrogen atom, a C₁₋₆ alkyl group, a C₁₋₆ alkylcarbonyl group or a C₆₋₁₄ arylcarbonyl group; R₂ is a hydrogen atom or a C₁₋₆ alkyl group; R₃ is a hydrogen atom or a hydroxyl group; R₄ is a hydrogen atom or a hydroxyl group; R₅ is a hydrogen atom or a C₁₋₆ alkyl group; R₆ is a hydrogen atom or a hydroxyl group; and R₇ is an acetyl group or the like.

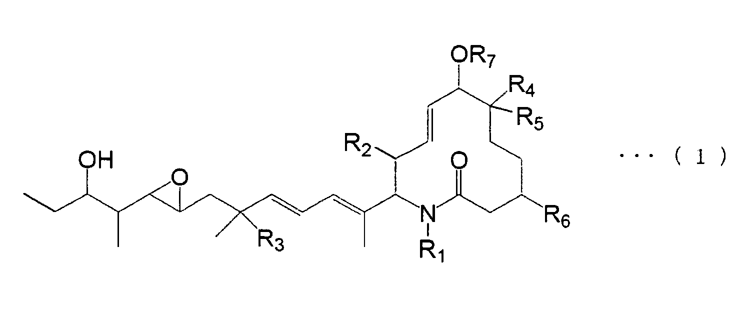

## Description

### TECHNICAL FIELD

The present invention relates to a 12-membered-ring macrolactam derivative, particularly to a 12-membered-ring macrolactam derivative having antitumor activities.

### BACKGROUND ART

Pladienolide compounds, such as pladienolide B represented by Formula (1'"): 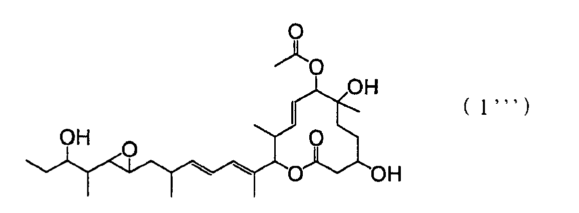 , and pladienolide D represented by Formula (2"'): 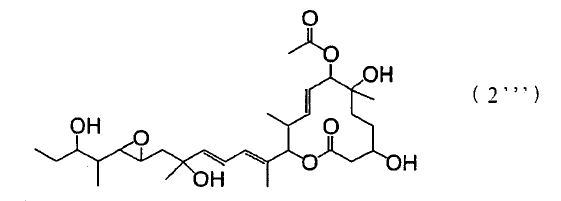 have been known. These compounds are 12-membered ring macrolide compounds found in a culture of *Streptomyces sp.* Mer-11107 strain by Sakai *et al,* which are known to have an excellent antitumor activity. For example, see Pamphlet of International Publication No. 2002/60890 (Patent Document 1). In addition, JP-A No. 4-352783 (Patent Document 2) and Pamphlets of International Publication Nos. 2003/099813, 2004/011459, 2004/011661, 2007/110704 and 2007/110705 (Patent Document 3-7) disclosed various compounds having a similar structure to pladienolide B or pladienolide D. Furthermore, Pamphlet of International Publication No. 2007/043621 (Patent Document 8) disclosed a method of synthesizing pladienolide B and pladienolide D.
Patent Document 1: Pamphlet of International Publication No.2002/60890
Patent Document 2: JP-ANo. 4-352783
Patent Document 3: Pamphlets of International Publication No.2003/099813
Patent Document 4: Pamphlets of International Publication No.2004/011459
Patent Document 5: Pamphlets of International Publication No.2004/011661
Patent Document 6: Pamphlets of International Publication No.2007/110704
Patent Document 7: Pamphlets of International Publication No.2007/110705
Patent Document 8: Pamphlets of International Publication No.2007/043621

### DISCLOSURE OF INVENTION

However, JP-ANo. 4-352783 and Pamphlets of International Publication Nos. 2003/099813, 2004/011459, 2004/011661 and 2007/043621 disclosed only compounds in which the ring moiety is 12-membered macrolactone, and did not disclose compounds in which the ring moiety is not 12-membered macrolactone and which are not obtainable from a fermentation and their synthetic method.

On the other hand, in Pamphlet of International Publication No. 2007/110704, although a compound in which the ring moiety is 12-membered macrolactam or the like is described as a general description, although Example corresponding to such a compound is not described. Furthermore, in Pamphlet of International Publication No. 2007/110705, a compound in which the ring moiety is 12-membered macrolactam or the like is described.

An object of the present invention is to provide 12-membered-ring macrolactam derivatives having antitumor activity and synthesis intermediates thereof.

The present inventors have carried out extensive studies to solve the above-mentioned problems. As a result, they have succeeded in synthesizing useful 12-membered-ring macrolactam derivatives in which the ring moiety is 12-membered macrolactam and also discovered that the 12-membered-ring macrolactam derivatives have antitumor activity. Thus, they arrived at the present invention. As mentioned above, a 12-membered macrolactam ring compound is described in Pamphlet of International Publication No. 2007/110705, but a structure including a side chain at the 11th position, of the compound described in the literature is absolutely different from that of the compound of the invention of the present application.

That is, the present invention provides:
(1) a compound represented by the following Formula (1) or a salt thereof: 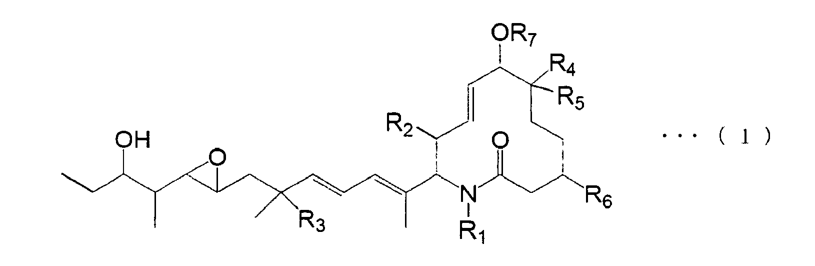 wherein R₁ is a hydrogen atom, a C₁₋₆ alkyl group, a C₁₋₆ alkylcarbonyl group or a C₆₋₁₄ arylcarbonyl group; R₂ is a hydrogen atom or a C₁₋₆ alkyl group; R₃ is a hydrogen atom or a hydroxyl group; R₄ is a hydrogen atom or a hydroxyl group; R₅ is a hydrogen atom or a C₁₋₆ alkyl group; R₆ is a hydrogen atom or a hydroxyl group; and R₇ is R^{a}C(=Y)-, wherein Y is an oxygen atom or a sulfur atom, and R^{a} is:
   a) a C₁₋₂₂ alkyl group optionally having a substituent(s);
   b) an unsaturated C₂₋₂₂ alkyl group optionally having a substituent(s);
   c) a C₆₋₁₄ aryl group optionally having a substituent(s);
   d) a 5 to 14-membered ring heteroaryl group optionally having a substituent(s);
   e) a C₇₋₂₂ aralkyl group optionally having a substituent(s);
   f) a 5 to 14-membered ring heteroaralkyl group optionally having a substituent(s);
   g) a C₁₋₂₂ alkoxy group optionally having a substituent(s);
   h) an unsaturated C₂₋₂₂ alkoxy group optionally having a substituent(s);
   i) a C₆₋₁₄ aryloxy group optionally having a substituent(s);
   j) a 5 to 14-membered ring heteroaryloxy group optionally having a substituent(s); or
   k) R^{N1}R^{N2}N- optionally having a substituent(s), wherein R^{N1} and R^{N2} may be the same or different from each other and are each:
      1) a hydrogen atom;
      2) a C₁₋₂₂ alkyl group optionally having a substituent(s);
      3) an unsaturated C₂₋₂₂ alkyl group optionally having a substituent(s);
      4) an aliphatic C₂₋₂₂ acyl group optionally having a substituent(s);
      5) an aromatic C₇₋₁₅ acyl group optionally having a substituent(s);
      6) a C₆₋₁₄ aryl group optionally having a substituent(s);
      7) a 5 to 14-membered ring heteroaryl group optionally having a substituent(s);
      8) a C₇₋₂₂ aralkyl group optionally having a substituent(s);
      9) a 3 to 14-membered ring non-aromatic heterocyclic group formed by R^{N1}, R^{N2} and the nitrogen atom to which R^{N1} and R^{N2} are bonded, wherein the non-aromatic heterocyclic group optionally has a substituent(s);
      10) a 5 to 14-membered ring heteroaralkyl group optionally having a substituent(s);
      11) a C₃₋₁₄ cycloalkyl group optionally having a substituent(s); or
      12) a 3 to 14-membered ring non-aromatic heterocyclic group optionally having a substituent(s);
(2) a compound represented by the following Formula (2) or a salt thereof: 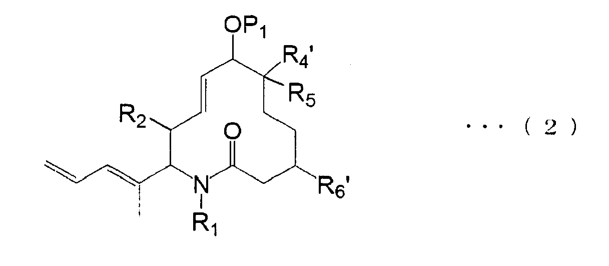 wherein R₁ is a hydrogen atom, a C₁₋₆ alkyl group, a C₁₋₆ alkylcarbonyl group or a C₆₋₁₄ arylcarbonyl group; R₂ is a hydrogen atom or a C₁₋₆ alkyl group; R₄' is a hydrogen atom, a hydroxyl group or an O-protecting group; R₅ is a hydrogen atom or a C₁₋₆ alkyl group; R₆' is a hydrogen atom, a hydroxyl group or an O-protecting group; and P₁ is a hydrogen atom or a protecting group: or R₄' and OP₁ may together represent the following formula: 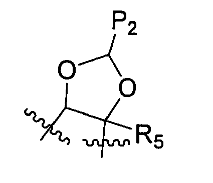 wherein P₂ is a phenyl group or a C₁₋₆ alkyl group;
(3) a compound represented by the following Formula (3) or a salt thereof: 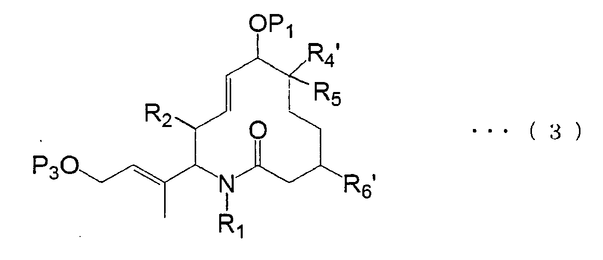 wherein R₁ is a hydrogen atom, a C₁₋₆ alkyl group, a C₁₋₆ alkylcarbonyl group, or a C₆₋₁₄ arylcarbonyl group; R₂ is a hydrogen atom or a C₁₋₆ alkyl group; R₄' is a hydrogen atom, a hydroxyl group, or an O-protecting group; R₅ is a hydrogen atom or a C₁₋₆ alkyl group; R₆' is a hydrogen atom, a hydroxyl group, or an O-protecting group; P₁ is a hydrogen atom or a protecting group; and P₃ is a hydrogen atom or a protecting group: or R₄' and OP₁ may together represent the following formula: 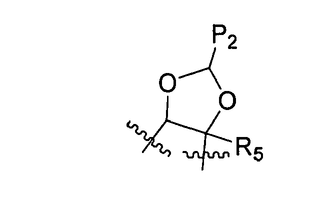 wherein P₂ is a phenyl group or a C₁₋₆ alkyl group;
(4) a compound represented by the following Formula (4-1) or a salt thereof: 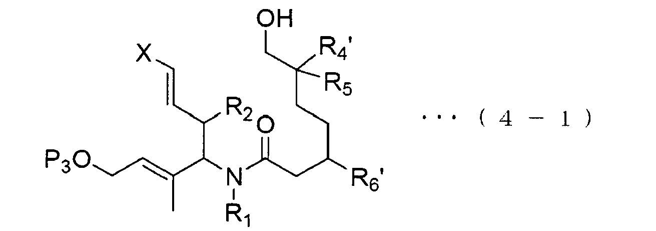 wherein R₁ is a hydrogen atom, a C₁₋₆ alkyl group, a C₁₋₆ alkylcarbonyl group or a C₆₋₁₄ arylcarbonyl group; R₂ is a hydrogen atom or a C₁₋₆ alkyl group; R₄' is a hydrogen atom, a hydroxyl group or an O-protecting group; R₅ is a hydrogen atom or a C₁₋₆ alkyl group; R₆' is a hydrogen atom, a hydroxyl group or an O-protecting group; P₃ is a hydrogen atom or a protecting group; and X is halogen;
(5) a compound represented by the following Formula (4-2) or a salt thereof: 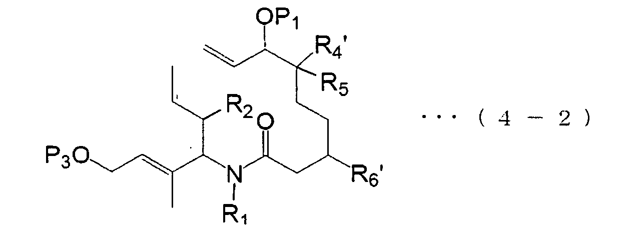 wherein R₁ is a hydrogen atom, a C₁₋₆ alkyl group, a C₁₋₆ alkylcarbonyl group or a C₆₋₁₄ arylcarbonyl group; R₂ is a hydrogen atom or a C₁₋₆ alkyl group; R₄' is a hydrogen atom, a hydroxyl group or an O-protecting group; R₅ is a hydrogen atom or a C₁₋₆ alkyl group; R₆' is a hydrogen atom, a hydroxyl group or an O-protecting group; P1 is a hydrogen atom or a protecting group; and P₃ is a hydrogen atom or a protecting group: or R₄' and OP₁ may together represent the following formula: 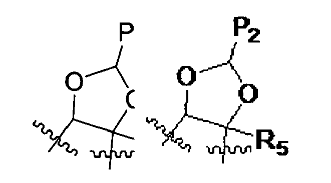 wherein P₂ is a phenyl group or a C₁₋₆ alkyl group;
(6) the compound of Formula (1) described in (1) or a salt thereof, wherein R₇ is R^{a}'C(=Y)- wherein Y is an oxygen atom or a sulfur atom and R^{a}' is a C₁₋₂₂ alkyl group optionally having a substituent(s);
(7) the compound described in (6) or a salt thereof, wherein Y is an oxygen atom;
(8) the compound described in (7) or a salt thereof, wherein R₇ is an acetyl group;
(9) the compound described in any of (1) or (6) to (8) or a salt thereof, wherein R₄, R₅ and R₆ are each a hydrogen atom;
(10) the compound described in any of (1) or (6) to (8) or a salt thereof, wherein R₄ is a hydroxyl group, R₅ is a hydrogen atom, a methyl group or an ethyl group, and R₆ is a hydroxyl group;
(11) the compound described in (10) or a salt thereof, wherein R₅ is a methyl group;
(12) the compound described in any of (1) or (6) to (11) or a salt thereof, wherein R₁ is selected from the group consisting of a hydrogen atom and a C₁₋₃ alkyl group;
(13) the compound described in (12) or a salt thereof, wherein R₁ is a hydrogen atom;
(14) the compound described in (12) or a salt thereof, wherein R₁ is a methyl group;
(15) the compound described in any of (1) or (6) to (14) or a salt thereof, wherein R₂ is selected from the group consisting of a hydrogen atom and a C₁₋₃ alkyl group;
(16) the compound described in (15) or a salt thereof, wherein R₂ is a hydrogen atom;
(17) the compound described in (15) or a salt thereof, wherein R₂ is a methyl group;
(18) the compound described in any of (1) or (6) to (17) or a salt thereof, wherein R₃ is a hydrogen atom;
(19) the compound described in any of (1) or (6) to (17) or a salt thereof, wherein R₃ is a hydroxyl group;
(20) the compound described in any of (2) to (5) or a salt thereof, wherein R₄', R₅ and R₆' are each a hydrogen atom;
(21) the compound described in any of (2) to (5) or a salt thereof, wherein R₄' is a hydroxyl group, R₅ is a hydrogen atom, a methyl group or an ethyl group; and R₆' is a hydroxyl group or an O-protecting group;
(22) the compound described in (21) or a salt thereof, wherein R₅ is a methyl group;
(23) the compound described in any of (2) to (5) or (20) to (22) or a salt thereof, wherein R₁ is selected from the group consisting of a hydrogen atom and a C₁₋₃ alkyl group;
(24) the compound described in (23) or a salt thereof, wherein R₁ is a hydrogen atom;
(25) the compound described in (23) or a salt thereof, wherein R₁ is a methyl group;
(26) the compound described in any of (2) to (5) or (20) to (25) or a salt thereof, wherein R₂ is selected from the group consisting of a hydrogen atom and a C₁₋₃ alkyl group;
(27) the compound described in (26) or a salt thereof, wherein R₂ is a hydrogen atom;
(28) the compound described in (26) or a salt thereof, wherein R₂ is a methyl group;
(29) a medicament containing at least one compound selected from the compounds described in (1) or (6) to (19) or a salt thereof as an active component;
(30) the medicament described in (29), which is an antitumor agent;
(31) the medicament described in (30), which is an agent for treating solid tumor;
(32) the medicament described in (31), wherein the agent for treating solid tumor is an agent for treating lung cancer, brain tumor, breast cancer, prostate cancer, ovarian cancer, colon cancer or skin cancer;
(33) the medicament described in (30), which is an agent for treating leukemia;
(34) a use of at least one compound selected from the compounds described in (1) or (6) to (19) or salts thereof, for production of a medicament;
(35) the use described in (34), wherein the medicament is an antitumor agent;
(36) the use described in (35), wherein the antitumor agent is an agent for treating solid tumor;
(37) the use described in (36), wherein the agent for treating solid tumor is an agent for treating lung cancer, brain tumor, breast cancer, prostate cancer, ovarian cancer, colon cancer or skin cancer;
(38) the use described in (35), wherein the medicament is an agent for treating leukemia;
(39) the compound described in any of (1) or (6) to (19) or a salt thereof, for treating a tumor;
(40) the compound described in (39) or a salt thereof, wherein the tumor is a solid tumor;
(41) the compound described in (40) or a salt thereof, wherein the solid tumor is lung cancer, brain tumor, breast cancer, prostate cancer, ovarian cancer, colon cancer or skin cancer.
(42) the compound described in (39) or a salt thereof, wherein the tumor is leukemia;
(43) a method of treating a tumor, which includes the step of administering to a patient an effective amount of the compound described in any of (1) or (6) to (19) or a salt thereof;
(44) the method described in (43), wherein the tumor is a solid tumor;
(45) the method described in (44), wherein the solid tumor is lung cancer, brain tumor, breast cancer, prostate cancer, ovarian cancer, colon cancer or skin cancer; or
(46) the method described in (43), wherein the tumor is leukemia.

According to the present invention, a compound which is a novel 12-membered-ring macrolactam derivative having antitumor activity or a salt thereof can be provided. Furthermore, according to the use of an intermediate of the present invention or a salt thereof, the compound which is a 12-membered-ring macrolactam derivative of the present invention or a salt thereof can be produced.

### BEST MODE FOR CARRYING OUT THE INVENTION

Hereinafter, the meaning of terms, symbols and the like described in this specification will be explained, and the present invention will be described in detail.

The compound of the present invention or salts thereof may be any of anhydrides, hydrates and solvates. Herein, the solvate of the compound of Formula (1) refers to a compound in which solvent molecules are solvated in a non-solvated compound of Formula (1). In particular, number of solvent molecules is not limited.

In this specification, compounds represented by each Formula are shown in planar chemical formulae for convenience, but they may also include certain isomers that may be derived from chemical formulae. Specifically, the invention may include all of structurally existing isomers and mixtures of the isomers, such as geometric isomers, and stereo isomers, tautomers, optical isomers based on asymmetric carbons, of the compound.

The 'salt' used in this specification is not particularly limited as long as it can form a salt with the compound of the invention. Examples include salts of inorganic bases and the like. Among the salts, pharmacologically acceptable salts are preferable. Preferred examples of the salts of inorganic bases include alkali metal salts such as lithium salts, sodium salts and potassium salts; alkaline earth metal salts such as calcium salts and magnesium salts; aluminum salts; ammonium salts; and the like.

The term "C₁₋₆ alkyl group" in the present specification refers to a linear or branched alkyl group having 1 to 6 carbon atom(s). Specific examples thereof include a methyl group, an ethyl group, a 1-propyl group (n-propyl group), a 2-propyl group (i-propyl group), a 2-methyl-1-propyl group (i-butyl group), a 2-methyl-2-propyl group (t-butyl group), a 1-butyl group (n-butyl group), a 2-butyl group (s-butyl group), a 1-pentyl group, a 2-pentyl group, a 3-pentyl group, a 2-methyl-1-butyl group, a 3-methyl-1-butyl group, a 2-methyl-2-butyl group, a 3-methyl-2-butyl group, a 2,2-dimethyl-1-propyl group, a 1-hexyl group, a 2-hexyl group, a 3-hexyl group, a 2-methyl-1-pentyl group, a 3-methyl-1-pentyl group, a 4-methyl-1-pentyl group, a 2-methyl-2-pentyl group, a 3-methyl-2-pentyl group, a 4-methyl-2-pentyl group, a 2-methyl-3-pentyl group, a 3-methyl-3-pentyl group, a 2,3-dimethyl-1-butyl group, a 3,3-dimethyl-1-butyl group, a 2,2-dimethyl-1-butyl group, a 2-ethyl-1-butyl group, a 3,3-dimethyl-2-butyl group, a 2,3-dimethyl-2-butyl group and the like. In particular, for R₁, R₂, R₅ and R₆, the "C₁₋₆ alkyl group" is preferably a methyl group.

The term "C₁₋₆ alkylcarbonyl group" in the present specification refers to a carbonyl group to which the "C₁₋₆ alkyl group" defined as above is bonded. Specific examples thereof include an acetyl group, a propionyl group, an isopropionyl group, a butylyl group, an isobutylyl group, a valeryl group, an isovaleryl group, a pivaloyl group and the like.

The term "C₆₋₁₄ arylcarbonyl group" in the present specification refers to a carbonyl group to which the "C₆₋₁₄ aryl group" is bonded. Specific examples thereof include a benzoyl group optionally having a substituent(s) and the like. Herein, specific examples of the "substituent" include a chlorine atom, a bromine atom, an iodine atom, NO₂ and the like.

The term "C₁₋₂₂ alkyl group" in the present specification refers to a linear or branched alkyl group having 1 to 22 carbon atom(s). Specific examples thereof include a 2-methylpentyl group, a 3-methylpentyl group, a n-heptyl group, a n-octyl group, a n-nonyl group, a n-decyl group and the like, in addition to the above-described examples of the C₁₋₆ alkyl group. Among these, preferred examples are a methyl group, an ethyl group, a n-propyl group, an iso-propyl group, a n-butyl group, an iso-butyl group, a sec-butyl group, a tert-butyl group and the like.

The term "unsaturated C₂₋₂₂ alkyl group" in the present specification refers to a linear or branched alkenyl group having 2 to 22 carbon atoms, or a linear or branched alkynyl group having 2 to 22 carbon atoms. Specific examples thereof include a vinyl group, an allyl group, a 1-propenyl group, a 2-propenyl group, an isopropenyl group, a 2-methyl-1-propenyl group, a 2-methyl-2-propenyl group, a 1-butenyl group, a 2-butenyl group, a 3-butenyl group, a 1-pentenyl group, a 1-hexenyl group, a 1,3-hexanedienyl group, a 1,5-hexanedienyl group, an ethynyl group, a 1-propynyl group, a 2-propynyl group, a 1-butynyl group, a 2-butynyl group, a 3-butynyl group, a 1-ethynyl-2-propynyl group, a 2-methyl-3-butypynyl group, a 1-pentynyl group, a 1-hexynyl group, a 1,3-hexanediyneyl group, a 1,5-hexanediyneyl group and the like. Among these, preferred examples include a vinyl group, an allyl group, a 1-propenyl group, a 2-propenyl group, an isopropenyl group, an ethynyl group, a 1-propynyl group, a 2-propynyl group, a 1-butynyl group, a 2-butynyl group, a 3-butynyl group and the like.

The term "C₆₋₁₄ aryl group" in the present specification refers to an aromatic hydrocarbon cyclic group composed of 6 to 14 carbon atoms, which also includes a monocyclic group or a condensed ring, such as a bicyclic group or a tricyclic group. Specific examples thereof include a phenyl group, an indenyl group, a 1-naphthyl group, a 2-naphthyl group, an azulenyl group, a heptalenyl group, an indacenyl group, an acenaphthyl group, a fluorenyl group, a phenalenyl group, a phenanthrenyl group, an anthracenyl group and the like. Among these, preferred examples are a phenyl group, an 1-naphthyl group, a 2-naphthyl group and the like.

The term "5 to 14-membered ring heteroaryl group" in the present specification refers to a monocyclic, bicyclic or tricyclic 5 to 14-membered aromatic heterocyclic group having at least one heteroatom selected from the group consisting of a nitrogen atom, a sulfur atom and an oxygen atom. Specific examples thereof include nitrogen-containing aromatic heterocyclic rings such as a pyrrolyl group, a pyridyl group, a pyridazinyl group, a pyrimidinyl group, a pyrazinyl group, a triazolyl group, a tetrazolyl group, a benzotriazolyl group, a pyrazolyl group, an imidazolyl group, a benzimidazolyl group, an indolyl group, an isoindolyl group, an indolizinyl group, a purinyl group, an indazolyl group, a quinolyl group, an isoquinolyl group, a quinolidyl group, a phthalazyl group, a naphthyridinyl group, a quinoxalyl group, a quinazolyl group, a cinnolinyl group, a pteridinyl group, an imidazotriazinyl group, a pyrazinopyridazinyl group, an acridinyl group, a phenanthridinyl group, a carbazolyl group, a carbazolinyl group, a perimidinyl group, a phenanthrolinyl group, a phenacinyl group and an imidazopyridinyl group; sulfur-containing aromatic heterocyclic rings such as a thienyl group and a benzothienyl group; oxygen-containing aromatic heterocyclic rings such as a furyl group, a pyranyl group, a cyclopentapyranyl group, a benzofuryl group and an isobenzofuryl group; and aromatic heterocyclic rings containing two or more different kinds of hetero atoms such as a thiazolyl group, an isothiazolyl group, a furazanyl group, a phenoxazinyl group, an oxazolyl group, an isoxazolyl group, an isoxazoyl group, a benzoxazolyl group, an oxadiazolyl group, a pyrazolooxazolyl group, an imidazothiazolyl group, a thienofuranyl group, a furopyrrolyl group or a pyridoxazinyl group. Among these, preferred examples are a thienyl group, a furyl group, a pyridyl group, a pyridazyl group, a pyrimidyl group, a pyrazyl group and the like.

The term "C₇₋₂₂ aralkyl group" in the present specification refers to a group obtained when a substitutable moiety in the "C₁₋₂₂ alkyl group" is substituted with the "C₆₋₁₄ aryl group". Specific examples thereof include a benzyl group, a phenethyl group, a 3-phenylpropyl group, a 4-phenylbutyl group, a 1-naphthylmethyl group, a 2-naphthylmethyl group and the like. Among these, preferred examples are a benzyl group, a phenethyl group and the like.

The term "5 to 14-membered ring heteroaralkyl group" in the present specification refers to a group obtained when a substitutable moiety in the "C₁₋₂₂ alkyl group" is substituted with the "5 to 14-membered ring heteroaryl group". Specific examples thereof include a thienylmethyl group, a furylmethyl group, a pyridylmethyl group, a pyridazylmethyl group, a pyrimidylmethyloxy group, a pyrazylmethyl group and the like.

The term "C₁₋₂₂ alkoxy group" in the present specification refers to a group obtained when an oxygen atom is bonded to the terminal of the "C₁₋₂₂ alkyl group". Specific examples thereof include a methoxy group, an ethoxy group, a n-propoxy group, an iso-propoxy group, a sec-propoxy group, a n-butoxy group, an iso-butoxy group, a sec-butoxy group, a tert-butoxy group, a n-pentyloxy group, an iso-pentyloxy group, a sec-pentyloxy group, a n-hexoxy group, an iso-hexoxy group, a 1,1-dimethylpropyloxy group, a 1,2-dimethylpropoxy group, a 2,2- dimethylpropyloxy group, a 1-methyl-ethylpropoxy group, an 1-ethyl-methylpropoxy group, a 1,1,2-trimethylpropoxy group, a 1,2,2-trimethylpropoxy group, a 1,1-dimethylbutoxy group, a 1,2-dimethylbutoxy group, a 2,2-dimethylbutoxy group, a 2,3-dimethylbutyloxy group, a 1,3-dimethylbutoxy group, a 2-ethylbutoxy group, a 2-ethylpentoxy group, a 3-ethylpentoxy group, a hexyloxy group and the like. Among these, preferred examples are a methoxy group, an ethoxy group, a n-propoxy group, an iso-propoxy group, a sec-propoxy group, a n-butoxy group, an iso-butoxy group, a tert-butoxy group and the like.

The term "unsaturated C₂₋₂₂ alkoxy group" in the present specification refers to a group obtained when an oxygen atom is bonded to the terminal of the "unsaturated C₂₋₂₂ alkoxy group". Specific examples thereof include a vinyloxy group, an allyloxy group, a 1-propenyloxy group, a 2-propenyloxy group, a isopropenyloxy group, a 1-butenyloxy group, an ethynyloxy group, a 2-methyl-1-propenyloxy group, a 2-methyl-2-propenyloxy group, a 1-butytenyloxy group, a 2- butenyloxy group, a 3-butenyloxy group, a 1-pentenyloxy group, a 1-hexenyloxy group, a 1,3-hexanedienyloxy group, a 1,5-hexanedienyloxy group, a propalgyloxy group, a 1-butynyloxy group, a 2-butynyloxy group and the like. Among these, preferred examples are a vinyloxy group, an allyloxy group, a 1-propenyloxy group, a 2-propenyloxy group, an isopropenyloxy group, an ethynyloxy group, a 1-butynyloxy group, a 2-butynyloxy group and the like.

The term "C₆₋₁₄ aryloxy group" in the present specification refers to a group obtained when an oxygen atom is bonded to the terminal of the "C₆₋₁₄ aryl group". Specific examples thereof include a phenyloxy group, an indenyloxy group, a 1-naphthyloxy group, a 2-naphthyloxy group, an azulenyloxy group, a heptalenyloxy group, an indacenyloxy group, an acenaphthyloxy group, a fluorenyloxy group, a phenalenyloxy group, a phenanthrenyloxy group, an anthracenyloxy group and the like. Among these, preferred examples are a phenyloxy group, a 1-naphthyloxy group, a 2-naphthyloxy group and the like.

The term "5 to 14-membered ring heteroaryloxy group" in the present specification refers to a group obtained when an oxygen atom is bonded to the terminal of the "5 to 14-membered ring heteroaryl group". Specific examples thereof include a pyrrolyloxy group, a pyridyloxy group, a pyridazinyloxy group, a pyrimidinyloxy group, a pyrazinyloxy group, a triazolyloxy group, a tetrazolyloxy group, a benzotriazolyloxy group, a pyrazolyloxy group, an imidazolyloxy group, a benzimidazolyloxy group, an indolyloxy group, an isoindolyloxy group, an indolizinyloxy group, a purinyloxy group, an indazolyloxy group, a quinolyloxy group, an isoquinolyloxy group, a quinolizyloxy group, a phthalazyloxy group, a naphthyridinyloxy group, a quinoxalyloxy group, a quinazolyloxy group, a cinnolinyloxy group, a pteridinyloxy group, an imidazotriazinyloxy group, a pyrazinopyridazinyloxy group, an acridinyloxy group, a phenanthridinyloxy group, a carbazolyloxy group, a carbazolinyloxy group, a perimidinyloxy group, a phenanthrolinyloxy group, a phenacinyloxy group, an imidazopyridinyloxy group, a thienyloxy group, a benzothienyloxy group, a furyloxy group, a pyranyloxy group, a cyclopentapyranyloxy group, a benzofuryloxy group, an isobenzofuryloxy group, a thiazolyloxy group, an isothiazolyloxy group, a furazanyloxy group, a phenoxazinyloxy group, an oxazolyloxy group, an isoxazolyloxy group, an isoxazoyloxy group, a benzoxazolyloxy group, an oxadiazolyloxy group, a pyrazolooxazolyloxy group, an imidazothiazolyloxy group, a thienofuranyloxy group, a furopyrrolyloxy group, a pyridoxazinyloxy group and the like. Among these, preferred examples are a thienyloxy group, a furyloxy group, a pyridyloxy group, a pyridazyloxy group, a pyrimidyloxy group, a pyrazyloxy group and the like.

The term "aliphatic C₂₋₂₂ acyl group" in the present specification refers to a group obtained when a carbonyl group is bonded to the terminal of the "C₁₋₂₂ alkyl group" or the "unsaturated C₂₋₂₂ alkyl group". Specific examples thereof include an acetyl group, a propionyl group, a butyryl group, an iso-butyryl group, a valeryl group, an iso-valeryl group, a pivalyl group, a caproyl group, a decanoyl group, a lauroyl group, a myristoyl group, a palmitoyl group, a stearoyl group, an arachidoyl group, an acryl group, a propyol group, a crotonyl group, an iso-crotonyl group, an oleinol group, a linolenoyl group and the like.

The term "aromatic C₇₋₁₅ acyl group" in the present specification refers to a group obtained when a carbonyl group is bonded to the terminal of the "C₆₋₁₄aryl group" and "5 to 14-membered ring heteroaryl group". Specific examples thereof include a benzoyl group, a 1-naphthoyl group, a 2-naphthoyl group, a picolinoyl group, a nicotinoyl group, an isonicotinoyl group, a furoyl group, a thiophenecarbonyl group and the like. Among these, preferred examples are a benzoyl group, a 1-naphthoyl group, 2-naphthoyl group and the like.

Examples of the "3 to 14-membered non-aromatic heterocyclic group" in the present specification include an aziridinyl group, an azetidyl group, a pyrrolidinyl group, a pyrrolyl group, a piperidinyl group, a piperazinyl group, a homopiperidyl group, a homopiperazyl group, an imidazolyl group, a pyrazolidyl group, an imidazolidyl group, a morpholyl group, a thiomorpholyl group, an imidazolinyl group, an oxazolinyl group, a quinuclidyl group and the like.

The term "C₃₋₁₄ cycloalkyl group" used in the present specification refers to a cycloalkyl group having 3 to 14 carbon atoms. Preferred examples of the group include a cyclopropyl group, a cyclobutyl group, a cyclopentyl group, a cyclohexyl group, a cycloheptyl group, a cyclooctyl sgroup and the like, and preferred are a cyclopentyl group, a cyclohexyl group, a cycloheptyl group, a cyclooctyl group and the like.

In the term "optionally having a substituent(s)" used in the present specification, the substituent is:
(1) a halogen atom;
(2) a hydroxyl group;
(3) a thiol group;
(4) a nitro group;
(5) a nitroso group;
(6) a cyano group;
(7) a carboxyl group;
(8) a hydroxysulfonyl group;
(9) an amino group;
(10) a C₁₋₂₂ alkyl group, for example, a methyl group, an ethyl group, a n-propyl group, an iso-propyl group, a n-butyl group, an iso-butyl group, a sec-butyl group, a tert-butyl group and the like;
(11) an unsaturated C₂₋₂₂ alkyl group, for example, a vinyl group, an allyl group, a 1-propenyl group, a 2-propenyl group, an isopropenyl group, an ethynyl group, a 1-propynyl group, a 2-propynyl group, a 1-butynyl group, a 2-butynyl group, a 3-butynyl group and the like;
(12) a C₆₋₁₄ aryl group, for example, a phenyl group, a 1-naphthyl group, a 2-naphthyl group and the like;
(13) a 5 to 14-membered ring heteroaryl group, for example, a thienyl group, a furyl group, a pyridyl group, a pyridazyl group, a pyrimidyl group, a pyrazyl group and the like;
(14) a 3 to 14-membered non-aromatic heterocyclic group, for example, an aziridinyl group, an azetidyl group, a pyrrolidinyl group, a pyrrolyl group, a piperidinyl group, a piperazinyl group, a homopiperidyl group, a homopiperazyl group, an imidazolyl group, a pyrazolidyl group, an imidazolidyl group, a morpholyl group, a thiomorpholyl group, an imidazolinyl group, an oxazolinyl group, a quinuclidyl group and the like;
(15) a C₃₋₁₄ cycloalkyl group, for example, a cyclopropyl group, a cyclobutyl group, a cyclopentyl group, a cyclohexyl group, a cycloheptyl group, a cyclooctyl group and the like;
(16) a C₁₋₂₂ alkoxy group, for example, a methoxy group, an ethoxy group, a n-propoxy group, an iso-propoxy group, a sec-propoxy group, a n-butoxy group, an iso-butoxy group, a tert-butoxy group and the like;
(17) an unsaturated C₂₋₂₂ alkoxy group, for example, a vinyloxy group, an allyloxy group, a 1-propenyloxy group, a 2-propenyloxy group, an isopropenyloxy group, an ethynyloxy group, a 1-propynyloxy group, a 2-propynyloxy group, a 1-butynyloxy group, a 2-butynyloxy group and the like;
(18) a C₆₋₁₄ aryloxy group, for example, a phenyloxy group, a 1-naphthyloxy group, a 2-naphthyloxy group and the like;
(19) a C₇₋₂₂ aralkyloxy group, for example, a benzyloxy group, a phenethyloxy group, a 3-phenylpropyloxy group, a 4-phenylbutyloxy group, a 1-naphthylmethyloxy group, a 2-naphthylmethyloxy group and the like;
(20) a 5 to 14-membered ring heteroaralkyloxy group, for example, thienylmethyloxy group, a furylmethyloxy group, a pyridylmethyloxy group, a pyridazylmethyloxy group, a pyrimidylmethyloxy group, a pyrazylmethyloxy group and the like;
(21) a 5 to 14-membered ring heteroaryloxy group, for example, a thienyloxy group, a furyloxy group, a pyridyloxy group, a pyridazyloxy group, a pyrimidyloxy group, a pyrazyloxy group and the like;
(22) an aliphatic C₂₋₂₂ acyl group, for example, an acetyl group, a propionyl group, a butyryl group, an iso-butyryl group, a valeryl group, an iso-valeryl group, a pivalyl group, a caproyl group, a decanoyl group, a lauroyl group, a myristoyl group, a palmitoyl group, a stearoyl group, an arachidoyl group, an acryl group, a propyol group, a crotonoyl group, an iso-crotonoyl group, an oleinol group, a linolenoyl group and the like;
(23) an aromatic C₇₋₁₅ acyl group, for example, a benzoyl group, a 1-naphthoyl group, 2-naphthoyl group and the like;
(24) an aliphatic C₂₋₂₂ acyloxy group, for example, an acetoxy group, a propionyloxy group, an acryloxy group and the like;
(25) a C₂₋₂₂ alkoxycarbonyl group, for example, a methoxycarbonyl group, an ethoxycarbonyl group, a n-propoxycarbonyl group, an iso-propxycarbonyl group, a n-butoxycarbonyl group, an iso-butoxycarbonyl group, a sec-butoxycarbonyl group, a tert-butoxycarbonyl group and the like; or
(26) an unsaturated C₃₋₂₂ alkoxycarbonyl group, for example, a vinyloxycarbonyl group, an allyloxycarbonyl group, a 1-propenyloxycarbonyl group, a 2-propenyloxycarbonyl group, an isopropenyloxycarbonyl group, a propalgyloxycarbonyl group, a 2-butynyloxycarbonyl group.

The "protecting group" in the present specification is not particularly limited as long as it is a group that can be used as a protecting group for a hydroxyl group in organic synthesis. Specific examples thereof include silyl protecting groups such as a tert-butyldimethylsilyl group, a tert-butyldiphenylsilyl group, a triethylsilyl group, a triisopropylsilyl group, a trimethylsilyl group, a diethylisopropylsilyl group, a dimethylisopropylsilyl group, a di-tert-butylmethylsilyl group, a diphenylmethylsilyl group, a trimethylsilylethoxymethyl group and a trimethylsilylethyl group; alkoxyalkyl protecting groups such as a methoxymethyl group, a 2-methoxyethoxymethyl group, a 2,2,2-trichloroethoxymethyl group, an 1-ethoxyethyl group, an 1-methyl-1-methoxyethyl group and a tetrahydropyranyl group; benzyl protecting groups such as a benzyl group, a 4-methoxybenzyl group, a 3,4-dimethoxybenzyl group, a 2,5-dimethoxybenzyl group, a 2,3,4-trimethoxybenzyl group, a 3,4,5-trimethoxybenzyl group, a 2-nitrobenzyl group, a 4-nitrobenzyl group, a 4-chlorobenzyl group, a 2,6-dichlorobenzyl group, a 4-cyanobenzyl group, a diphenylmethyl group and a triphenymethyl group; acetyl protecting groups such as an acetyl group, a chloroacetyl group, a dichloroacetyl group, a trichloroacetyl group, a fluoroacetyl group, a difluoroacetyl group, a trifluoroacetyl group, a bromoacetyl group, a tribromoacetyl group, a methoxyacetyl group, a pivaloyl group and a benzoyl group; and alkoxycarbonyl protecting groups such as a methoxycarbonyl group, 2,2,2-trichloroethoxycarbonyl group and a benzyloxycarbonyl group; and the like.

For P₁, preferred examples of the "protecting group" include an acetyl group, a tert-butyldimethylsilyl group, a tert-butyldiphenylsilyl group, a triethylsilyl group, a triisopropylsilyl group, a diethylisopropylsilyl group, a dimethylisopropylsilyl group, a methoxymethyl group, an 1-ethoxyethyl group, a tetrahydropyranyl group, a benzyl group, a benzoyl group and the like. It is particularly preferable that P₁ is an acetyl group or a tert-butyldimethylsilyl group. Additionally, OP₁ may represent the following formula together with R₄' described below: 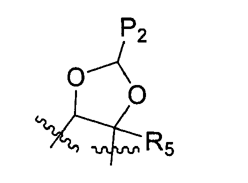 in which P₂ is a phenyl group or a C₁₋₆ alkyl group.

For P₃, preferred examples of the "protecting group for a hydroxyl group" include an acetyl group, a tert-butyldimethylsilyl group, a tert-butyldiphenylsilyl group, a triethylsilyl group, a triisopropylsilyl group, a diethylisopropylsilyl group, a dimethylisopropylsilyl group, a methoxymethyl group, a benzyl group, a 4-methoxybenzyl group, a 3,4-dimethoxybenzyl group, a 2,5-dimethoxybenzyl group, a 2,3,4-trimethoxybenzyl group, a 3,4,5-trimethoxybenzyl group, a benzoyl group and the like, and more preferably include an acetyl group, a benzyl group, a 4-methoxybenzyl group, and a benzoyl group.

For R₄', preferred examples of the "protecting group for a hydroxyl group" include a triethylsilyl group, a diethylisopropylsilyl group, a dimethylisopropylsilyl group, a methoxymethyl group, an 1-ethoxyethyl group and the like, and more preferably include a methoxymethyl group and an 1-ethoxyethyl group.

For R₆', preferred examples of the "protecting group for a hydroxyl group" include a tert-butyldimethylsilyl group, a tert-butyldiphenylsilyl group, a triethylsilyl group, a triisopropylsilyl group, a diethylisopropylsilyl group, a dimethylisopropylsilyl group, a methoxymethyl group, an 1-ethoxyethyl group, a tetrahydropyranyl group, a benzyl group, a benzoyl group and the like, and more preferably include a tert-butyldimethylsilyl group and a triethylsilyl group.

For the 12-membered-ring macrolactam derivative which is the compound represented by Formula (1) of the invention, preferred is a compound in which R₁ is selected from the group consisting of a hydrogen atom and a C₁₋₃ alkyl group; R₂ is selected from the group consisting of a hydrogen atom and a C₁₋₃ alkyl group; R₃ is a hydrogen atom or a hydroxyl group; R₄ is a hydrogen atom or a hydroxyl group; R₅ is selected from the group consisting of a hydrogen atom and a C₁₋₃ alkyl group; R₆ is a hydrogen atom or a hydroxyl group; and R₇ is R^{a}'C(=Y), wherein Y represents an oxygen atom or a sulfur atom and R^{a}' represents a C₁₋₂₂ alkyl group optionally having a substituent(s) or R^{N1}R^{N2}N-, wherein R^{N1} and R^{N2} may be the same or different from each other and each represents
1) Formula (I) shown below which may have a substituent(s) or m) Formula (II) shown below which may have a substituent(s): 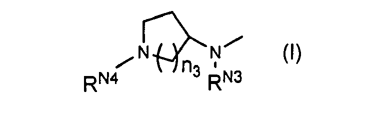 wherein n₃ is an integer from 1 to 3; R^{N3} is a hydrogen atom, a methyl group or an ethyl group; R^{N4} is a hydrogen atom, a C₁₋₂₂ alkyl group or a C₃₋₁₀ cycloalkyl group; 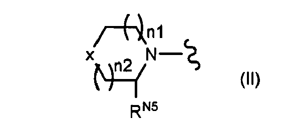 wherein n1 and n2 may be the same or different from each other and are each an integer from 0 to 4; R^{N5} is a hydrogen atom or a C₁₋₆ alkyl group; and X is -CH₂, -O-, -S-or -NR^{N6}-, wherein R^{N6} is a hydrogen atom, a C₁₋₆ alkyl group optionally having a substituent(s), an unsaturated C₂₋₁₀ alkyl group optionally having a substituent(s), a C₆₋₁₄ aryl group optionally having a substituent(s), a 5 to 14-membered ring heteroaryl group optionally having a substituent(s), a C₇₋₁₀ aralkyl group optionally having a substituent(s), a C₃₋₁₀ cycloalkyl group optionally having a substituent(s), a C₄₋₉ cycloalkylalkyl group optionally having a substituent(s), a 5 to 14-membered ring heteroaralkyl group optionally having a substituent(s) or a 5 to 14-membered non-aromatic heterocyclic group optionally having a substituent(s).

More preferred is a compound in which R₁ is selected from the group consisting of a hydrogen atom and a C₁₋₃ alkyl group; R₂ is selected from the group consisting of a hydrogen atom and a C₁₋₃ alkyl group; R₃ is a hydrogen atom or a hydroxyl group; R₄ is a hydrogen atom or a hydroxyl group; R₅ is selected from the group consisting of a hydrogen atom and a C₁₋₃ alkyl group; R₆ is a hydrogen atom or a hydroxyl group; and R₇ is R^{a}'C(=Y), wherein Y is an oxygen atom or a sulfur atom and R^{a}' is a C₁₋₂₂ alkyl group optionally having a substituent(s). Moreover, still more preferred is a compound in which R₁ is selected from the group consisting of a hydrogen atom, a methyl group and an ethyl group; R₂ is selected from the group consisting of a hydrogen atom, a methyl group and an ethyl group; R₃ is a hydrogen atom or a hydroxyl group; R₄ is a hydrogen atom or a hydroxyl group; R₅ is selected from the group consisting of a hydrogen atom, a methyl group and an ethyl group; R₆ is a hydrogen atom or a hydroxyl group; and R₇ is an acetyl group. As the compound represented by Formula (1) of the invention, particularly preferred are compounds (P15), (P16), (P24) and (P36) which will be described later.

The compound represented by Formula (1) of the invention, for example, can be synthesized by general organic chemical synthesis means chosen in accordance with production processes described below. In addition, the compound of interest can be purified from a reaction mixture by an common method after completing a reaction in each process.

Groups such as R₁ in the formulae shown below have the same meanings as defined above. The list of abbreviation used in process for production and examples is shown below.
Bn: benzyl
Et: ethyl
Me: methyl
Ph: phenyl
Ac: acetyl
TBS: tert-butyldimethylsilyl
Boc: tert-butoxycarbonyl
TES: triethylsilyl
Ts: para-toluenesulfonyl
PMP: para-methoxyphenyl
PMB: para-methoxybenzyl(4-methoxybenzyl)
DME: 1,2-dimethoxyethane
DMF: N,N-dimethylformamide
THF: tetrahydrofuran

### Production Process A

The compound of Formula (1') of the invention, which is a compound having P₁ in place of R₇ and R₄' and R₆' each may be an O-protecting group in the compound of Formula (1), can be synthesized by reacting the compound of Formula (11) with the compound of Formula (2) in a solvent in the presence of a catalyst.

### Production Process A

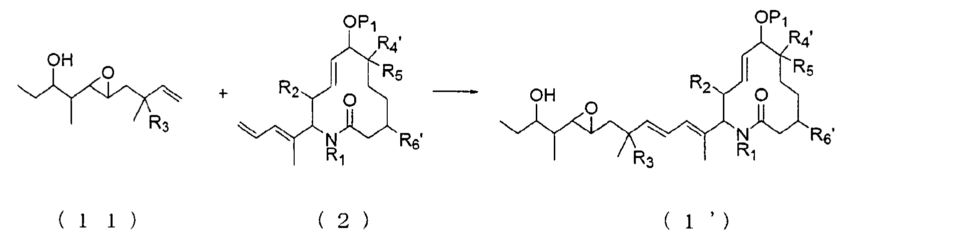

When one or both of R₄' and R₆' in the compound of Formula (1') is an O-protecting group such as O-TBS, the protecting group can be removed by a usual deprotection reaction thereby obtaining the compound of Formula (1). When P₁ is a protecting group such as an acetyl group not requiring a conversion into R₇, the compound of Formula (1) can be obtained according to Production Process A. When P₁ i is a protecting group such as TBS requiring a conversion into R₇ such as an acetyl group, it can be converted by a method pursuant to Production Process B' or B" described below thereby obtaining the compound of Formula (1). Furthermore, when P₁ has to be converted into R₇ such as a urethane group or a thiourethane group, the compound of Formula (1) can be obtained by a method pursuant to Production Processes E and F which will be described later.

This process can be carried out according to a generally used method described in a literature, Grubbs, R. H., "Handbook of metathesis", Wiley-VCH, 2003, Vol. 2, p 246-292, or the like. More specifically, the process can be carried out with reference to reaction conditions, work up procedures after the reaction, a purification method and the like described in Processes (1-21), (2-3), (3-8) and (4-12) in Examples to be described later. This process can be carried out in a stream of or under an atmosphere of an inert gas such as nitrogen and argon.

For the compound of Formula (11), a compound synthesized by the production method described in Pamphlet of International Publication No. 2007/043621 and a method pursuant thereto can be used. The compound of Formula (11) is preferably compounds (Q9), (R1) or the like which will be described in Examples later.

For the compound of Formula (2), a compound synthesized by Production Process B which will be described later and a method pursuant thereto can be used. The compound of Formula (2) is preferably compounds (P14), (P22), (P23), (P33), (P34), (P35) or the like which will be described in Examples later.

As to the solvent for use in the process, there is no particular limitation as long as it can dissolve starting materials to some extent and does not obstruct the reaction. Specific examples thereof include aromatic hydrocarbon solvents such as benzene, toluene, xylene, chlorobenzene and dichlorobenzene; halogenated hydrocarbon solvents such as dichloromethane, 1,2-dichloroethane, chloroform and carbon tetrachloride; and the like, and more preferably used are halogenated hydrocarbon solvents.

Examples of the catalyst for use in the process include
[1,3-bis-(2,4,6)trimethylphenyl-2-imidazolidinylidene]dichloro(phenylmethylene)-tricycl ohexylphosphine)ruthenium,
[1,3-bis-(2,4,6)trimethylphenyl-2-imidazolidinylidene]dichloro(O-isopropoxyphenylmet hylene)ruthenium,
tricyclohexylphosphine[1,3-bis-(2,4,6)trimethylphenyl-4,5-dihydroimidazol-2-ylidene[be nzylidene]ruthenium(IV)dichloride,
[1,3-bis-(2,4,6)trimethylphenyl-2-imidazolidinylidene]dichloro(2-isopropoxy-5-nitrophe nylmethylene)ruthenium,
[1,3-bis-(2,4,6)trimethylphenyl-2-imidazolidinylidene]dichloro(2-isopropoxy-3-phenylph enylmethylene)ruthenium,
3-bis-(2,4,6)trimethylphenyl-2-imidazolidinylidene]dichloro(2,2'-diisopropoxy-1,1'-bina phthalene-3-ylmethylene)ruthenium,
[1,3-bis-(2,4,6)trimethylphenyl-2-imidazolidinylidene]dichloro(2-methoxyphenylmethyl ene)ruthenium,
[1,3-bis-(2,4,6)trimethylphenyl-2-imidazolidinylidene]dichloro(2,4,5-trimethoxyphenylm ethylene)ruthenium,
tricyclohexylphosphine[1,3-bis-(2,4,6)trimethylphenyl-2,3-dihydroimidazol-2-yiliden][b enzylidene]ruthenium(IV)dichloride,
bistricyclohexylphosphine[3,3-diphenylprop-2-en-1-yiliden]ruthenium(IV)dichloride, bis[3-bromopyridine][1,3-bis-(2,4,6)trimethylphenyl-2-imidazolidinylidene][benzylidene ]ruthenium(IV)dichloride,
bistricyclohexylphosphine[benzylidene]ruthenium(IV)dichloride, 2,6-diisopropylphenylimido neophylidene molybdenum bis(hexafluoro-t-butoxide) and the like. Among these, preferably used are
[1,3-bis-(2,4,6)trimethylphenyl-2-imidazolidinylidene]dichloro(phenylmethylene)-tricycl ohexylphosphine)ruthenium,
[1,3-bis-(2,4,6)trimethylphenyl-2-imidazolidinylidene]dichloro(O-isopropoxyphenylmet hylene)ruthenium, and
tricyclohexylphosphine[1,3-bis-(2,4,6)trimethylphenyl-4,5-dihydroimidazol-2-ylidene[be nzylidene]ruthenium(IV)dichloride.
The above catalyst can be used in an amount of 0.001 to 1 time molar equivalent, preferably 0.01 to 0.3 times molar equivalent, to the compound of Formula (2).

Reaction conditions such as reaction temperature and reaction time for the process can be suitably determined in consideration of types of reagents to be used in the reaction such as starting materials and solvents, and the like. For example, the reaction temperature is preferably from 20°C to the reflux temperature (internal temperature of the reaction vessel), and more preferably the reflux temperature (internal temperature of the reaction vessel). In regard to the reaction time, it is preferable to stir the reaction solution at the above reaction temperature for 0.1 to 96 hours, more preferably 0.5 to 12 hours, and even more preferably about 1 to 5 hours, after adding the reagents.

### Production Process B

The compound of Formula (2) can be synthesized by converting -OP₃ of the compound of Formula (3) into a methylene group. The conversion can be done by various reactions in consideration of the protecting group P₃. Preferably, the compound of Formula (2) can be synthesized by optionally deprotecting -OP₃ of the compound of Formula (3) to give a hydroxyl group, oxidizing the hydroxyl group to give a carbonyl group, and then converting the thus obtained carbonyl group into a methylene group. When R₄' and R₆' are each an O-protecting group, conversion or removal of the protecting group can be done at the same time of the conversion of P₃.

### Production Process B

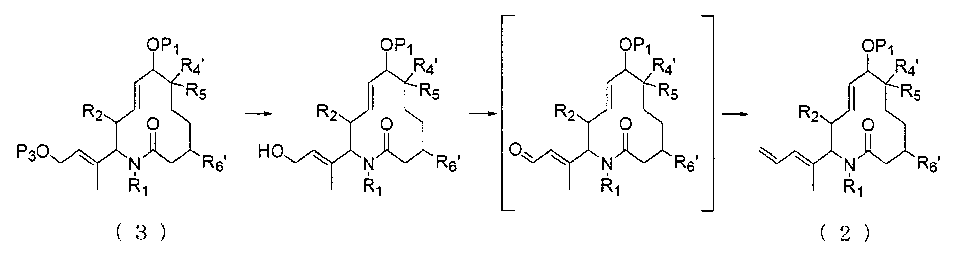

When P₃ in the compound of Formula (3) is a hydrogen atom, deprotection of P₃ is not needed and an oxidation reaction can be directly carried out. When P₃ is a protecting group, it is preferable to convert P₃ into a hydrogen atom by deprotection reaction and subject the oxidation reaction thereafter. The deprotection reaction in the process can be carried out by a general organic synthesis means that is commonly employed, considering the kind of protecting group, for deprotecting a protecting group of a hydroxyl group. More specifically, the process can be carried out with reference to reaction conditions, work up procedures after the reaction, a purification method and the like described in Processes (1-12), (1-13), (3-5) and (4-9) in Examples described later.

In addition, when P₁ in the compound of Formula (3) is a hydrogen atom, it is preferable to convert P₁ into a protecting group by a protection reaction and subject the oxidation reaction thereafter. The protection reaction of a hydroxyl group in the process varies depending on the kind of protecting group, but can be carried out by a general organic synthesis means that is commonly employed for protecting a hydroxyl group. More specifically, the process can be carried out with reference to reaction conditions, work up procedures after the reaction, a purification method and the like described in Processes (1-11) and (3-4) in Examples described later.

The oxidation reaction in the process can be carried out according to a generally used method such as an oxidation reaction employing Dess-Martin Periodinane (DMP). In addition, the conversion reaction into a methylene group in the process can be carried out according to a generally used method such as a Wittig reaction. More specifically, the process can be carried out with reference to reaction conditions, work up procedures after the reaction, a purification method and the like described in Processes (1-14) and (3-6) in Examples described later.

For the compound of Formula (3), a compound synthesized by Production Processes C1 and C2 which will be described later and a method pursuant thereto can be used. The compound of Formula (3) is preferably compounds (P11), (P12), (P 13), (P20), (P21), (P32) or the like which will be described in Examples later.

### Production Process B'

Optionally, the protecting group P₁ in the compound of Formula (2) obtained according to Production Process B can be converted to give the compound of Formula (2'). Specifically, before carrying out the above-mentioned Production Process A, the protecting group P₁ such as TBS can be converted preliminarily into a protecting group such as an acetyl group not requiring a conversion into R₇. The compound of Formula (2') can be synthesized by deprotecting -OP₁ of the compound of Formula (2) to give a hydroxyl group, and thereafter modifying the obtained hydroxyl group.

### Production Process B'

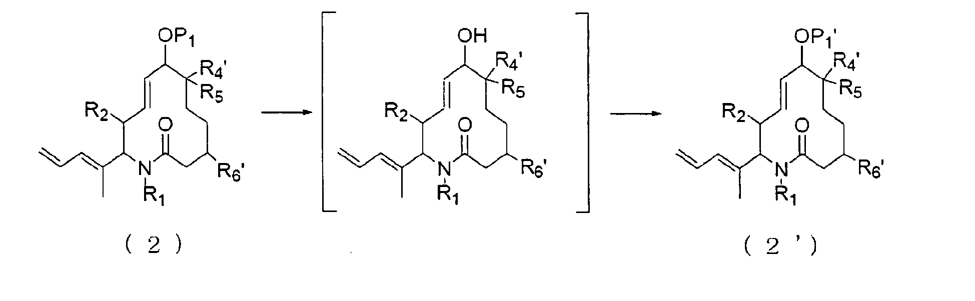

wherein P₁' is a hydrogen atom or a protecting group such as an acetyl group not requiring a conversion into R₇.

The deprotection reaction in the process can be carried out by a general organic synthesis means that is commonly employed, considering the kind of protecting group, for deprotecting a protecting group of a hydroxyl group. Also, the modification reaction in the process can be carried out by a general organic synthesis means that is commonly employed, considering the kind of modifying group, for modifying a hydroxyl group. For example, a modification of introducing an acetyl group to the hydroxyl group can be carried out by deprotecting -OP₁ of the compound of Formula (2) to give a hydroxyl group and reacting the thus obtained compound with acetic anhydride. More specifically, the process can be carried out with reference to reaction conditions, work up procedures after the reaction, a purification method and the like described in Process (3-7) in Examples described later.

For the compound of Formula (2), a compound synthesized according the above-mentioned Production Process B and a method pursuant thereto can be used. The compound of Formula (2) is preferably a compound (P22) or the like which will be mentioned in Examples described later.

### Production Process B"

In particular, when R₄' and OP₁ in the compound of Formula (2) together represent the following formula (Formula (2")): 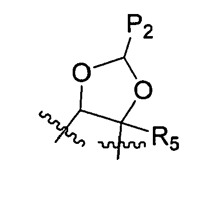 wherein P₂ is a phenyl group or a C₁₋₆ alkyl group,
the compound of Formula (2') can be synthesized in a solvent in the presence of reagents by removing a protecting group of the hydroxyl groups at the 6th and the 7th positions in the compound of Formula (2") and then modifying the hydroxyl group at the 7th position in the thus obtained compound.

### Production Process B"

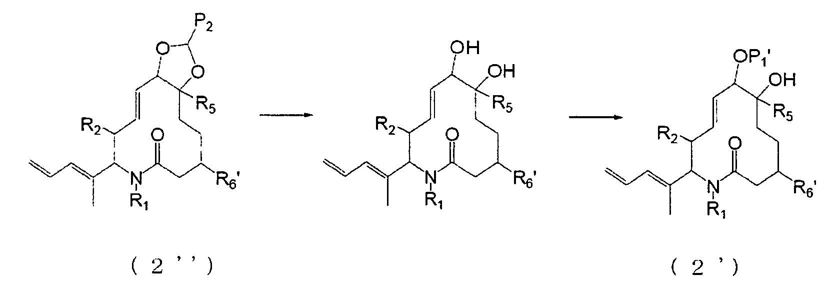

The removal reaction in this process can be carried out according to a generally used method described in a literature, Green, T. W., "Protective Groups in Organic Synthesis, 3rd Edition", Wiley-interscience, 1999, or the like. More specifically, the process can be carried out with reference to reaction conditions, work up procedures after the reaction, a purification method and the like described in Process (4-10) in Examples to be described later.

For the compound of Formula (2"), a compound synthesized according to the above-mentioned Production Process B and a method pursuant thereto can be used. The compound of Formula (2") is preferably a compound (P33) and the like which will be described in Examples later.

As the solvent for use in the removal reaction of the process, there is no particular limitation as long as it can dissolve starting materials to some extent and does not obstruct the reaction. Specific examples thereof include alcohol solvents such as methanol and ethanol, water and the like, and preferably used are alcohol solvents.

Examples of the reagent for use in the removal reaction of the process include inorganic acids such as hydrochloric acid, sulfuric acid, hydrofluoric acid and perchloric acid; organic acids such as p-toluenesulfonic acid, pyridinium p-toluenesulfonate (PPTS), trifluoromethane sulfonic acid, methane sulfonic acid, acetic acid and trifluoroacetic acid; and the like. As the reagent for use in the process, PPTS is preferably used.

The reagent for use in the removal reaction of the process can be used in an amount of 0.05 to 20 times molar equivalent, preferably 0.1 to 10 times molar equivalent, more preferably 1 to 2 times molar equivalent, to the compound of Formula (2").

Reaction conditions such as reaction temperature and reaction time for the removal reaction of the process can be suitably determined in consideration of kinds of reagents to be used in the reaction such as starting materials and solvents, and the like. For example, the reaction temperature is preferably from 0 to 50°C (internal temperature of the reaction vessel), and more preferably room temperature (internal temperature of the reaction vessel). In regard to the reaction time, it is preferable to stir the reaction solution at the above reaction temperature for 0.5 to 96 hours, after adding the reagents.

The compound of Formula (2') can be synthesized by modifying a hydroxyl group at the 7th position of the compound obtained by the removal reaction. The modification reaction in the process can be carried out by a general organic synthesis means that is commonly employed, considering the kind of modifying group, for modifying a hydroxyl group. More specifically, the process can be carried out with reference to reaction conditions, work up procedures after the reaction, a purification method and the like described in Process (4-11) in Examples described later.

### Production Process C1

For one embodiment, the compound of Formula (3) can be synthesized by closing a ring in the compound of Formula (4-1). The ring-closure reaction can be carried out according to a Nozaki-Hiyama-Kishi reaction. Preferably, the compound of Formula (3) can be synthesized by oxidizing a hydroxyl group in the compound of Formula (4-1) and thereafter subjecting a ring closure between the aldehyde group thus obtained and a halogen atom. In particular, when R₂ is a C₁₋₆ alkyl group, this process is preferably used.

### Production Process C1

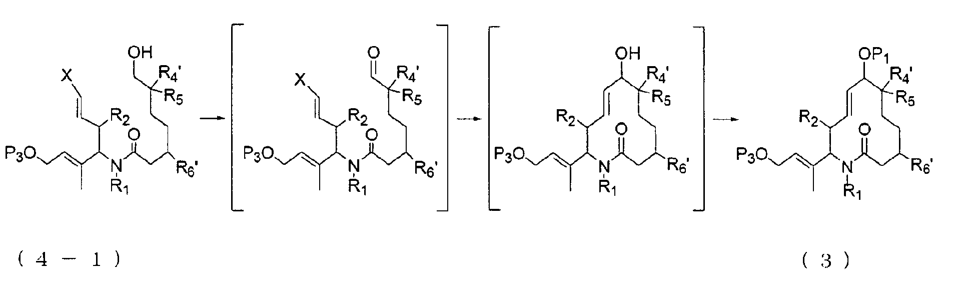

This process can be carried out according to a well-known method such as a method by Furstner et al., Chemical Review, 1999, Vol. 99, p 991-1045; a method by Stamos et al., Tetrahedron Letter, 1997, Vol. 38, Issue No. 36, p 6355-6358; a method by Pilli et al., The Journal of Organic Chemistry, 1998, Vol. 63, p 7811-7819; or the like. More specifically, the process can be carried out with reference to reaction conditions, work up procedures after the reaction, a purification method and the like described in Processes (1-11) and (3-4) in Examples described later. This reaction can be carried out in a stream of or under an atmosphere of an inert gas such as nitrogen and argon.

As to the solvent for the process, there is no particular limitation as long as it can dissolve starting materials to some extent and does not obstruct the reaction. Examples of the solvent include N,N-dimethylformamide, dimethylsulfoxide, tetrahydrofuran and mixed solvents thereof. Preferred are N,N-dimethylformamide, dimethylsulfoxide and tetrahydrofuran, and more preferred is N,N-dimethylformamide.

As the catalyst for the process, chromium (II) chloride, nickel (II) chloride or the like can be used. The catalyst can be used in an amount of 0.002 to 15 times molar equivalent, preferably 0.04 to 10 times molar equivalent, more preferably 0.06 to 6 times molar equivalent, to the compound of Formula (4-1).

The reaction temperature varies depending on starting materials, solvent and reagents to be used in reactions, but is preferably from 0 to 30°C (internal temperature of the reaction vessel), more preferably 0°C to room temperature, even more preferably room temperature (15 to 25°C).

The reaction time varies usually depending on starting materials, solvent, reagents to be used in reactions and reaction temperature, but it is preferable to stir the reaction solution at the above reaction temperature for 1 to 24 hours, more preferably 2 to 13 hours, and even more preferably about 3 to 13 hours, after adding the reagents.

For the compound of Formula (4-1), a compound synthesized according to Production Process D1 which will be described below and a method pursuant thereto can be used. The compound of Formula (4-1) is preferably compounds (P10), (P19) or the like which will be described in Examples later.

### Production Process D 1

The compound of Formula (4-1) can be synthesized by reacting the compound of Formula (17) with the compound of Formula (18). Preferably, the compound of Formula (4-1) can be obtained by removing a protecting group of the amino group in the compound of Formula (17) by deprotection reaction and thereafter subjecting a dehydration-condensation reaction with the carbonyl group in the compound of Formula (18) to form an amide bond.

### Production Process D1

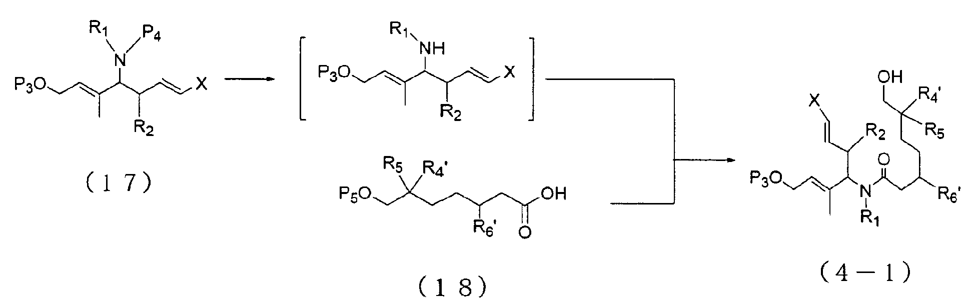

In the formula, P₄ is a protecting group and P₅ is a protecting group.

The deprotection reaction in the process can be carried out by a general organic synthesis means that is commonly employed, considering the kind of protecting group, for deprotecting a protecting group of an amino group. More specifically, the process can be carried out with reference to reaction conditions, work up procedures after the reaction, a purification method and the like described in Processes (1-10) and (3-3) in Examples described later.

The dehydration-condensation reaction in the process can be carried out by a general organic synthesis means that is commonly employed for forming an amide bond. More specifically, the process can be carried out with reference to reaction conditions, work up procedures after the reaction, a purification method and the like described in Processes (1-10) and (3-3) in Examples described later.

For the compound of Formula (17), a compound synthesized according to Examples (1-10) and (3-3) which will be described later and a method pursuant thereto can be used. The compound of Formula (17) is preferably compounds (P7), (P18) or the like which will be described in Examples later.

For the compound of Formula (18), a compound synthesized according to Example (1-9) which will be described later and a method pursuant thereto can be used. Particularly preferred are compounds (P9) and the like which will be mentioned in Examples described later.

### Production Process C2

For the other embodiment, the compound of Formula (3) can be synthesized by closing a ring in the compound of Formula (4-2). The ring-closure reaction can be carried out according to a RCM reaction. Preferably, the compound of Formula (3) can be synthesized by subjecting ring-closure reaction of the compound of Formula (4-2) in a solvent in the presence of a catalyst. In particular, when R₂ is a hydrogen atom, this process is preferably used.

### Production Process C2

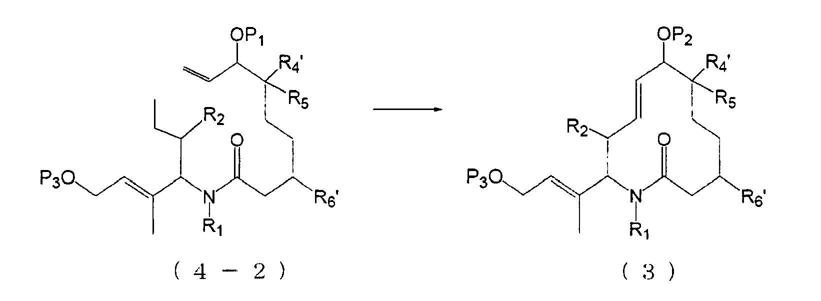

This process can be carried out according to a generally used method described in a literature, Grubbs, R.H., "Handbook of metathesis" Wiley-VCH, 2003, Vol. 1-3, or the like. More specifically, the process can be carried out with reference to reaction conditions, work up procedures after the reaction, a purification method and the like described in Process (4-8) in Examples described later. This reaction can be carried out in a stream of or under an atmosphere of an inert gas such as nitrogen and argon.

For the compound of Formula (4-2), a compound synthesized by Production Process D2 which will be described later and a method pursuant thereto can be used. Particularly preferred are compounds (P31) and the like which will be mentioned in Examples described later.

As to the solvent for use in the process, there is no particular limitation as long as it can dissolve starting materials to some extent and does not obstruct the reaction. Examples of the solvent include aromatic hydrocarbon solvents such as benzene, toluene, xylene, chlorobenzene and dichlorobenzene; halogenated hydrocarbon solvents such as dichloromethane, 1,2-dichloroethane, chloroform and carbon tetrachloride; and the like, and preferably used are benzene, toluene, dichloromethane and 1,2-dichloroethane.

For the process, the catalyst means
[1,3-bis-(2,4,6)trimethylphenyl-2-imidazolidinylidene]dichloro(O-isopropoxyphenylmet hylene)ruthenium,
tricyclohexylphosphine[1,3-bis-(2,4,6)trimethylphenyl-4,5-dihydroimidazol-2-ylidene[be nzylidene]ruthenium(IV)dichloride,
[1,3-bis-(2,4,6)trimethylphenyl-2-imidazolidinylidene]dichloro(2-isopropoxy-5-nitrophe nylmethylene)ruthenium,
[1,3-bis-(2,4,6)trimethylphenyl-2-imidazolidinylidene]dichloro(2-isopropoxy-3-phenylph enylmethylene)ruthenium,
[1,3-bis-(2,4,6)trimethylphenyl-2-imidazolidinylidene]dichloro(2,2'-diisopropoxy-1,1'-bi naphthalene-3-ylmethylene)ruthenium,
[1,3-bis-(2,4,6)trimethylphenyl-2-imidazolidinylidene]dichloro(2-methoxyphenylmethyl ene)ruthenium,
[1,3-bis-(2,4,6)trimethylphenyl-2-imidazolidinylidene]dichloro(2,4,5-trimethoxyphenylm ethylene)ruthenium,
tricyclohexylphosphine[1,3-bis-(2,4,6)trimethylphenyl-2,3-dihydroimidazol-2-yiliden][b enzylidene]ruthenium(IV)dichloride,
bistricyclohexylphosphine[3,3-diphenylprop-2-en-1-yiliden]ruthenium(IV)dichloride, bis[3-bromopyridine][1,3-bis-(2,4,6trimethylphenyl)-2-imidazolidinylidene][benzylidene ]ruthenium(IV)dichloride and the like. Preferred catalyst is
[1,3-bis-(2,4,6)trimethylphenyl-2-imidazolidinylidene]dichloro(O-isopropoxyphenylmet hylene)ruthenium.

The above catalyst can be used in an amount of 0.001 to 1 times molar equivalent, preferably 0.01 to 1 times molar equivalent, to the compound of Formula (4-2).

The reaction temperature varies usually depending on starting materials, solvent and reagents to be used in reactions, but is preferably from 25°C to the reflux temperature (internal temperature of the reaction vessel), more preferably the reflux temperature (internal temperature of the reaction vessel). In addition, the reaction time varies usually depending on starting materials, solvent, reagents to be used in reactions and reaction temperature, but it is preferable to stir the reaction solution at the above reaction temperature for 0.5 to 48 hours, more preferably 1 to 8 hours, after adding the reagents.

### Production Process D2

The compound of Formula (4-2) can be synthesized by reacting the compound of Formula (23) with the compound of Formula (24). Preferably, the compound of Formula (4-2) can be obtained by subjecting a dehydration-condensation reaction between an amino group in the compound of Formula (23) and the carboxyl group in the compound of Formula (24) to form an amide bond.

### Production Process D2

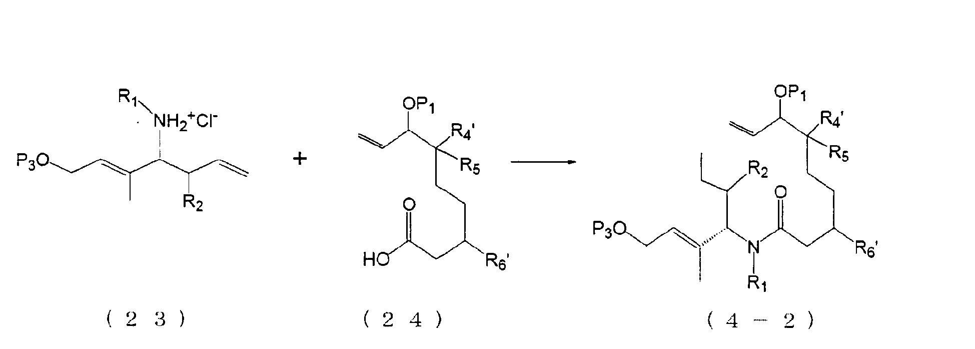

The dehydration-condensation reaction in the process can be carried out by a general organic synthesis means that is commonly employed for forming an amide bond. More specifically, the process can be carried out with reference to reaction conditions, work up procedures after the reaction, a purification method and the like described in Process (4-7) in Examples described later.

For the compound of Formula (23), a compound synthesized according to Example (4-6) which will be described later and a method pursuant thereto can be used. The compound of Formula (23) is preferably compound (P30) or the like which will be described in Examples later.

For the compound of Formula (24), a compound synthesized by the production method described in Pamphlet of International Publication No. 2007/043621 and a method pursuant thereto can be used. Particularly preferred are a compound (Q25) and the like which will be mentioned in Examples to be described later.

Furthermore, others among the compounds of Formula (1) of the invention can be synthesized by converting an acetoxy group of the compounds of Formula (1) of the invention which has an acetyl group as R₇ (the following Formula (25)) into a urethane group, a thiourethane group or the like by a general organic synthesis means. As the representative methods, a method of producing a urethane derivative, a method of producing thiourethane and the like are described below.

### Production Process E

Among the compounds of Formula (1) of the invention, a urethane derivative can be synthesized through the following reaction route with the compound of Formula (25) as a starting compound.

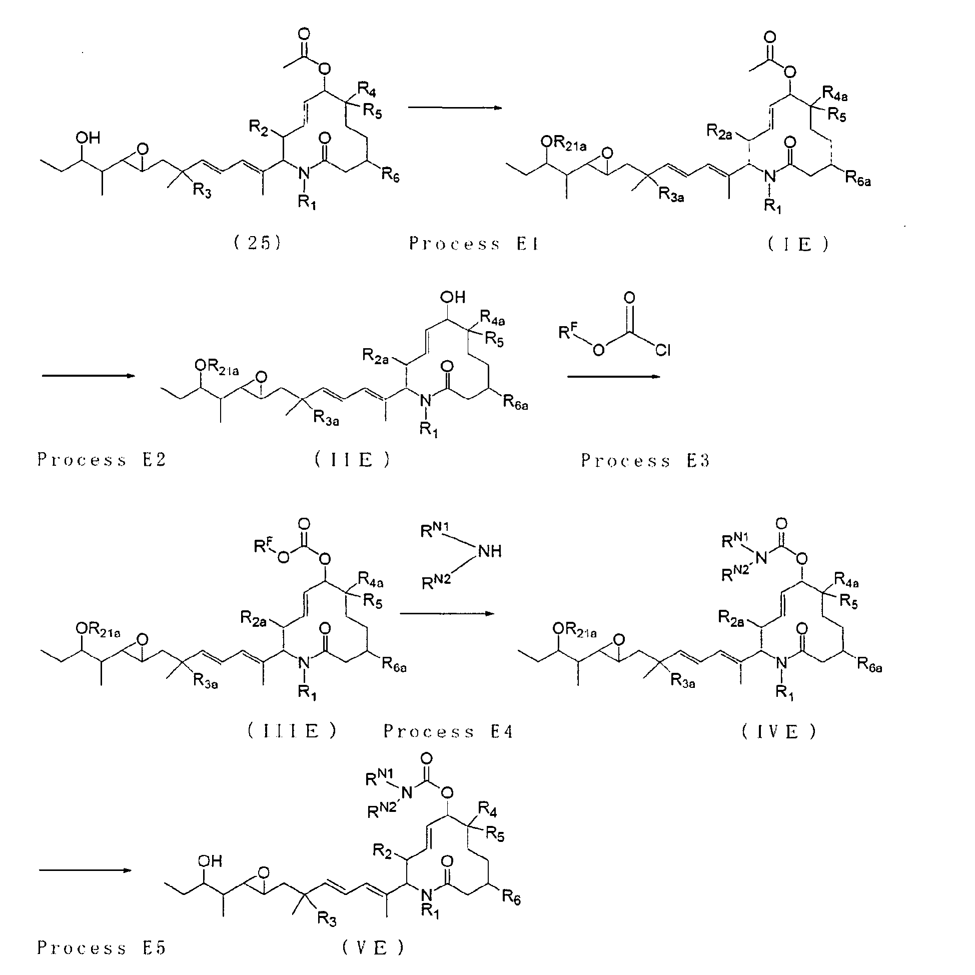

In the formula, when R₃, R₄, R₆ are each a hydroxyl group, R₃ₐ, R₄ₐ, R₆ₐ each represent O-P wherein P is a protecting group; R₂₁ₐ is a protecting group; R^{F} is a C₆₋₁₄ aryl group optionally having a substituent(s).

Process E1 is a process for producing the compound of Formula (IE). This process can be done by protecting a hydroxyl group in the compound of Formula (25). The reaction for protecting a hydroxyl group can be carried out by a method well known in organic synthesis chemistry considering the kind of protecting group.

Examples of the protecting group include 1-ethoxyethyl, tetrahydropyranyl, 1-methyl-1-methoxyethyl, 1-(2-chloroethoxy)ethyl, 1-methoxycyclohexyl, 4-methoxytetrahydropyranyl, 4-methoxytetrahydrothiopyranyl, 4-methoxytetrahydrothiopyranyl-S,S-dioxide, methoxymethyl, methylthiomethyl, methoxyethoxymethyl, trichloroethoxymethyl, trimethylsilylethyl, trimethylsilylethoxymethyl, tert-butyldimethylsilyl, triethylsilyl, diethylisopropylsilyl, trimethylsilyl, triisopropylsilyl, methyl di-tert-butylsilyl, diphenylmethylsilyl, benzyl, p-methoxybenzyl, p-methylbenzyl, p-nitrobenzyl, p-chlorobenzyl, triphenylmethyl and the like. A part or whole of hydroxyl group in the compound of Formula (25) can be appropriately protected.

Protectied derivatives at hydroxyl groups by such as 1-ethoxyethyl, tetrahydropyranyl, 1-methoxycyclohexyl, 4-methoxytetrahydropyranyl, 4-methoxytetrahydrothiopyranyl and 4-methoxytetrahydrothiopyranyl-S,S-dioxide, can be synthesized by treating corresponding vinyl ether such as ethylvinylether or dihydropyran with the compound of Formula (25) in the presence of an acid. As the acid, general acid can be used, for example, organic acids such as pyridinium p-toluenesulfonate (PPTS), p-toluenesulfonic acid, camphorsulfonic acid, acetic acid, trifluoroacetic acid and methane sulfonic acid; inorganic acids such as hydrogen chloride, nitric acid, hydrochloric acid and sulfuric acid; and the like can be used. Among these, preferably used are, for example, PPTS, p-toluenesulfonic acid, camphorsulfonic acid and the like.

The solvent for use in the reaction is not particularly limited, but it is desirably an inert solvent which does not readily react with the starting material. Examples thereof include ethers such as tetrahydrofuran, diethylether, diisopropylether, dioxane and dimethoxyethane; halogenated hydrocarbons such as dichloromethane, chloroform, carbon tetrachloride and 1,2-dichloroethane; hydrocarbons such as hexane, benzene and toluene; ketones such as acetone and methyl ethyl ketone; nitriles such as acetonitrile; amides such as N,N-dimethylformamide, N,N-dimethylacetoamide, N-methyl-2-pyridone and hexamethylphosphorylamide; sulfoxides such as dimethylsulfoxide; and the like. Among these, preferably used are, for example, dichloromethane, chloroform, tetrahydrofuran and the like.

Reaction conditions such as reaction temperature and reaction time for the process can be suitably determined in consideration of kinds of reagents to be used in the reaction such as starting materials and solvents, and the like. For example, the reaction temperature is preferably from -78°C to the heat-reflux temperature (internal temperature of the reaction vessel), and more preferably room temperature (internal temperature of the reaction vessel). In regard to the reaction time, it is preferable to stir the reaction solution at the above reaction temperature for 10 minutes to 5 days, more preferably 1 to 2 day(s), after adding the reagents. Vinyl ether and the acid to be used in the reaction can be used in an amount of 1 to 200 equivalent(s) and 0.05 to 2 equivalents, respectively, preferably 30 to 50 equivalents and 0.1 to 0.3 equivalents, respectively, to the compound of Formula (25).

Moreover, examples of other protecting groups include methoxymethyl, methylthiomethyl, methoxyethoxymethyl, trichloroethoxymethyl, trimethylsilylethyl, trimethylsilylethoxymethyl, tert-butyldimethylsilyl, triethylsilyl, trimethylsilyl, diethylisopropylsilyl, triisopropylsilyl, di-tert-butylmethylsilyl, diphenylmethylsilyl, benzyl, p-methoxybenzyl, p-methylbenzyl, p-nitrobenzyl, p-chlorobenzyl, triphenylmethyl and the like. The derivatives protected at a hydroxyl group by these groups can be synthesized by reacting a chloro derivative, a bromo derivative or a trifluoromethanesulfonyl derivative in each protecting group in the presence of a base.

As the base, general organic base or inorganic base can be used. Examples of the organic base include aromatic bases such as imidazole, 4-(N,N-dimethylamino)pyridine (4-dimethylaminopyridine, N,N-dimethylaminopyridine and dimethylaminopyridine used in this specification have the same meanings), pyridine, 2,6-lutidine and collidine; tertiary amines such as N-methylpiperidine, N-methylpyrrolidine, triethylamine, trimethylamine, di-iso-propylethylamine, cyclohexyldimethylamine, N-methylmorpholine and 1,8-bis(dimethylamino)naphthalene; secondary amines such as di-iso-butylamine and dicyclohexylamine; alkyllithium such as methyllithium and butyllithium; metal alkoxides such as sodium methoxide and sodium ethoxide; and the like. Examples of the inorganic base include alkali metal hydrides such as sodium hydride and potassium hydride; alkaline-earth metal hydrides such as calcium hydrides; alkali metal hydroxides such as sodium hydroxide and potassium hydroxide; alkali metal carbonates such as sodium carbonate, potassium carbonate and cesium carbonate; alkali metal hydrogen carbonates such as sodium hydrogen carbonate; and the like. As the base preferably used in the case of protecting a hydroxyl group with a silyl protecting group, for example, aromatic bases such as imidazole and 4-dimethylaminopyridine; tertiary amines such as triethylamine; and the like can be used.

The solvent for use in the reaction is not particularly limited, but it is desirably a solvent which does not readily react with the starting material, such as the above-mentioned inert solvents. Preferred are tetrahydrofuran, dichloromethane, N,N-dimethylformamide and the like. In addition, the reaction time is from 10 minutes to 3 days, preferably from 1 to 2 day(s).

By selecting the reagent to be used for protecting a hydroxyl group and the equivalent amount thereof, a hydroxyl group can be protected selectively. For example, a compound in which the hydroxyl groups at the third and the 21 st positions are protected selectively can be obtained by carrying out a reaction in dichloromethane using chlorotriethylsilane, triethylamine, 4-dimethylaminopyridine, or in N,N-dimethylformamide using tert-butylchlorodimethylsilane, imidazole, at room temperature. At this time, it is possible to protect the hydroxyl group at the third position and 21-position by priority, for example, by controlling the equivalent amount of chlorotriethylsilane or tert-butylchlorodimethylsilane. In addition, it is possible to protect two or three hydroxyl groups among the four hydroxyl groups with a silyl group, and then protect the remaining two or one hydroxyl group(s) with the above-mentioned ethoxyethyl group or the like.

Process E2 is a process for producing the compound of Formula (IIE). This process can be done by converting an acetoxy group in the compound of Formula (IE) into a hydroxyl group by treating with a base in an inert solvent.

Examples of the base to be used include alkali metal hydrides such as sodium hydride and potassium hydride; alkaline-earth metal hydrides such as calcium hydride; alkali metal hydroxides such as lithium hydroxide, sodium hydroxide and potassium hydroxide; alkali metal carbonates such as lithium carbonate, sodium carbonate and potassium carbonate; alkali metal hydrogen carbonates such as sodium hydrogen carbonate; metal alkoxides such as lithium methoxide, sodium methoxide, sodium ethoxide and potassium tert-butoxide; and the like, as well as bases such as guanidine and ammonia. Preferred bases are potassium carbonate, guanidine and the like.

As the inert solvent to be used, in addition to the inert solvents mentioned before, alcohols solvents such as methanol, ethanol, isopropanol and tert-butanol, water and the like can be used. These solvents may also be used in combination. Preferred solvents are alcohol solvents and a mixed solvent of alcohol and a halogen solvent. The reaction time is from 10 minutes to 5 days, preferably from 30 minutes to 1 day. The reaction temperature is from -78°C to the heat-reflux temperature, preferably room temperature. The base to be used in the reaction is used in an amount of 1 to 10 equivalent(s), preferably 2 to 5 equivalents, to the compound of Formula (IE).

Process E3 is a process for producing the compound of Formula (IIIE). This process can be done by treating a hydroxyl group of the compound of Formula (IIE) with a chloroformate derivative or carbonyldiimidazole in the presence of a base. Examples of the chloroformate derivative include 4-nitrophenylchloroformate, phenylchloroformate, 4-chlorophenylchloroformate, 4-bromophenylchloroformate and 2,4-dinitrophenylchloroformate. As the base, the above-mentioned organic bases, inorganic bases and the like can be used, and preferred are diisopropylethylamine, 4-dimethylaminopyridine, triethylamine, pyridine, 2,6-lutidine, sodium hydride and the like. The solvent for use in the reaction is not particularly limited, but it is desirably a solvent which does not readily react with the starting material, such as the above-mentioned inert solvents. Preferred are tetrahydrofuran, dichloromethane, N,N-dimethylformamide and the like. The chloroformate derivative and the base to be used in the reaction can be used in an amount of 1 to 10 equivalent(s) and 1 to 20 equivalent(s), respectively, preferably 1 to 5 equivalent(s) and 1 to 10 equivalent(s), respectively, to the compound of Formula (IIE). The reaction time is from 10 minutes to 30 hours, preferably from 1 to 4 hour(s). The reaction temperature is from -78°C to the heat-reflux temperature, preferably from -10 to 50°C.

Process E4 is a process for producing the compound of Formula (IVE). This process can be done by treating carbonate ester of Formula (IIIE) with amine, R^{N1}R^{N2}H, which is capable of forming compound of Formula (1) of interest in the presence of a base, or with the amine only, in an inert solvent.

Examples of the amine used in the process include a methylamine, ethylamine, propylamine, butylamine, octylamine, decylamine, cyclopropylamine, cyclopentylamine, cyclohexylamine, dimethylamine, diethylamine, ethylmethylamine, ethylenediamine, 1,3-propanediamine, 1,4-butanediamine, N,N-dimethylethylenediamine, N,N-dimethyl-1,3-propanediamine, N,N-dimethyl-1,4-butanediamine, N,N-diethylethylenediamine, N,N-diethyl-1,3-propanediamine, N,N-diethyl-1,4-butanediamine, N,N,N'-trimethlyethylenediamine, N,N,N'-trimethyl-1,3-propanediamine, N,N,N'-trimethyl-1,4-butanediamine, N-ethyl-N',N'-dimethylethylenediamnie, N-ethyl-N';N'-dimethyl-1,3-propanediamine, N-ethyl-N',N'-dimethyl-1,4-butanediamine, N,N,N'-triethylethylenediamine, N,N,N'-triethyl-1,3-propanediamine, N,N,N'-triethyl-1,4-butanediamine, N,N-diethyl-N'-methylethylenediamine, N,N-diethyl-N'-methyl-1,3-propanediamine, N,N-diethyl-N'-methyl-1,4-butanediamine, N,N'-dimethyl-N-phenylethylenediamine, N,N'-dimethyl-N-phenyl-1,3-propanediamine, N-benzyl-N,N'-dimethylethylenediamine, N-benzyl-N,N'-dimethyl-1,3-propanediamine, morpholine, thiomorpholine, thiomorpholine-S-oxide, thiomorpholine-S,S-dioxide, pyrrolidine, piperidine, piperazine, homopiperazine, 4-hydoxypiperazine, 4-methoxypiperidine, 1-methylpiperazine, 1-ethylpiperazine, 1-propylpiperazine, 1-butylpiperazine, 1-isopropylpiperazine, 1-cyclobutylpiperazine, 1-cyclopentylpiperazine, 1-cyclohexylpiperazine, 1-cycloheptylpiperazine, 1-cyclooctylpiperazine, 1-(cyclopropylmethyl)piperazine, 1-benzylpiperazine, 1-methylhomopiperazine, 1-ethylhomopiperazine, 1-(2-aminoethyl)pyrrolidine, 1-(2-(N-methylamino)ethyl)pyrrolidine, 1-(3-aminopropyl)pyrrolidine, 1-(3-(N-methylamino)propyl)pyrrolidine, 1-(2-aminoethyl)piperidine, 1-(2-(N-methylamino)ethyl)piperidine, 1-(3-aminopropyl)piperidine, 1-(3-(N-methylamino)propyl)piperidine, 4-(2-aminoethyl)morpholine, 4-(2-(methylamino)ethyl)morpholine, 4-(3-aminopropyl)morpholine, 4-(3-(N-methylamino)propyl)morpholine, 1-(2-aminoethyl)-4-methylpiperazine, 1-(3-aminopropyl)-4-methylpiperazine, 1-(3-(N-methylamino)propyl)-4-methylpiperazine, 1-amino-4-methylpiperidine, 1-methylamino-4-methylpiperidine, 1-ethyl-4-(N-methylamino)piperidine, 1-methylamino-4-propylpiperidine, 1-butyl-4-(N-methylamino)piperidine, 1-(N,N-dimethylamino)piperidine, 1-(N,N-diethylamino)piperidine, 4-(pyrrolidine-1-yl)piperidine, 4-(piperidine-1-yl)piperidine, 3-aminoquinuclidine, 3-(N-methylamino)quinuclidine, aniline, N-methylaniline, N,N-dimethyl-p-phenylenediamine, N,N-dimethyl-m-phenylenediamine, N,N,N'-trimethyl-p-phenylenediamine, N,N,N'-trimethyl-m-phenylenediamine, 1-naphthylamine, 2-naphthylamine, benzylamine, N-methylbenzylamine, phenethylamine, N-methylphenethylamine, 2-picolylamine, 3-picolylamine, 4-picolylamine, N-methyl-2-picolylamine, N-methyl-3-picolylamine, N-methyl-4-picolylamine, 2,5-diazabicyclo[2.2.1]heptane, 2-methyl-2,5-diazabicyclo[2.2.1]heptane, 3,8-diazabicyclo[3.2.1]octane, 1,4-diazabicyclo[4.3.0]nonane and the like.

As the base, the above-mentioned organic bases, inorganic bases and the like can be used, and preferred are diisopropylethylamine, dimethylaminopyridine, triethylamine, pyridine, 2,6-lutidine, sodium hydride and the like. The solvent for use in the reaction is not particularly limited, but it is desirably a solvent which does not readily react with the starting material, such as the above-mentioned inert solvents. Preferred solvents are tetrahydrofuran, dichloromethane, N,N-dimethylformamide and the like. The amine and the base to be used in the reaction can be used in an amount of 1 to 10 equivalent(s) and 2 to 20 equivalents, respectively, preferably 1.5 to 5 equivalent(s) and 2 to 10 equivalent(s), respectively, to the compound of Formula (IIIE). The reaction time is from 10 minutes to 30 hours, preferably from 1 to 2 hour(s). The reaction temperature is from -78°C to the heat-reflux temperature, preferably from -10 to 50°C.

Process E5 is a process for producing the compound of Formula (VE) which is the compound of Formula (1) of the invention. This process can be done by subjecting the urethane derivative which is the compound of Formula (IVE) to a deprotection treatment as shown below in an inert solvent. The reaction for deprotecting the protecting group of a hydroxyl group varies depending on the kind of protecting group, but can be carried out by a general organic synthesis means that is commonly employed for deprotecting a protecting group of a hydroxyl group.

The deprotection of hydroxyl groups of, for example, 1-ethoxyethyl, tetrahydropyranyl, 1-methoxycyclohexyl, 4-methoxytetrahydropyranyl, 4-methoxytetrahydrothiopyranyl, 4-methoxytetrahydrothiopyranyl-S,S-dioxide or the like can be simply carried out subjecting an acid treatment in an inert solvent. As the acid, the above-mentioned organic acids, inorganic acids and the like can be used, and preferred examples are PPTS, p-toluenesulfonic acid, camphorsulfonic acid and the like. The solvent for use in the reaction is not particularly limited, but it is desirably a solvent which does not readily react with the starting material. Preferred are alcohol-based solvents such as methanol, ethanol, isopropanol and tert-butanol, and in addition, these may be used in combination with the above-mentioned inert solvents. The acid to be used in the reaction can be used in an amount of 0.5 to 5 equivalents, preferably 1 to 3 equivalent(s), to the compound of Formula (IVE). The reaction time is from 10 minutes to 10 days, preferably from 1 to 4 day(s). The reaction temperature is from -78°C to the heat-reflux temperature, preferably from -10 to 50°C.

When another protecting group such as tert-butyldimethylsilyl, triethylsilyl, diethylisopropylsilyl, trimethylsilyl, triisopropylsilyl, di-tert-butylmethylsilyl or diphenylmethylsilyl is used for protection, the deprotection can be done, for example, by fluorine anion or acid treatment. Examples of the fluorine anion include tetrabutylammoniumfluoride, hydrogen fluoride, potassium fluoride, pyridinium hydrogen fluoride and the like. As the acid, the above-mentioned organic acids, inorganic acids and the like can be used, and preferred are acetic acid, formic acid, trifluoroacetic acid, PPTS, camphorsulfonic acid and the like. The solvent for use in the reaction is not particularly limited, but it is desirably a solvent which does not readily react with the starting material, such as the above-mentioned inert solvents. Preferably used are tetrahydrofuran, diethylether, water and the like. The fluorine anion and the acid to be used in the reaction can be used in an amount of 1 to 5 equivalent(s) and 0.5 to 5 equivalents, respectively, preferably 1 to 4 equivalent(s) and 0.5 to 3 equivalent(s), respectively, to the compound of Formula (IVE). The reaction time is from 10 minutes to 30 hours, preferably from 1 to 2 hour(s). The reaction temperature is from -78°C to the heat-reflux temperature, preferably from -10 to 50°C.

### Production Process F

Among the compounds of Formula (1) of the invention, a thiourethane derivative can be synthesized through the following reaction route with the compound of Formula (IIE) as a starting compound.

### Production Process F

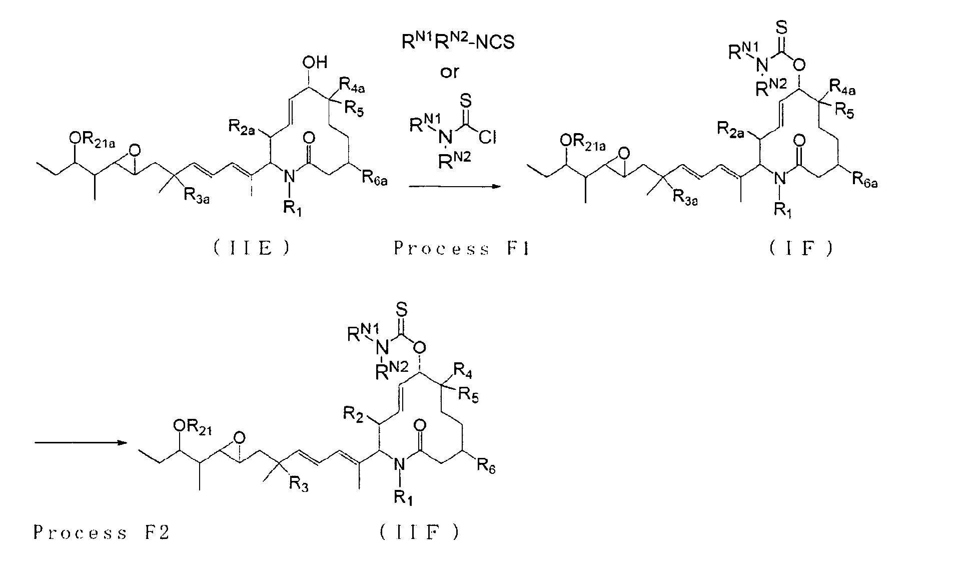

In the formula, when R₃, R₄ and R₆ are each a hydroxyl group, R₃ₐ, R₄ₐ and R₆ₐ each represent O-P wherein P is a protecting group; R₂₁ₐ is a protecting group; and R^{F} is a C₆₋₁₄ aryl group optionally having a substituent(s).

Process F1 is a process for synthesizing the compound of Formula (IF) with the use of thioisocyanate or thiocarbamoylchloride in place of isocyanate. This process can be done by treating the compound of Formula (IIE) with isothiocyanate or thiocarbamoylchloride in an inert solvent in the presence of a base or bis(tributyltin)oxide.

The isothiocyanate to be used is not particularly limited, and examples thereof include ethylisothiocyanate, methylisothiocyanate, phenylisothiocyanate, benzylisothiocyanate, allylisothiocyanate, 2-(N,N-dimethylamino)ethylisothiocyanate, 2-(N,N-diethylamino)ethylisothiocyanate, 3-(N,N-dimethylamino)propylisothiocyanate, 3-(N,N-diethylamino)propylisothiocyanate, 2-(morpholin-4-yl)ethylisothiocyanate, 2-(piperidin-1-yl)ethylisothiocyanate, 2-(pyrrolidin-1-yl)ethylisothiocyanate and the like.

The thiocarbamoylchloride to be used is not particularly limited, and examples thereof include N,N-dimethylthiocarbamoylchloride, N-phenyl-N-methylthiocarbamoylchloride, (morpholin-4-yl)thiocarbamoylchloride, (4-methylpiperazin-1-yl)thiocarbamoylchloride, (4-methylhomopiperazin-1-yl)thiocarbamoylchloride and the like.

As the base to be used, the above-mentioned organic bases, inorganic bases and the like can be mentioned, and preferred are diisopropylethylamine, 4-dimethylaminopyridine, triethylamine, pyridine, 2,6-lutidine, sodium hydride and the like. The solvent for use in the reaction is not particularly limited, but it is desirably a solvent which does not readily react with the starting material, such as the above-mentioned inert solvents. Preferred are tetrahydrofuran, dichloromethane, N,N-dimethylformamide, toluene and the like. The base or bis(tributyltin)oxide and isothiocyanate or thiocarbamoylchloride to be used in the reaction can be used in an amount of 1 to 5 equivalent(s) and 1 to 10 equivalent(s), respectively, preferably 1 to 3 equivalent(s) and 2 to 5 equivalents, respectively, to the compound of Formula (IIE). The reaction time is from 10 minutes to 72 hours, preferably from 1 to 24 hour(s). The reaction temperature is from -78°C to the heat-reflux temperature, preferably from -10 to 70°C.

Subsequently, by removing the protecting group of a hydroxyl group in the same manner as in Process E5, the thiourethane derivative of Formula (IIF) which is the compound of Formula (1) of the invention can be synthesized.

For the compound of Formula (25) used in Production Processes E and F, a compound synthesized according to Production Process A and a method pursuant thereto can be used. Particularly preferred are compounds (P15), (P16), (P36) and the like which will be mentioned in Examples to be described later.

According to the above-mentioned production methods, particularly, compounds in Tables 1 and 2 below can be synthesized.

**TABLE 1**

| | | | | | | |
|---|---|---|---|---|---|---|
| 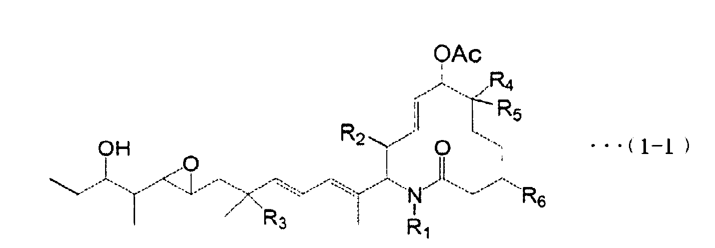 | | | | | | |

| | R₃ | R₁ | R₂ | R₄ | R₅ | R₆ |
|---|---|---|---|---|---|---|
| I-Ia.1 | OH | H | H | H | H | H |
| I-Ia.2 | OH | CH₃ | H | H | H | H |
| I-Ia.3 | OH | C₂H₅ | H | H | H | H |
| I-Ia.4 | OH | n-C₃H₇ | H | H | H | H |
| I-Ia.5 | OH | iso-C₃H₇ | H | H | H | H |
| I-Ia.6 | H | H | H | H | H | H |
| I-Ia.7 | H | CH₃ | H | H | H | H |
| I-Ia.8 | H | C₂H₅ | H | H | H | H |
| I-Ia.9 | H | n-C₃H₇ | H | H | H | H |
| I-Ia.10 | H | iso-C₃H₇ | H | H | H | H |
| I-Ia.11 | OH | H | CH₃ | H | H | H |
| I-Ia.12 | OH | CH₃ | CH₃ | H | H | H |
| I-Ia.13 | OH | C₂H₅ | CH₃ | H | H | H |
| I-Ia.14 | OH | n-C₃H₇ | CH₃ | H | H | H |
| I-Ia.15 | OH | iso-C₃H₇ | CH₃ | H | H | H |
| I-Ia.16 | H | H | CH₃ | H | H | H |
| I-Ia.17 | H | CH₃ | CH₃ | H | H | H |
| I-Ia.18 | H | C₂H₅ | CH₃ | H | H | H |
| I-Ia.19 | H | n-C₃H₇ | CH₃ | H | H | H |
| I-Ia.20 | H | iso-C₃H₇ | CH₃ | H | H | H |
| I-Ia.21 | OH | H | C₂H₅ | H | H | H |
| I-Ia.22 | OH | CH₃ | C₂H₅ | H | H | H |
| I-Ia.23 | OH | C₂H₅ | C₂H₅ | H | H | H |
| I-Ia.24 | OH | n-C₃H₇ | C₂H₅ | H | H | H |
| I-Ia.25 | OH | iso-C₃H₇ | C₂H₅ | H | H | H |
| I-Ia.26 | H | H | C₂H₅ | H | H | H |
| I-Ia.27 | H | CH₃ | C₂H₅ | H | H | H |
| I-Ia.28 | H | C₂H₅ | C₂H₅ | H | H | H |
| I-Ia.29 | H | n-C₃H₇ | C₂H₅ | H | H | H |
| I-Ia.30 | H | iso-C₃H₇ | C₂H₅ | H | H | H |
| I-Ia.31 | OH | H | n-C₃H₇ | H | H | H |
| I-Ia.32 | OH | CH₃ | n-C₃H₇ | H | H | H |
| I-Ia.33 | OH | C₂H₅ | n-C₃H₇ | H | H | H |
| I-Ia.34 | OH | n-C₃H₇ | n-C₃H₇ | H | H | H |
| I-Ia.35 | OH | iso-C₃H₇ | n-C₃H₇ | H | H | H |
| I-Ia.36 | H | H | n-C₃H₇ | H | H | H |
| I-Ia.37 | H | CH₃ | n-C₃H₇ | H | H | H |
| I-Ia.38 | H | C₂H₅ | n-C₃H₇ | H | H | H |
| I-Ia.39 | H | n-C₃H₇ | n-C₃H₇ | H | H | H |
| I-Ia.40 | H | iso-C₃H₇ | n-C₃H₇ | H | H | H |
| I-Ia.41 | OH | H | iso-C₃H₇ | H | H | H |
| I-Ia.42 | OH | CH₃ | iso-C₃H₇ | H | H | H |
| I-Ia.43 | OH | C₂H₅ | iso-C₃H₇ | H | H | H |
| I-Ia.44 | OH | n-C₃H₇ | iso-C₃H₇ | H | H | H |
| I-Ia.45 | OH | iso-C₃H₇ | iso-C₃H₇ | H | H | H |
| I-Ia.46 | H | H | iso-C₃H₇ | H | H | H |
| I-Ia.47 | H | CH₃ | iso-C₃H₇ | H | H | H |
| I-Ia.48 | H | C₂H₅ | iso-C₃H₇ | H | H | H |
| I-Ia.49 | H | n-C₃H₇ | iso-C₃H₇ | H | H | H |
| I-Ia.50 | H | iso-C₃H₇ | iso-C₃H₈ | H | H | H |

**TABLE 2**

| | | | | | | |
|---|---|---|---|---|---|---|
| 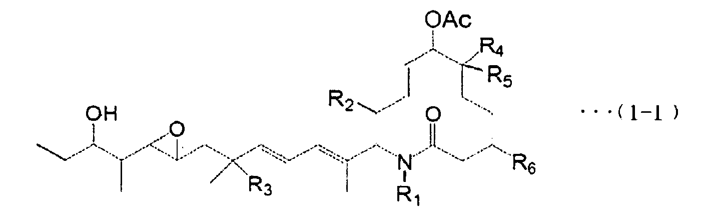 | | | | | | |

| | R₃ | R₁ | R₂ | R₄ | R₅ | R₆ |
|---|---|---|---|---|---|---|
| I-Ib.1 | OH | H | H | OH | CH₃ | OH |
| I-Ib.2 | OH | CH₃ | H | OH | CH₃ | OH |
| I-Ib.3 | OH | C₂H₅ | H | OH | CH₃ | OH |
| I-Ib.4 | OH | n-C₃H₇ | H | OH | CH₃ | OH |
| I-Ib.5 | OH | iso-C₃H₇ | H | OH | CH₃ | OH |
| I-Ib.6 | H | H | H | OH | CH₃ | OH |
| I-Ib.7 | H | CH₃ | H | OH | CH₃ | OH |
| I-Ib.8 | H | C₂H₅ | H | OH | CH₃ | OH |
| I-Ib.9 | H | n-C₃H₇ | H | OH | CH₃ | OH |
| I-Ib.10 | H | iso-C₃H₇ | H | OH | CH₃ | OH |
| I-I6.11 | OH | H | CH₃ | OH | CH₃ | OH |
| I-Ib.12 | OH | CH₃ | CH₃ | OH | CH₃ | OH |
| I-Ib.13 | OH | C₂H₅ | CH₃ | OH | CH₃ | OH |
| I-Ib.14 | OH | n-C₃H₇ | CH₃ | OH | CH₃ | OH |
| I-Ib.15 | OH | iso-C₃H₇ | CH₃ | OH | CH₃ | OH |
| I-Ib.16 | H | H | CH₃ | OH | CH₃ | OH |
| I-Ib.17 | H | CH₃ | CH₃ | OH | CH₃ | OH |
| I-Ib.18 | H | C₂H₅ | CH₃ | OH | CH₃ | OH |
| I-Ib.19 | H | n-C₃H₇ | CH₃ | OH | CH₃ | OH |
| I-Ib.20 | H | iso-C₃H₇ | CH₃ | OH | CH₃ | OH |
| I-Ib.21 | OH | H | C₂H₅ | OH | CH₃ | OH |
| I-Ib.22 | OH | CH₃ | C₂H₅ | OH | CH₃ | OH |
| I-Ib.23 | OH | C₂H₅ | C₂H₅ | OH | CH₃ | OH |
| I-Ib.24 | OH | n-C₃H₇ | C₂H₅ | OH | CH₃ | OH |
| I-Ib.25 | OH | iso-C₃H₇ | C₂H₅ | OH | CH₃ | OH |
| I-Ib.26 | H | H | C₂H₅ | OH | CH₃ | OH |
| I-Ib.27 | H | CH₃ | C₂H₅ | OH | CH₃ | OH |
| I-Ib.28 | H | C₂H₅ | C₂H₅ | OH | CH₃ | OH |
| I-Ib.29 | H | n-C₃H₇ | C₂H₅ | OH | CH₃ | OH |
| I-Ib.30 | H | iso-C₃H₇ | C₂H₅ | OH | CH₃ | OH |
| I-Ib.31 | OH | H | n-C₃H₇ | OH | CH₃ | OH |
| I-Ib.32 | OH | CH₃ | n-C₃H₇ | OH | CH₃ | OH |
| I-Ib.33 | OH | C₂H₅ | n-C₃H₇ | OH | CH₃ | OH |
| I-Ib.34 | OH | n-C₃H₇ | n-C₃H₇ | OH | CH₃ | OH |
| I-Ib.35 | OH | iso-C₃H₇ | n-C₃H₇ | OH | CH₃ | OH |
| I-Ib.36 | H | H | n-C₃H₇ | OH | CH₃ | OH |
| I-Ib.37 | H | CH₃ | n-C₃H₇ | OH | CH₃ | OH |
| I-Ib.38 | H | C₂H₅ | n-C₃H₇ | OH | CH₃ | OH |
| I-Ib.39 | H | n-C₃H₇ | n-C₃H₇ | OH | CH₃ | OH |
| I-Ib.40 | H | iso-C₃H₇ | n-C₃H₇ | OH | CH₃ | OH |
| I-Ib.41 | OH | H | iso-C₃H₇ | OH | CH₃ | OH |
| I-Ib.42 | OH | CH₃ | iso-C₃H₇ | OH | CH₃ | OH |
| I-Ib.43 | OH | C₂H₅ | iso-C₃H₇ | OH | CH₃ | OH |
| I-Ib.44 | OH | n-C₃H₇ | iso-C₃H₇ | OH | CH₃ | OH |
| I-Ib.45 | OH | iso-C₃H₇ | iso-C₃H₇ | OH | CH₃ | OH |
| I-Ib.46 | H | H | iso-C₃H₇ | OH | CH₃ | OH |
| I-Ib.47 | H | CH₃ | iso-C₃H₇ | OH | CH₃ | OH |
| I-Ib.48 | H | C₂H₅ | iso-C₃H₇ | OH | CH₃ | OH |
| I-Ib.49 | H | n-C₃H₇ | iso-C₃H₇ | OH | CH₃ | OH |
| I-Ib.50 | H | iso-C₃H₇ | iso-C₃H₈ | OH | CH₃ | OH |

The compound of Formula (1) of the invention or a salt thereof is effective as an agent for treating tumors (hereinafter, referred to as an antitumor agent) on the basis of its antitumor activity.

In the present specification, the term "treatment" refers to prevention or treatment, or both.

More specifically, the compound of Formula (1) of the invention or a salt thereof is effective as an antitumor agent, in particular, as an antitumor agent/cancer metastasis inhibitor against a solid tumor or a hematologic tumor. As a solid tumor, there is, for example, pancreatic cancer, gastric cancer, colon cancer, breast cancer, prostate cancer, lung cancer, kidney cancer, brain tumor, head and neck tumor, esophageal cancer, skin cancer, liver cancer, uterine cancer, cervical cancer, bladder cancer, thyroid cancer, testicular tumor, choriocarcinom, osteosarcoma, soft tissue sarcoma, ovarian cancer and the like. In particular, the invention is preferably used against lung cancer, brain tumor, breast cancer, prostate cancer, ovarian cancer, colon cancer or skin cancer. Here, the hematologic tumor includes leukemia.

When administering the compound of Formula (1) of the invention or a salt thereof as an agent for treatment/prevention of various diseases, it may be administered orally in the form of a tablet, powder, granulars, a capsules or syrups, or parenterally in the form of a spray, a suppository, an injection, an external preparation or drips. The dosage varies significantly depending on the severity of symptoms, age or type of hepatic disease. However, the usual dosage for an adult is about 1 to 100 mg per day, and the administration can be conducted once or several times a day.

Upon forming a formulation, the formulation can be prepared according to a common method with the use of a known formulation carrier. Specifically, when preparing the compound of Formula (1) of the invention or a salt thereof as an injection, a pH regulator, buffer, a stabilizer, a solubilizer and the like are added to a principal agent as necessary and prepared as a subcutaneous injection, an intramuscular injection, an intraarticular injection or an intravenous injection according to a common method. When preparing a solid preparation for oral administration, a binder, a disintegrant, a lubricant, a colorant, a flavoring agent and the like are added as necessary, in addition to an excipient, to a principal agent, and then prepared as a tablet, a coated tablet, a granular preparation, power, a capsule or the like according to a common method. It is also fine to coat the tablet and granular preparation suitably with a sugar coating, a gelatin coating or any other coatings, as necessary.

### EXAMPLES

Next, the invention will be described in more detail with Examples, but the invention is not limited by the examples shown below.

### Example 1

Synthesis Example of (2S, 3S, 4E)-2-((1E, 3E, 5R)-5-hydroxy-6-{(2R, 3R)-3-[(1R,2S)-2-hydroxy-1-methylbutyl]oxiran-2-yl}-1,5-dimethylhexa-1,3-dien-1-yl)-3-methyl-12-oxaazacyclododec-4-en-6-yl acetate (P15)

### Process (1-1): Synthesis of ethyl(3R)-4-{[tert-butyl(dimethyl)silyl]oxy}-N-(4-methoxyphenyl)-L-valinate (P1)

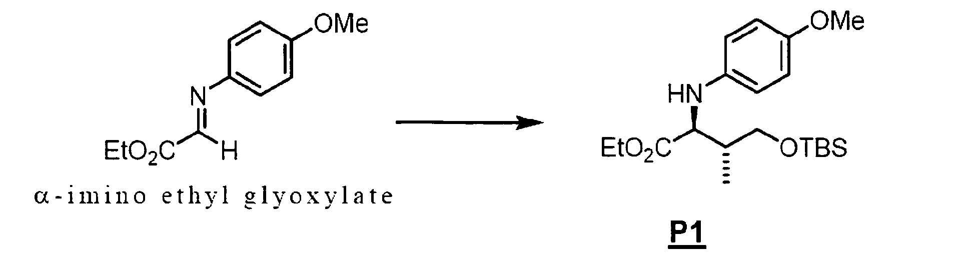

This process was carried out with reference to Mitsumori, S. and 5 others, Journal of the American Chemical Society, 2006, Vol. 128, p1040-1041.

α-imino ethyl glyoxylate and (3R,5R)-5-methyl-pyrrolidine-3-carboxylic acid used in this process were prepared according to a method described in the above reference document.

Propionaldehyde (2.09 ml, 29 mmol) and (3R, 5R)-5-methyl-pyrrolidine-3-carboxylic acid (93.6 mg, 0.725 mmol) were added to a DMSO (40ml) solution of α-imino ethyl glyoxylate (3g, 14.5 mmol) at room temperature. The reaction solution was stirred for 1 hour at the same temperature. To the reaction solution, methanol (40 ml) was added, and sodium borohydride (548 mg, 14.5 mmol) was added thereto at 0°C. After stirring the reaction solution at the same temperature for 30 minutes, a saturated aqueous solution of ammonium chloride was added thereto, and extraction with ethyl acetate was performed. The organic layer was washed with saturated brine, dried over anhydrous magnesium sulfate, and concentrated under reduced pressure.

The thus obtained residue was dissolved in DMF (40 ml), and imidazole (1.98g, 29 mmol) and tert-butyldimethylsilyl chloride (2.62g, 17.4 mmol) were added thereto. The mixture was stirred for 10.5 hours at room temperature. To the reaction solution, a saturated aqueous solution of ammonium chloride was added, and extracted with diethyl ether. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 20:1 → 5:1) to obtain the title compound (3.6 g) as a colorless oil.

400MHz ¹H-NMR (CDCl₃) δ (ppm) 0.06 (s, 3H), 0.07 (s, 3H), 0.93 (s, 9H), 0.98 (d, J = 7.1 Hz, 3H), 1.24 (t, J = 7.1 Hz, 3H), 2.09-2.19 (m, 1H), 3.64-3.71 (m, 2H), 3.73 (s, 3H), 4.02-4.09 (brs, 1H), 4.15 (d, J = 7.1Hz, 1H), 4.19 (d, J = 7.1 Hz, 1H), 4.26-4.34 (brs, 1H), 6.62 (d, J = 8.8 Hz, 2H), 6.75 (d, J = 8.8 Hz, 2H); MS m/z 403.91 (M + Na)⁺

### Process (1-2): Synthesis of (3R)-4-{[tert-butyl(dimethyl)silyl]oxy}-N-methoxy-N2-(4-methoxyphenyl)-N-methyl-L-valinamide (P2)

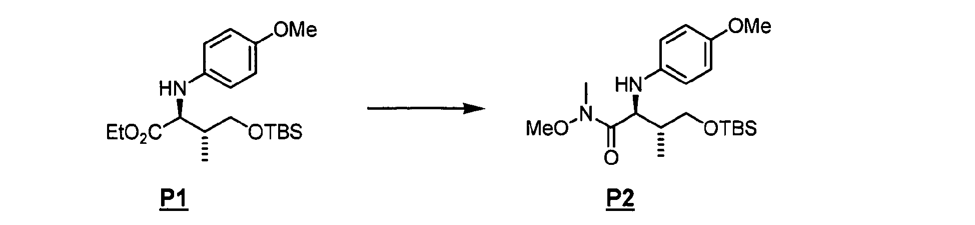

This process was carried out with reference to Williams, J. M. and 4 others, Tetrahedron Letters, 1995, Vol. 36, Issue No. 31, p5461-5464.

To a THF solution (4 ml) of ethyl(3R)-4-{[tert-butyl(dimethyl)silyl]oxy}-N-(4-methoxyphenyl)-L-valinate (310 mg, 0.812 mmol) and N,O-dimethylhydroxylamine hydrochloride (122 mg, 1.22 mmol), a solution of 1M lithium bis(trimethylsilyl)amide in THF (2.44 ml) was added while stirring at -10°C under a nitrogen atmosphere. The reaction solution was stirred at the same temperature for 1.5 hours. A saturated aqueous solution of ammonium chloride was added to the reaction solution, and extraction with ethyl acetate was performed. The organic layer was washed with saturated brine, dried over anhydrous magnesium sulfate, and concentrated under reduced pressure. The obtained residue was purified by silica gel column chromatography (Kanto Chemical, trade name: Silica gel 60N, granular, 0.040 to 0.100 mm; n-heptane : ethyl acetate = 6:1) to obtain the title compound (283 mg) as a yellow oil.

400MHz ¹H-NMR (CDCl₃) δ (ppm) 0.05 (s, 3H), 0.05 (s, 3H), 0.94 (s, 9H), 1.00 (d, J = 7.2 Hz, 3H), 1.87-1.98 (m, 1H), 3.17 (s, 3H), 3.59-3.67 (m, 1H), 3.61 (s, 3H), 3.73 (s, 3H), 3.85 (dd, J = 4.4, 9.6 Hz, 1H), 4.05-4.16 (brs, 1H), 4.41-4.54 (brs, 1H), 6.69 (d, J = 9.2 Hz, 2H), 6.73 (d, J = 9.2 Hz, 2H); MS m/z 418.89 (M + Na)⁺

### Process (1-3): Synthesis of ethyl (2E, 4S, 5R)-6-{[tert-butyl(dimethyl)silyl]oxy}-4-[(4-methoxyphenyl)amino]-3,5-dimethylhex-2-enoate (P3)

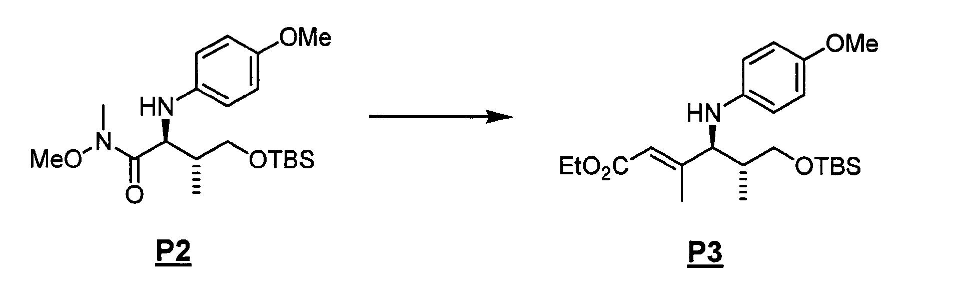

To a solution of (3R)-4-{[tert-butyl(dimethyl)silyl]oxy}-N-methoxy-N2-(4-methoxyphenyl)-N-methyl-L-valinamide (20g, 50.4 mmol) in THF(500 ml), a diethylether solution (134 ml) of 1.13 M methyllithium was added dropwise at -78°C under a nitrogen atmosphere. The reaction solution was stirred at the same temperature for 30 minutes. A saturated aqueous solution of ammonium chloride was added to the reaction solution, and then diluted with ethyl acetate. The organic layer was washed with water and saturated brine. The organic layer was dried over anhydrous magnesium sulfate, and concentrated under reduced pressure, to obtain a brown solid (17.5g).

5g of the obtained brown solid was dissolved in toluene (100 ml). To this solution, (carbometoxymethylene)triphenylphosphorane (30g, 86.1 mmol) was added, and the mixture was heated to reflux for 1.5 days. The reaction solution was cooled to 0°C, and the resulting white solid was removed by filtration. Thereafter, the filtrate was concentrated under reduced pressure.

The thus obtained residue was dissolved in methanol (50 ml), and sodium borohydride (537 mg, 14.2 mmol) was added thereto at 0°C. The mixture was stirred at the same temperature for 1 hour. A saturated aqueous solution of ammonium chloride was added to the reaction solution, and extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 30:1 → 10:1 → 4:1) to obtain the title compound (3.81 g, E:Z = 7:1) as a yellow oil.

400MHz ¹H-NMR (CDCl₃) (main product data) δ (ppm) 0.06 (s, 3H), 0.07 (s, 3H), 0.94 (s, 9H), 1.05 (d, J = 7.0 Hz, 3H), 1.26 (t, J = 7.0 Hz, 3H), 1.84-1.96 (m, 1H), 2.13 (s, 3H), 3.58 (dd, J = 4.4, 10.1 Hz, 1H), 3.64 (d, J = 5.7 Hz, 1H), 3.72 (s, 3H), 3.76 (d, J = 3.1 Hz, 1H), 4.07-4.19 (m, 2H), 4.76-4.90 (brs, 1H), 5.95 (s, 1H), 6.41 (d, J = 8.8 Hz, 2H), 6.73 (d, J = 8.8 Hz, 2H); MS m/z 422.00 (M + H)⁺

### Process (1-4): Synthesis of N-[(1S, 2E)-4-(benzyloxy)-1-((1R)-2{[tert-butyl(dimethyl)silyl]oxy}-1-methylethyl)-2-methylbut -2-en-1-yl]-4-methoxyaniline (P4)

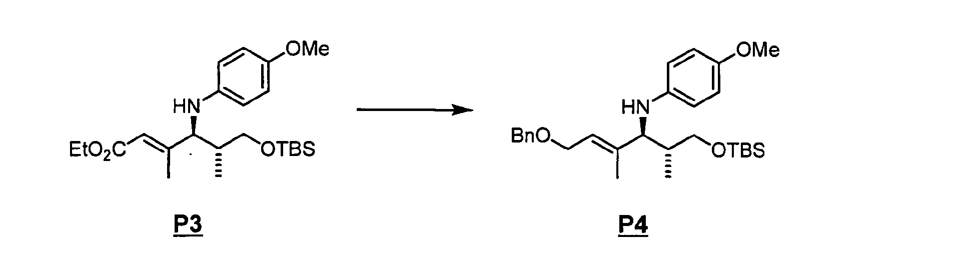

To a dichloromethane solution (400 ml) of ethyl(2E, 4S, 5R)-6-{[tert-butyl(dimethyl)silyl]oxy}-4-[(4-methoxyphenyl)amino]-3,5-dimethylhex-2-enoate (18.1g, 43.1 mmol), a 0.97 M n-hexane solution (112 ml) of diisobutylaluminum hydride was added dropwise at -78°C under a nitrogen atmosphere, and this reaction solution was stirred at the same temperature for 30 minutes. After ice cooling the reaction solution, saturated aqueous solution of Rochelle salt was added thereto. After vigorously stirring the reaction solution for 1 hour, extraction with ethyl acetate was performed. The organic layer was washed with saturated brine, dried over anhydrous magnesium sulfate, and concentrated under reduced pressure.

The thus obtained residue was dissolved in THF (400 ml), and under a nitrogen atmosphere, sodium hydride (60%, 4.32g, 108 mmol) was added thereto at 0°C, and the mixture was stirred at the same temperature for 30 minutes. To this reaction solution, tetra-n-butylammonium iodide (3.18g, 8.62 mmol) and benzylbromide (7.69 ml, 64.7 mmol) were added dropwise. After stirring the reaction solution at room temperature for 12 hours, sodium hydride (1.73g, 43.2 mmol) and benzylbromide (5.13 ml, 43.1 mmol) were further added thereto and stirred at room temperature for 7 hours. To the reaction solution, water was added, and extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 30:1 → 15:1 → 2:1) to obtain the title compound (16.3g) as a yellow oil.

400MHz ¹H-NMR (CDCl₃) δ (ppm) 0.05 (s, 3H), 0.06 (s, 3H), 0.93 (s, 9H), 0.98 (d, J = 6.8 Hz, 3H), 1.57 (s, 3H), 1.74-1.85 (m, 1H), 3.57 (d, J = 7.2 Hz, 1H), 3.64 (dd, J = 4.8, 10.0 Hz, 1H), 3.70 (s, 3H), 3.73 (dd, J = 3.6, 10.0 Hz, 1H), 4.09 (d, J = 6.4 Hz, 2H), 4.39 (d, J = 11.6 Hz, 1H), 4.40 (d, J = 11.6 Hz, 1 H), 4.53-4.62 (brs, 1H), 5.64 (t, J = 6.8 Hz, 1H), 6.48 (d, J = 8.8 Hz, 2H), 6.72 (d, J = 8.8 Hz, 2H), 7.22-7.38 (m, 5H); MS m/z 492.03 (M + Na)⁺

### Process (1-5): Synthesis of (2R, 3S, 4E)-6-(benzyloxy)-3-[(4-methoxyphenyl)amino]-2,4-dimethylhex-4-en-1-ol (P5)

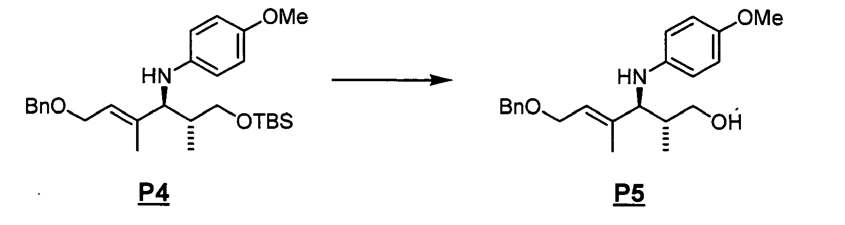

To a solution ofN[(1S, 2E)-4-(benzyloxy)-1-((R)-2-{[tert-butyl(dimethyl)silyl]oxy}-1-methylethyl)-2-methylb ut-2-en-1-yl]-4-methoxyaniline (14.8g, 31.4 mmol) in THF(150 ml), a 1M THF solution of tetra-n-butylammoniumfluoride (62.6 ml) was added. The reaction solution was stirred at room temperature for 1 hour. After diluting this reaction solution by adding water, extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane: ethyl acetate = 2.5:1 → 1:1) to obtain the title compound (10.8g) as a colorless oil.

400MHz ¹H-NMR (CDCl₃) δ (ppm) 0.86 (d, J = 6.8 Hz, 3H), 1.51 (s, 3H), 1.83-1.95 (m, 1H), 2.05 (s, 1H), 3.58-3.68 (brs, 1H), 3.60 (d, J = 9.3 Hz, 1H), 3.69 (s, 3H), 3.70-3.79 (m, 2H), 3.96-4.08 (m, 2H), 4.29 (d, J = 11.7 Hz, 1H), 4.35 (d, J = 11.7 Hz, 1H), 5.51 (t, J = 6.4 Hz, 1 H), 6.64 (d, J = 9.0 Hz, 2H), 6.74 (d, J = 9.0 Hz, 2H), 7.19-7.35 (m, 5H); MS m/z 377.88 (M + Na)⁺

### Process (1-6): Synthesis oftert-butyl{(1S, 2E)-4-(benzyloxy)-1-[(1R)-2-hydroxy-1-methylethyl]-2-methylbut-2-en-1-yl}carbamate (P6)

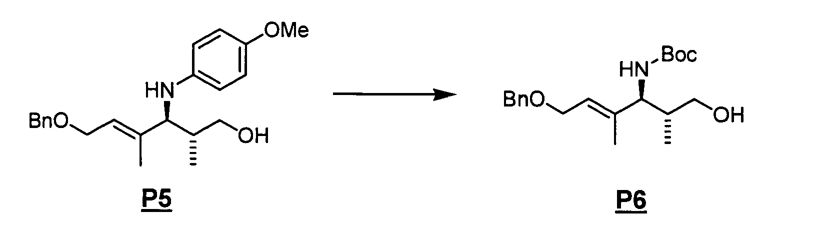

This process was carried out with reference to Verkade, J.M.M and 5 others, Tetrahedron Letters, 2006, Vol. 47, Issue No. 46, p8109-8113.

To a mixed solution of acetonitrile-water (2:1, 315 ml) of (2R, 3S, 4E)-6-(benzyloxy)-3-[(4-methoxyphenyl)amino]-2,4-dimethylhex-4-en-1-ol (10.5g, 29.6 mmol), orthoperiodic acid (6.77g, 29.6mmol) and a 1M aqueous solution of sulfuric acid (59.2 ml) were added. The reaction solution was stirred at room temperature for 1.5 hours. The reaction solution was diluted with water (200 ml) and an aqueous layer was washed with diethyl ether. To the aqueous layer, dichloromethane (500 ml) was added, and di-tert-butyldicarbonate (25.8g, 119 mmol) and potassium carbonate (40.9g, 296 mmol) were then added thereto. The mixture was vigorously stirred at room temperature for 8 hours. The reaction solution was extracted with dichloromethane and the obtained organic layer was combined, followed by washing with saturated brine. The organic layer was dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 2:1 → 1:1) to obtain the title compound (6.61 g) as a colorless oil.

400MHz ¹H-NMR (CDCl₃) δ (ppm) 0.98 (d, J = 7.0 Hz, 3H), 1.45 (s, 9H), 1.64 (s, 3H), 1.59-1.74 (m, 1H), 2.56-2.59 (brs, 1H), 3.42-3.56 (brs, 1H), 3.68-3.78 (brd, J = 10.1 Hz, 1H), 3.92 (t, J = 8.1 Hz, 1H), 4.08 (d, J = 6.4 Hz, 2H), 4.52 (s, 2H), 4.95 (d, J = 8.4 Hz, 1H), 5.65 (t, J = 5.6 Hz, 1H), 7.23-7.30 (m, 5H); MS m/z 371.90 (M + Na)⁺

### Process (1-7): Synthesis of tert-butyl{(1S,2E)-4-(benzyloxy)-1-[(1S, 2E)-3-iodo-1-methylprop-2-en-1-yl]-2-methylbut-2-en-1-yl}carbamate (P7)

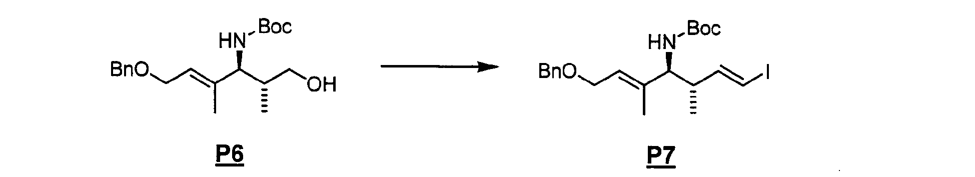

This process was carried out with reference to Takai, K. and 2 others, Journal of the American Chemical Society, 1986, Vol. 108, p7408-7410.

In the process, anhydrous THF was distilled from benzophenone ketyl just before it was used. Also, chromium (II) chloride was dried for 3 hours at 200°C under reduced pressure using a vacuum pump just before it was used.

To a dichloromethane solution (8 ml) of tert-butyl {(1S, 2E)-4-(benzyloxy)-1-[(1R)-2-hydroxy-1-methylethyl]-2-methylbut-2-en-1-yl}carbamate (300 mg, 0.858 mmol), a Dess-Martin reagent (436 mg, 1.03 mmol) was added, and the reaction solution was stirred at room temperature for 10 minutes. The reaction solution was diluted with ethyl acetate, and washed sequentially with saturated aqueous solution of sodium bicarbonate containing sodium sulfite and saturated brine. The organic layer was dried over anhydrous magnesium sulfate, and concentrated under reduced pressure.

The thus obtained residue and the anhydrous THF solution (10 ml) of iodoform (1.01g, 2.57 mmol) were added dropwise to an anhydrous THF solution (10 ml) of chromium (II) chloride under an argon atmosphere at 0°C. Thereafter, the reaction solution was stirred at the same temperature for 30 minutes, and then further stirred at room temperature for 5 hours. After diluting the reaction solution by adding water, extraction with ethyl acetate was performed. The organic layer was washed sequentially with water and saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 10:1) to obtain the title compound (179 mg) as a yellow oil.

400MHz ¹H-NMR (CDCl₃) δ (ppm) 1.02 (d, J = 6.8 Hz, 3H), 1.44 (s, 9H), 1.61 (s, 3H), 2.34-2.51 (m, 1H), 3.81-3.98 (brs, 1H), 4.09 (d, J = 6.2 Hz, 2H), 4.41-4.58 (m, 1H), 4.50 (s, 2H), 5.55 (t, J = 6.2 Hz, 1H), 6.10 (d, J = 14.5 Hz, 1H), 6.43 (dd, J = 7.7, 14.5 Hz, 1H), 7.23-7.40 (m, 5H); MS m/z 493.92 (M + Na)⁺

### Process (1-8): Synthesis of ethyl 7-hydroxyheptanoate (P8)

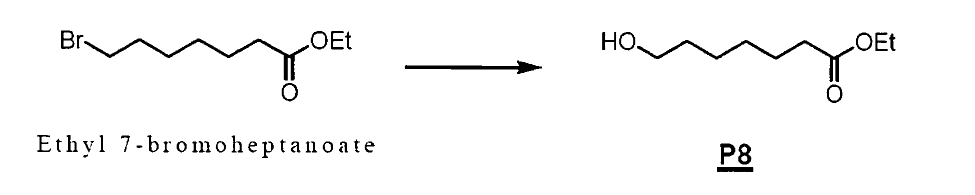

This process was carried out with reference to Alexander, J. and 2 others, Synthetic Communications, 1995, Vol. 25, Issue No. 23, p3875-3881).

To a mixed solution of ethyl 7-bromoheptanoate (25g, 105 mmol) and formic acid (12.1g, 263 mmol), triethylamine (36.7 ml, 263 mmol) was added dropwise at 0°C under a nitrogen atmosphere, and the reaction solution was stirred at 50°C for 21 hours. After diluting the reaction solution by adding water, extraction with ethyl acetate was performed. The organic layer was washed sequentially with 1N hydrochloric acid, saturated aqueous solution of sodium bicarbonate, water and saturated brine. The organic layer was dried over anhydrous magnesium sulfate and concentrated under reduced pressure to obtain the title compound (17.9g) as a colorless oil.

400MHz ¹H-NMR (CDCl₃) δ (ppm) 1.26 (t, J = 7.1 Hz, 3H), 1.30-1.44 (m, 4H), 1.50-1.74 (m, 4H), 2.30 (t, J = 7.3 Hz, 2H), 3.64 (t, J = 6.6 Hz, 2H), 3.67 (s, 1H), 4.13 (q, J = 7.1 Hz, 2H); MS m/z 197.01 (M + Na)⁺

### Process (1-9): Synthesis of 7-{[tert-butyl(dimethyl)silyl]oxy}heptanoate (P9)

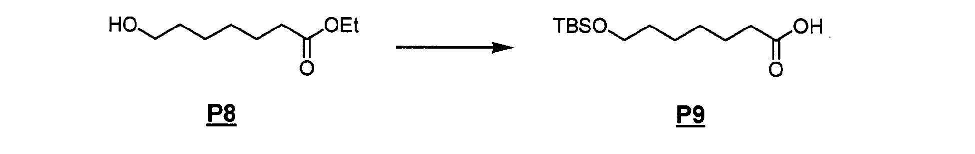

Imidazole (2.93g, 43.1 mmol) and tert-butyldimethylsilyl chloride (5.2g, 34.5 mmol) were added to a DMF solution (50 ml) of ethyl 7-hydroxyheptanoate (5g, 28.7 mmol) at room temperature. The reaction solution was stirred at the same temperature for 30 minutes. After diluting the reaction solution by adding water, extraction with diethyl ether was subjected. The organic layer was washed with saturated brine, dried over anhydrous magnesium sulfate, and concentrated under reduced pressure.

The thus obtained residue was dissolved in a mixed solution of THF-methanol-water (2:2:1, 150 ml), and anhydrous lithium hydroxide (1.37g, 57.3 mmol) was added thereto. The reaction solution was stirred at room temperature for 1.5 hours. The reaction solution was diluted by adding water, and then washed with dichloromethane. An aqueous layer was acidified by adding 1N hydrochloric acid, and extracted with ethyl acetate. The organic layer was washed with saturated brine, dried over anhydrous magnesium sulfate, and concentrated under reduced pressure, to obtain the title compound (7.41 g) as a colorless oil.

400MHz ¹H-NMR (CDCl₃) δ (ppm) 0.05 (s, 6H), 0.89 (s, 9H), 1.29-1.42 (m, 4H), 1.46-1.57 (m, 2H), 1.58-1.70 (m, 2H), 2.35 (t, J = 7.5 Hz, 2H), 3.60 (t, J = 6.6 Hz, 2H); MS m/z 282.85 (M+Na)⁺

### Process (1-10): Synthesis of N-{(1S,2E)-4-(benzyloxy)-1-[(1S, 2E)-3-iodo-1-methylprop-2-en-1-yl]-2-methylbut-2-en-1-yl}-7-hydroxyheptaneamide (P10)

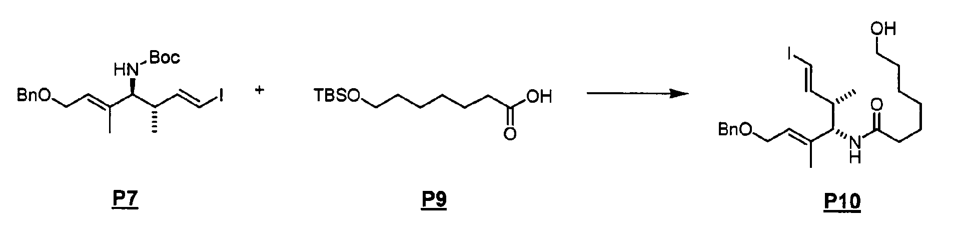

To a dichloromethane solution (15 ml) of tert-butyl {(1S, 2E)-4-(benzyloxy)-1-[(1S, 2E)-3-iodo-1-methylprop-2-en-1-yl]-2-methylbut-2-en-1-yl}carbamate (1.44g, 3.03 mmol), trifluoroacetic acid (2.25 ml, 30.3 mmol) was added. This reaction solution was stirred at room temperature for 3 hours, and then concentrated under reduced pressure.

The thus obtained residue and 7-{[tert-butyl(dimethyl)silyl]oxy}heptanoate (1.09g, 4.19 mmol) were dissolved in DMF (20 ml). To this solution, triethylamine (1.17 ml, 8.35 mmol), 1-hydroxybenzotriazole (850 mg, 6.26 mmol) and 1-ethyl-3-(3-dimethylaminopropyl)carbodiimide hydrochloride (1.2g, 6.26 mmol) were added sequentially at room temperature, and the mixture was stirred at the same temperature for 1.5 hours. After diluting the reaction solution by adding water, extraction with diethyl ether was performed. The organic layer was washed sequentially with 0.5N hydrochloric acid, saturated aqueous solution of sodium bicarbonate and saturated brine. The organic layer was dried over anhydrous magnesium sulfate, and concentrated under reduced pressure.

To the solution prepared by dissolving the obtained residue in THF (34 ml), a 1M THF solution of tetra-n-butylammonium fluoride (10.4 ml) was added at room temperature, and the mixture was stirred for 12 hours. After diluting the reaction solution by adding water, extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 1:2) to obtain the title compound (1.34g) as a yellow oil.

400MHz ¹H-NMR (CDCl₃) δ (ppm) 1.02 (d, J = 6.6 Hz, 3H), 1.28-1.46 (m, 6H), 1.48-1.73 (m, 2H), 1.62 (s, 3H), 2.19 (t, J = 7.3 Hz, 2H), 2.42-2.56 (m, 1H), 3.62 (t, J = 6.4 Hz, 2H), 4.08 (d, J = 6.2 Hz, 2H), 4.22-4.33 (m, 1H), 4.50 (s, 2H), 5.35 (d, J = 8.8 Hz, 1H), 5.55 (t, J = 6.2 Hz, 1H), 6.10 (d, J = 14.5 Hz, 1H), 6.42 (dd, J = 7.9, 14.5 Hz, 1H), 7.21-7.42 (m, 5H); MS m/z 522.16 (M + Na)⁺

### Process (1-11): Synthesis of (2S, 3S, 4E)-2-[(1E)-3-(benzyloxy)-1-methylprop-1-en-1-yl]-3-methyl-12-oxaazacyclododec-4-e n-6-yl acetate (P11)

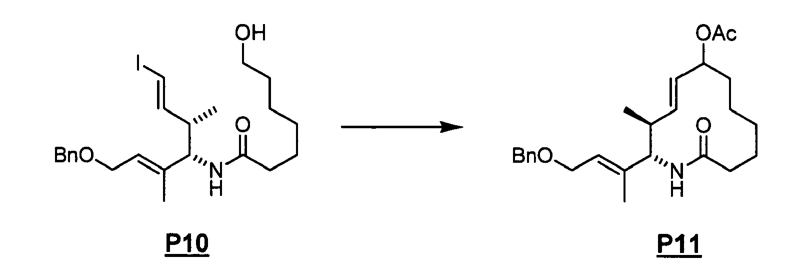

This process was carried out with reference to literatures, Furstner, A., Chemical Reviews, 1999, Vol. 99, p 991-1045; Stamos, D.P. and 3 others, Tetrahedron Letters, 1997, Vol. 38, Issue No. 36, p6355-6358; Pilli, R.A. and 3 others, The Journal of organic Chemistry, 1998, Vol. 63, p 7811-7819.

In the process, DMF was subjected to freezing degasification just before it was used. Also, chromium (II) chloride and nickel (II) chloride were dried for 5 hours at 200°C under reduced pressure using a vacuum pump just before they were used.

To a dichloromethane solution (31.6 ml) of N-{(1S, 2E)-4-(benzyloxy)-1-[(1S, 2E)-3-iodo-1-methylprop-2-en-1-yl]-2-methylbut-2-en-1-yl}-7-hydroxyheptaneamide (700 mg, 1.4 mmol), a Dess-Martin reagent (893 mg, 2.1 mmol) was added, and the mixture was stirred at room temperature for 1 hour. The reaction solution was diluted with ethyl acetate, and washed sequenatilly with saturated aqueous solution of sodium bicarbonate containing sodium sulfite and saturated brine. The organic layer was dried over anhydrous magnesium sulfate, and concentrated under reduced pressure.

The solution prepared by dissolving the obtained residue in DMF (17 ml) was added dropwise to a DMF solution (170 ml) of nickel (II) chloride (11.3 mg, 87.1 µmol) and chromium (II) chloride (1.07g, 8.71 mmol) at 0°C under argon atmosphere. The mixture was stirred at room temperature for 3 hours. To this reaction solution, a solution (10 ml) prepared by dissolving serine in saturated aqueous solution of sodium bicarbonate to give 1 M concentration was added, and then the reaction solution was stirred until it gives a blackish-purple color. After dilution the reaction solution by adding water, extraction with ethyl acetate was performed. The organic layer was washed with saturated brine, dried over anhydrous magnesium sulfate, and concentrated under reduced pressure.

The thus obtained residue was dissolved in dichloromethane (20 ml), and triethylamine (977 µl, 7 mmol), 4-dimethylaminopyridine (88.5 mg, 0.7 mmol) and acetic anhydride (397 µl, 4.2 mmol) were added sequentially thereto. The mixture was stirred at room temperature for 1 hour. After diluting the reaction solution by adding water, extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane: ethyl acetate = 1:1) to obtain the title compound (333 mg) as a white solid.

400MHz ¹H-NMR (CDCl₃) δ (ppm) 0.98 (d, J = 6.4 Hz, 1.5H), 0.99 (d, J = 6.2 Hz, 1.5H), 1.14-1.65 (m, 6H), 1.62 (s, 3H), 1.66-2.06 (m, 3H), 2.01 (s, 1.5H), 2.08 (s, 1.5H), 2.20-2.35 (m, 2H), 4.01-4.14 (m, 2H), 4.19-4.30 (m, 1H), 4.51 (s, 2H), 5.17-5.47 (m, 3.5H), 5.58 (dd, J = 9.9, 13.9 Hz, 0.5H), 5.66-5.73 (m, 1H), 7.24-7.36 (m, 5H); MS m/z 435.99 (M + Na)⁺

### Process (1-12): Synthesis of (2E)-3-[(2S, 3S, 4E)-6-(acetoxy)-3-methyl-12-oxaazacyclododec-4-en-2-yl]but-2-en-1-yl acetate (P12)

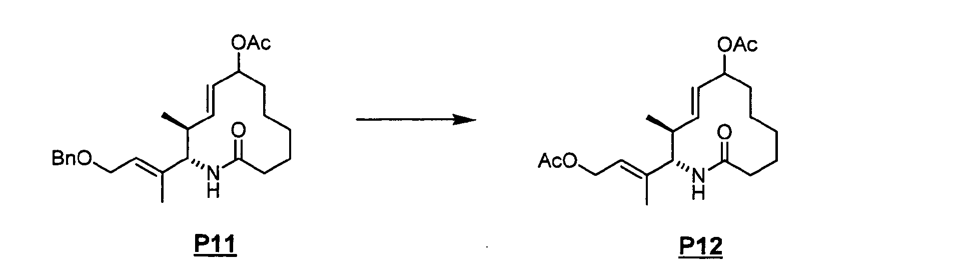

This process was carried out with reference to Ireland, R.E. and 3 others, Journal of the American Chemical Society, 1985, Vol. 107, p 3285-3294.

In the process, anhydrous THF distilled from benzophenone ketyl just before it was used. Also, lithium-4,4'-di-tert-butylbiphenyl to be used in the process was prepared just before its use in accordance with an common method using lithium (7.56 mg, 1.09 mmol) and 4,4'-di-tert-butylbiphenyl (193 mg, 0.726 mmol).

To an anhydrous THF solution (726 µl) of (2S, 3S, 4E)-2-[(1E)-3-(benzyloxy)-1-methylprop-1-en-1-yl]-3-methyl-12-oxaazacyclododec-4-e n-6-yl acetate (30 mg, 72.6 µmol), an anhydrous THF solution (7.26 ml) of lithium-4,4'-di-tert-butylbiphenyl was added dropwise at -78°C under a nitrogen atmosphere until the reaction solution gives a dark blue color. After stirring the reaction solution at -78°C for 1 hour, it was diluted by adding saturated aqueous solution of ammonium chloride, and then extraction with ethyl acetate was performed. The organic layer was washed with saturated brine, dried over anhydrous magnesium sulfate, and concentrated under reduced pressure.

The thus obtained residue was dissolved in dichloromethane (1 ml), and triethylamine (50.7 µl, 0.363 mmol), 4-dimethylaminopyridine (4.43 mg, 36.3 µmol) and acetic anhydride (20.6 µl, 0.218 mmol) were added sequentially thereto. The mixture was stirred at room temperature for 1 hour. After diluting the reaction solution by adding water, extraction with dichloromethane was subjected. The organic layer was dried over anhydrous magnesium sulfate and concentrated under reduced pressure. The thus obtained residue was purified by silica gel column chromatography (Kanto Chemical, trade name: Silica gel 60N, granular, 0.040 to 0.100 mm; n-heptane : ethyl acetate = 1:2) to obtain the title compound (15 mg) as a colorless oil.

400MHz ¹H-NMR (CDCl₃) δ (ppm) 0.94 (d, J = 6.8 Hz, 1.5H), 0.96 (d, J = 6.8 Hz, 1.5H), 1.08-1.86 (m, 7H), 1.62 (s, 3H), 1.87-2.12 (m, 2H), 2.01 (s, 1.5H), 2.06 (s, 3H), 2.08 (s, 1.5H), 2.15-2.39 (m, 2H), 4.17-4.30 (m, 1H), 4.53-4.69 (m, 2H), 5.16-5.35 (m, 1H), 5.36-5.74 (m, 4H); MS m/z 388.14 (M +Na)⁺

### Process (1-13): Synthesis of (2S, 3S, 4E)-2-[(1E)-3-hydroxy-1-methylprop-1-en-1-yl]-3-methyl-12-oxaazacyclododec-4-en-6-yl acetate (P13)

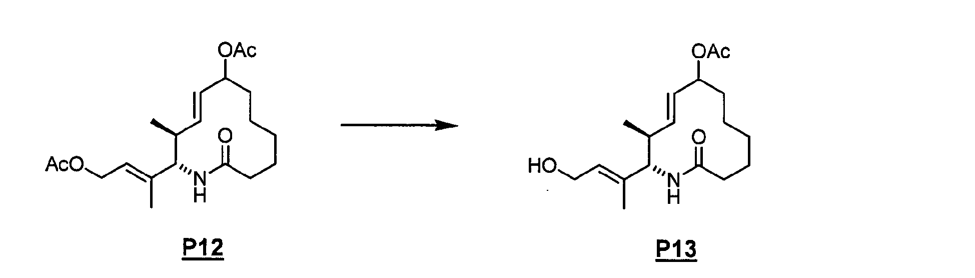

To a methanol solution (3ml) of (2E)-3-[(2S, 3S, 4E)-6-(acetoxy)-3-methyl-12-oxaazacyclododec-4-en-2-yl]but-2-en-1-yl acetate (92 mg, 0.251 mmol), activated molecular sieve powder 4A (200 mg) was added under a nitrogen atmosphere. The mixture was stirred at room temperature for 7.5 hours. The reaction solution was filtered through celite, and then the filtrate was concentrated under reduced pressure. The thus obtained residue was purified by silica gel column chromatography (Kanto Chemical, trade name: Silica gel 60N, granular, 0.040 to 0.100 mm; n-heptane : ethyl acetate = 1:3 → dichloromethane : methanol = 15:1) to obtain the title compound (61.2 mg) as a white solid.

400MHz ¹H-NMR (CDCl₃) δ (ppm) 0.96 (d, J = 6.4 Hz, 1.5H), 0.97 (d, J = 6.4 Hz, 1.5H), 1.11-1.87 (m, 7H), 1.65 (s, 3H), 1.88-2.16 (m, 2H), 2.01 (s, 1.5H), 2.08 (s, 1.5H), 2.17-2.38 (m, 2H), 4.08-4.27 (m, 3H), 5.16-5.37 (m, 1H), 5.38-5.50 (m, 1.5H), 5.51-5.75 (m, 2.5H); MS m/z 346.12 (M + Na)⁺

### Process (1-14): Synthesis of (25, 3S, 4E)-3-methyl-2-[(1E)-1-methylbuta-1,3-dien-1-yl]-12-oxaazacyclododec-4-en-6-yl acetate (P 14)

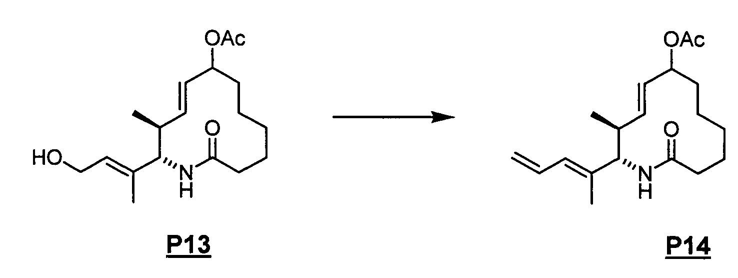

To a dichloromethane solution (2 ml) of (2S, 3S, 4E)-2-[(1E)-3-hydroxy-1-methylprop-1-en-1-yl]-3-methyl-12-oxaazacyclododec-4-en-6-yl acetate (20 mg, 61.8 µmol), a Dess-Martin reagent (31.6 mg, 74.2 µmol) was added at room temperature, and the mixture was stirred at room temperature for 30 minutes. The reaction solution was diluted with ethyl acetate, and then washed sequentially with saturated aqueous solution of sodium bicarbonate containing sodium sulfite and saturated brine. The organic layer was dried over anhydrous magnesium sulfate, and concentrated under reduced pressure.

The solution prepared by dissolving the obtained residue in anhydrous THF (1 ml) was added dropwise at 0°C under a nitrogen atmosphere to an anhydrous THF solution (1 ml) of methylenetriphenylphosphorane prepared according to an common method using methyltriphenylphosphonium iodide (37.6 mg, 92.8 µmol) and a solution of 1.59M n-butyllithium in n-hexane (58.4 µl, 92.8 µmol). The mixture was stirred at the same temperature for 30 minutes. The reaction solution was diluted with a saturated aqueous solution of ammonium chloride, and then extracted with ethyl acetate. The organic layer was dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 1:3) to obtain the title compound (10.4 mg) as a white solid.

400MHz ¹H-NMR (CDCl₃) δ (ppm) 0.95 (d, J = 6.8 Hz, 1.5H), 0.96 (d, J = 6.8 Hz, 1.5H), 1.16-1.87 (m, 7H), 1.73 (s, 3H), 1.90-2.12 (m, 2H), 2.01 (s, 1.5H), 2.08 (s, 1.5H), 2.20-2.37 (m, 2H), 4.20-4.30 (m, 1H), 5.08-5.16 (m, 1H), 5.17-5.50 (m, 4.5H), 5.58 (dd, J = 10.1 Hz, 14.6 Hz, 0.5H), 6.10 (d, J = 10.8 Hz, 1H), 6.48-6.61 (m, 1H); MS m/z 342.14 (M + Na)⁺

### Process (1-15): Synthesis of methyl-(2E)-3-methyl-5-[(1-phenyl-1-H-tetrazol-5-yl)thio]pent-2-enoate (Q1a) and Synthesis of ethyl-(2E)-3-methyl-5-[(1-phenyl-1-H-tetrazol-5-yl)thio]pent-2-enoate (Q1b)

### Process (1-15-1): Synthesis of methyl-(2E)-3-methyl-5-[(1-phenyl-1-H-tetrazol-5-yl)thio]pent-2-enoate (Q1a)

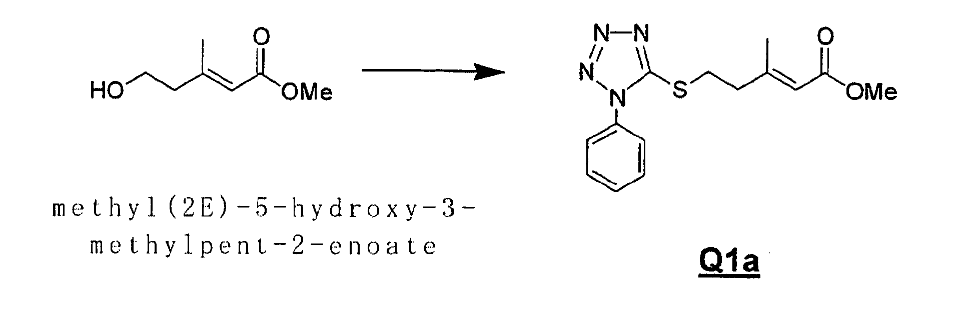

To a THF solution (36.8 ml) of methyl-(2E)-5-hydroxy-3-methylpent-2-enoate (1.16g, 7.31 mmol), 5-mercapto-1-phenyltetrazole (2.62g, 14.60 mmol), triphenylphosphine (3.83g, 14.60 mmol) and diisopropylazodicarboxylate (95%, 2.96g, 14.60 mmol) were added under ice cooling. The reaction solution was warmed up to a room temperature, and stirred for 2 hours. The reaction solution was diluted with ethyl acetate, and then washed with distilled water and saturated brine. The organic layer was dried over anhydrous magnesium sulfate and concentrated under reduced pressure. The thus obtained residue was purified by silica gel column chromatography (Merck, trade name: Silica gel 60, 230-400 mesh; hexane : ethyl acetate = 5:1) to obtain the title compound (2.33g) as a white solid.

400MHz ¹H-NMR (CDCl₃) δ (ppm) 2.22 (d, J = 1.2 Hz, 3H), 2.70 (t, J = 7.2 Hz, 2H), 3.54 (t, J = 7.2 Hz, 2H), 3.70 (s, 3H), 5.70-5.74 (q, J = 1.2 Hz, 1H), 7.52 - 7.59 (m, 5H); 100MHz ¹³C-NMR (CDCl₃) δ (ppm) 18.41, 30.75, 39.68, 50.94, 117.30, 123.73, 129.81, 130.18, 133.46, 153.79, 155.84, 166.49; IR (KBr) = 3071, 2991, 2948, 1707, 1648, 1593, 1499, 1437, 1412, 1380, 1226, 1153, 1057, 877, 761, 693, 559, 485, 407cm⁻¹; HRMS C₁₄H₁₆AgN₄O₂S (M+Ag⁺)
Calculated.: 411.0045, Found: 411.0073

### Process (1-15-2): Synthesis of ethyl-(2E)-3-methyl-5-[(1-phenyl-1-H-tetrazol-5-yl)thio]pent-2-enoate (Q1b)

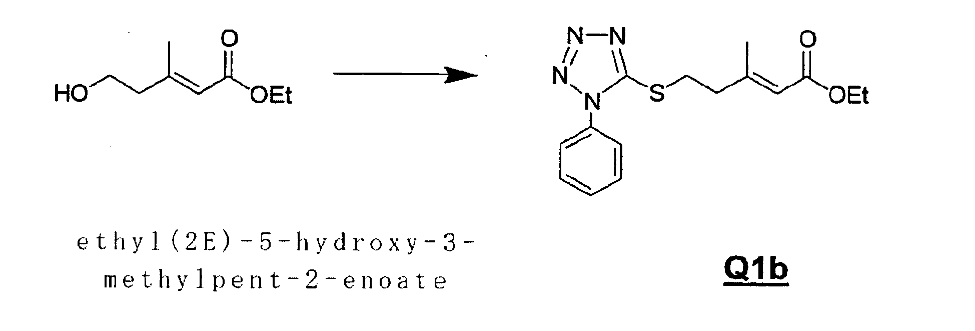

This reaction was carried out with reference to Mitsunobu, O., Synthesis, 1981, p 1-28.

To a THF solution (410 ml) of ethyl-(2E)-5-hydroxy-3-methylpent-2-enoate (13.80g, 86.90 mmol), 5-mercapto-1-phenyltetrazole (16.30g, 91.20 mmol), triphenylphosphine (27.30g, 104 mmol) and diisopropylazodicarboxylate (95%, 21.10g, 104 mmol) were added under ice cooling. The reaction solution was warmed up to a room temperature, and stirred for 4 hours. The reaction solution was diluted with ethyl acetate, and then washed with distilled water and saturated brine. After drying the organic layer over anhydrous magnesium sulfate, desiccant was filtered off, and concentration was performed under pressure. The thus obtained residue was purified by silica gel column chromatography (Merck, trade name: Silica gel 60, 230-400 mesh; hexane : ethyl acetate = 5:1) to obtain the title compound (27.16g) as a white solid.

400MHz ¹H-NMR (CDCl₃) δ (ppm) 1.26 (t, J = 7.2 Hz, 3H), 2.22 (s, 3H), 2.70 (t, J = 7.2 Hz, 2H), 3.54 (t, J = 7.2 Hz, 2H), 4.15 (q, J = 7.2 Hz, 2H), 5.72 (brs, 1H), 7.54-7.59 (m, 5H); 100 MHz ¹³C-NMR (CDCl₃) δ (ppm) 14.05, 18.22, 30.59, 39.53, 59.48, 117.58, 123.57, 129.61, 129.98, 133.33, 153.61, 155.24, 165.96; IR (KBr) = 3064, 2981, 2939, 2903, 2358, 2341, 1713, 1650, 1597, 1500, 1386, 1278, 1222, 1145, 1054, 763,694 cm⁻¹; HRMS C₁₅H₁₈AgN₄O₂S (M + Ag⁺)
Calculated: 425.0201, Found: 425.0170

### Process (1-16): Synthesis of methyl-(2E)-3-methyl-5-[(1-phenyl-1-H-tetrazol-5-yl)sulfonyl]pent-2-enoate (Q2a) and ethyl-(2E)-3-methyl-5-[(1-phenyl-1-H-tetrazol-5-yl)sulfonyl]pent-2-enoate (Q2b)

### Process (1-16-1): Synthesis of methyl-(2E)-3-methyl-5-[(1-phenyl-1-H-tetrazol-5-yl)sulfonyl]pent-2-enoate (Q2a)

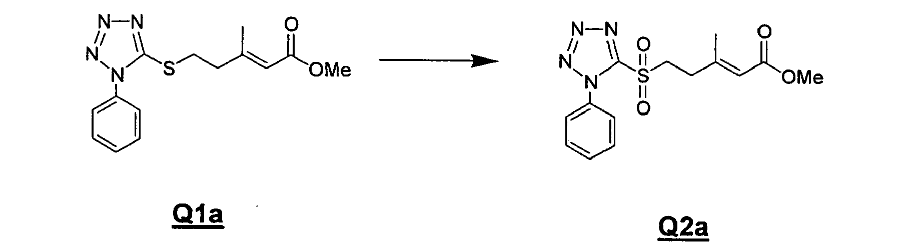

This reaction was carried out with reference to Shultz, H.S. and 2 others, Journal of Organic Chemistry, 1963, Vol. 28, Issue No. 4, p 1140-1140.

To an ethanol solution (150 ml) of methyl-(2E)-3-methyl-5-[(1-phenyl-1-H-tetrazol-5-yl)thio]pent-2-enoate (7.53g, 24.70 mmol), about 30% hydrogen peroxide solution (26.7 ml, 235 mmol) of hexaammonium heptamolybdate tetrahydrate (2.91 mg, 2.35 mmol) was added at room temperature. After stirring the mixture at the same temperature for 12 hours, the reaction solution was diluted with ethyl acetate, and washed with distilled water and saturated brine. The organic layer was dried over anhydrous magnesium sulfate, and concentrated under reduced pressure. The thus obtained residue was purified by silica gel column chromatography (Merck, trade name: Silica gel 60, 230-400 mesh; hexane : ethyl acetate = 4:1) to obtain the title compound (8.24g) as a white solid.

400MHz ¹H-NMR (CDCl₃) δ (ppm) 2.24 (d, J = 1.2 Hz, 3H), 2.79-2.83 (m, 2H), 3.71 (s, 3H), 3.88-3.92 (m, 2H), 5.77-5.78 (q, J = 1.2 Hz, 1H), 7.60-7.71 (m, 5H); 100 MHz ¹³C-NMR (CDCl₃) δ (ppm) 18.25, 32.44, 50.90, 53.59, 117.67, 124.83, 129.51, 131.30, 132.66, 152.97, 165.99; IR (KBr) = 3102, 3075, 2952, 2913, 1703, 1651, 1495, 1439, 1346, 1238, 1160, 1047, 998, 923, 882, 764, 689, 593, 550, 507, 456, 420 cm⁻¹; HRMS C₁₄H₁₆AgN₄O₄S (M +Ag⁺)
Calculated: 442.9943, Found: 442.9929

### Process (1-16-2): Synthesis of ethyl-(2E)-3-methyl-5-[(1-phenyl-1-H-tetrazol-5-yl)sulfonyl]pent-2-enoate (Q2b)

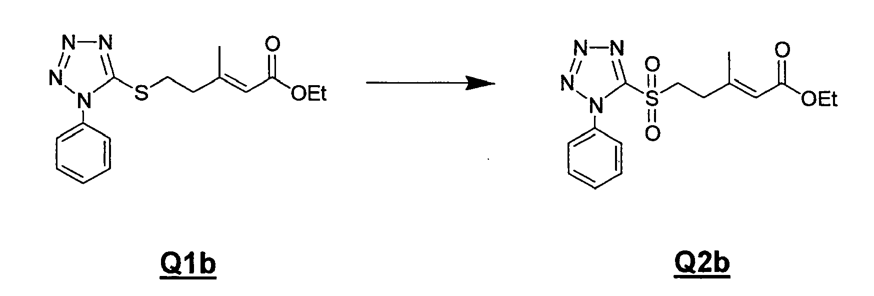

This reaction was carried out with reference to Shultz, H.S. and 2 others, Journal of Organic Chemistry, 1963, Vol. 28, Issue No. 4, p 1140-1140.

To an ethanol solution (200 ml) of ethyl-(2E)-3-methyl-5-[(1-phenyl-1-H-tetrazol-5-yl)thio]pent-2-enoate (13.31 g, 41.80 mmol), about 30% hydrogen peroxide solution (47.4 ml, 418.0 mmol) of hexaammonium heptamolybdate tetrahydrate (542 mg, 0.44 mmol) was added at room temperature. After stirring the mixture at the same temperature for 12 hours, the reaction solution was diluted with ethyl acetate, and washed with distilled water and saturated brine. The organic layer was dried over anhydrous magnesium sulfate, and concentrated under reduced pressure. The thus obtained residue was purified by silica gel column chromatography (Merck, trade name: Silica gel 60, 230-400 mesh; hexane : ethyl acetate = 4:1) to obtain the title compound (12.65g) as a white solid.

400MHz ¹H-NNM (CDCl₃) δ (ppm) 1.29 (t, J = 7.2 Hz, 3H), 2.24 (d, J = 1.2 Hz, 3H), 2.78-2.82 (m, 2H), 3.88-3.92 (m, 2H), 4.17 (q, J = 7.2 Hz, 2H), 5.77 (brs, 1H), 7.59-7.71 (m, 5H); 100 MHz ¹³C-NMR (CDCl₃) δ (ppm) 14.22, 18.51, 32.72, 53.93, 59.97, 118.44, 124.95, 129.77, 131.55, 132.88, 152.56, 153.19, 165.81; IR (KBr) = 3077, 3008, 2991, 2906, 1698, 1660, 1495, 1342, 1235, 1158, 1045, 877, 764, 689, 589, 550, 508, 454 cm⁻¹; HRMS C₁₅H₁₈AgN₄O₄S (M + Ag⁺)
Calculated: 457.0100, Found: 457.0133

### Process (1-17): Synthesis of methyl-(2E, 5E, 7S, 8S)-3,7-dimethyl-8-[(triethylsilyl)oxy]deca-2,5-dienoate (Q3a) and ethyl-(2E, 5E, 7S, 8S)-3,7-dimethyl-8-[(triethylsilyl)oxy]deca-2,5-dienoate (Q3b)

### Process (1-17-1): Synthesis of methyl-(2E, 5E, 7S, 8S)-3,7-dimethyl-8-[(triethylsilyl)oxy]deca-2,5-dienoate (Q3a)

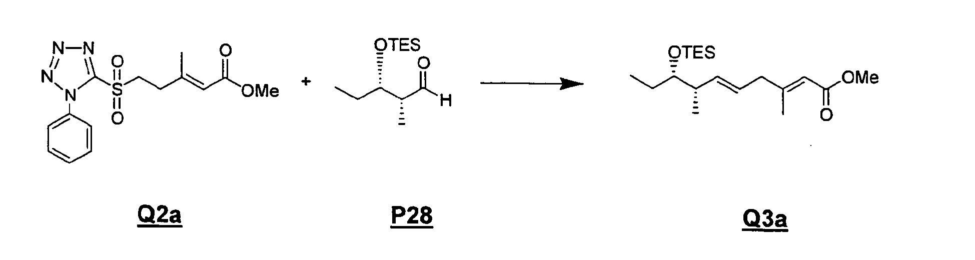

This reaction was carried out with reference to Blakemore, P.R. and 3 others, Synlett, 1998, p 26-28.

THF to be used in the reaction was distilled from lithium aluminum hydride. Also, DME was distilled from calcium hydride.

To a DME solution (40 ml) of methyl-(2E)-3-methyl-5-[(1-phenyl-1-H-tetrazol-5-yl)sulfonyl]pent-2-enoate (1.19g, 3.53 mmol), a 0.5M toluene solution (8.48 ml, 4.24 mmol) of potassium bis(trimethylsilyl)amide was added dropwise at -60°C, and the mixture was stirred at the same temperature for 30 minutes. Subsequently, a THF solution (5 ml) of (2R, 3S)-2-methyl-3-[(triethylsilyl)oxy]pentanal (1.63g, 7.06 mmol) was added dropwise at -78°C, and then the mixture was stirred for 2 hours. The reaction solution was warmed up to a room temperature, and then distilled water was added thereto. It was then diluted with ethyl acetate, and washed with saturated brine. The organic layer was dried over anhydrous magnesium sulfate, and concentrated under reduced pressure. The thus obtained residue was purified by silica gel column chromatography (Merck, trade name: Silica gel 60, 230-400 mesh; hexane : diethylether = 50:1) to obtain the title compound (1.17g) as a colorless oil. The title compound was determined to be a mixture of E:Z = 17:1 by ¹H-NNM.

400MHz ¹H-NMR (CDCl₃) δ (ppm) 0.60 (q, J = 8.0 Hz, 6H), 0.87 (t, J = 7.2 Hz, 3H), 0.95 (t, J = 8.0 Hz, 9H), 0.96 (d, J = 6.8 Hz, 3H), 1.35-1.50 (m,2 H), 2.14 (d, J = 1.2 Hz, 3H), 2.25-2.31 (m, 1H), 2.82 (d, J = 6.4 Hz, 2H), 3.44-3.48 (m, 1H), 3.69 (s, 3H), 5.36 (ddd, J = 6.8, 13.6, 15.6 Hz, 1H), 5.51 (dd, J = 7.6, 15.2 Hz, 1H), 5.68 (q, J = 1.2 Hz, 1H); 100 MHz ¹³C-NMR (CDCl₃) δ (ppm) 5.04, 6.78, 9.35, 15.41, 18.57, 26.65, 41.50, 43.86, 50.48, 77.18, 115.38, 124.80, 136.84, 158.86, 166.97; IR (neat) = 2958, 2879, 2352, 2330,1723, 1651, 1435, 1219, 1147, 1013, 740 cm⁻¹; HRMS C₁₉H₃₆AgO₃Si (M + Ag⁺)
Calculated: 447.1485, Found: 447.1461; [α]_{D}²¹ -22.1 (c1.10, CHCl₃)

### Process (1-17-2): Synthesis of ethyl-(2E, 5E, 7S, 8S)-3,7-dimethyl-8-[(triethylsilyl)oxy]deca-2,5-dienoate (Q3b)

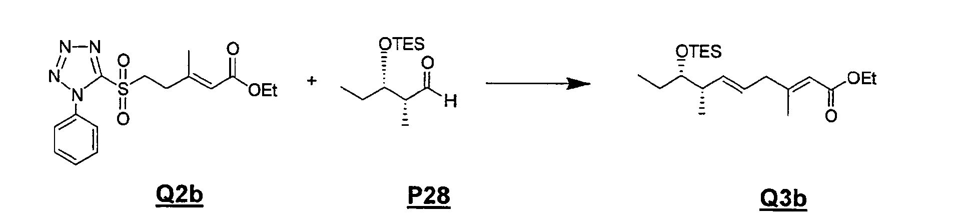

This reaction was carried out with reference to Blakemore, P. R. and 3 others, Synlett, 1998, p 26-28.

THF to be used in the reaction was distilled from lithium aluminum hydride. Also, DME was distilled from calcium hydride.

To a DME solution (280 ml) of ethyl-(2E)-3-methyl-5-[(1-phenyl-1-H-tetrazol-5-yl)sulfonyl]pent-2-enoate (9.0g, 25.7 mmol), a 0.5M toluene solution (64.2 ml) of potassium bis(trimethylsilyl)amide was added dropwise at -60°C, and the mixture was stirred at the same temperature for 30 minutes. Subsequently, a THF solution (50 ml) of (2R, 3S)-2-methyl-3-[(triethylsilyl)oxy]pentanal (12.0g, 52.1 mmol) was added dropwise at -78°C, and then the mixture was stirred for 1 hour. The reaction solution was warmed up to a room temperature, and then distilled water was added thereto. It was then diluted with ethyl acetate, and washed with saturated brine. The organic layer was dried over anhydrous magnesium sulfate, and concentrated under reduced pressure. The thus obtained residue was purified by silica gel column chromatography (Merck, trade name: Silica gel 60, 230-400 mesh; hexane : diethylether = 100: 1) to obtain the title compound (8.92g) as a colorless oil. The title compound was determined to be a mixture of E:Z = 18:1 by ¹H-NMR.

400MHz ¹H-NMR (CDCl₃) δ (ppm) 0.60 (q, J = 8.0 Hz, 6H), 0.87 (t, J = 7.2 Hz, 3H), 0.95 (d, J = 7.6 Hz, 3H), 0.96 (t, J = 8.0 Hz, 9H), 1.27 (t, J = 7.2 Hz, 3H), 1.36-1.53 (m, 2H), 2.13 (d, J = 1.2 Hz, 3H), 2.26-2.32 (m, 1H), 2.81 (d, J = 6.8 Hz, 2H), 3.44-3.48 (m, 1H), 4.14 (q, J = 7.2 Hz, 2H), 5.36 (ddd, J = 6.8, 13.6, 15.2 Hz, 1H), 5.51 (dd, J = 7.6, 15.2 Hz, 1H), 5.67-5.68 (m, 1H); 100 MHz ¹³C-NMR (CDCl₃) δ (ppm) 5.05, 6.77, 9.35, 14.11, 15.37, 18.51, 26.67, 41.50, 43.89, 59.18, 77.20, 115.85, 124.88, 136.78, 158.38, 166.52; IR (neat) = 2958, 2879, 2362, 2345, 1719, 1650, 1460, 1218, 1144, 1050, 1013, 740 cm⁻¹; HRMS C₂₀H₃₈AgO₃Si (M + Ag⁺)
Calculated: 461.1641, Found: 461.1640; [α]_{D}²² -19.0 (c2.60, CHCl₃)

### Process (1-18): Synthesis of (2E, 5E, 7S, 8S)-3,7-dimethyl-8-[(triethylsilyl)oxy]deca-2,5-dien-1-ol (Q4)

### Process (1-18-1): Synthesis of (2E, 5E, 7S, 8S)-3,7-dimethyl-8-[(triethylsilyl)oxy]deca-2,5-dien-1-ol (Q4)

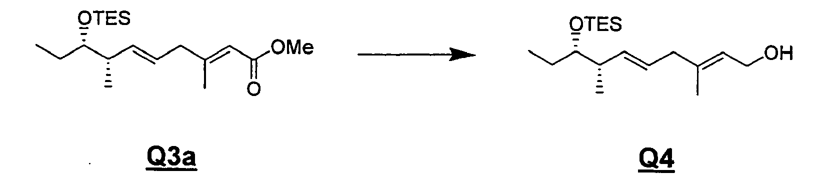

To a toluene solution (6 ml) of methyl-(2E, 5E, 7S, 8S)-3,7-dimethyl-8-[(triethylsilyl)oxy]deca-2,5-dienoate (0.22g, 0.64 mmol), a 1M diisobutylaluminum hydride toluene solution (1.76 ml, 1.76 mmol) was added dropwise at -78°C, and it was stirred at the same temperature for 30 minutes. The reaction solution was diluted with diethyl ether, a saturated aqueous solution (1.0 ml) of potassium sodium tartrate tetrahydrate was added thereto, and the mixture was warmed up to a room temperature and then stirred for 2 hours. The organic layer was dried over anhydrous sodium sulfate, and concentrated under reduced pressure. The thus obtained residue was purified by silica gel column chromatography (Merck, trade name: Silica gel 60, 230-400 mesh; hexane : diethylether = 8:1) to obtain the title compound (150 mg) as a colorless oil.

400MHz ¹H-NMR (CDCl₃) δ (ppm) 0.60 (q, J = 7.6 Hz, 6H), 0.87 (t, J = 7.6 Hz, 3H), 0.95 (d, J = 14.0 Hz, 3H), 0.96 (t, J = 7.6 Hz, 9H), 1.35-1.50 (m, 2H), 1.65 (brs, 3H), 2.24-2.29 (m, 1H), 2.70 (d, J = 6.4 Hz, 2H), 3.45 (dd, J = 5.6 Hz, 1H), 4.16 (d, J = 7.2 Hz, 2H), 5.32-5.48 (m, 3H); 100 MHz ¹³C-NMR (CDCl₃) δ (ppm) 5.08, 6.85, 9.37, 15.73, 16.08, 26.72, 41.50, 42.82, 59.10, 77.39, 123.82, 126.73, 135.25, 138.53; IR (neat) = 3330, 2959, 2875, 1671, 1459, 1419, 1009, 740 cm⁻¹; HRMS C₁₈H₃₆AgO₂Si (M +Ag⁺)
Calculated: 419.1536, Found: 419.1572; [α]_{D}²¹ -23.4 (c1.45, CHCl₃)

### Process (1-18-2): Synthesis of (2E, 5E, 7S, 8S)-3,7-dimethyl-8-[(triethylsilyl)oxy]deca-2,5-dien-1-ol (Q4)

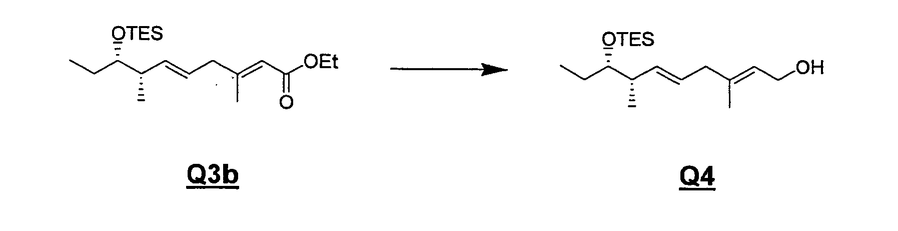

To an anhydrous diethylether solution (37.5 ml) of ethyl-(2E, 5E, 7S, 8S)-3,7-dimethyl-8-[(triethylsilyl)oxy]deca-2,5-dienoate (8.92g, 25.20 mmol), a 1M diisobutylaluminum hydride toluene solution (52.2 ml, 52.2 mmol) was added dropwise at -78°C, and it was stirred at the same temperature for 1 hour. To the reaction solution, methanol (1 ml) and saturated aqueous solution (9.3 ml) of potassium sodium tartrate tetrahydrate were added thereto, and the mixture was warmed up to room temperature and then stirred for 2 hours. The organic layer was dried over anhydrous sodium sulfate, and concentrated under reduced pressure. The thus obtained residue was purified by silica gel column chromatography (Merck, trade name: Silica gel 60, 230-400 mesh; hexane : diethylether = 8:1) to obtain the title compound (3.84g) as a colorless oil. ¹H-NMR data of the title compound obtained in (2-2) was completely identical to those of the title compound obtained in (2-1).

### Process (1-19): Synthesis of {(2R, 3R)-3-[(2E, 4S, 5S)-5-hydroxy-4-methylhept-2-en-1-yl]-3-methyloxiran-2-yl} methyl 4-methylbenzenesulfonate (Q7)

### Process (1-19-1): Synthesis of (2R, 3R)-3-methyl-3-{(2E, 4S, SS)-4-methyl-5-[(triethylsilyl)oxy]hept-2-en-1-yl}oxiran-2-yl)methanol (Q5)

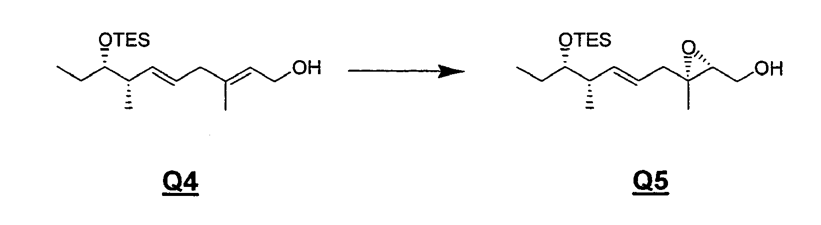

This reaction was carried out with reference to literatures, Katsuki, T. and 2 others, Journal of the American Chemical Society, 1980, Vol. 102, p 5976-5978; Gao, Y. and 5 others, Journal of the American Chemical Society, 1987, Vol. 109, p 5765-5780.
(i) Distilled anhydrous dichloromethane (22 ml) was added to activated molecular sieves 4A powder (880 mg) under argon atmosphere and the solution was cooled to -30°C. Subsequently, after adding diethyl (-)-tartrate (2.01 ml, 11.7 mmol) and titanium tetraisopropoxide (2.32 ml, 7.89 mmol) to the reaction solution while stirring and the mixture was stirred for 5 minutes, and then a dichloromethane solution (4 ml) of (2E,5E,7S,8S)-3,7-dimethyl-8-[(triethylsilyl)oxy]deca-2,5-dien-l-ol (1.76 g, 5.63 mmol) was added dropwise. After stirring for 30 minutes, 5M tert-butyl hydroperoxide decane solution (2.26 ml, 11.20 mmol) was added dropwise to the reaction solution over 15 minutes. Then the reaction solution was further stirred at the same temperature for 1.5 hours. Distilled water (5 ml) was added to the reaction solution and filtered through celite. The filtrate was diluted with ethyl acetate and washed with distilled water and saturated brine. The organic layer was dried over anhydrous sodium sulfate and concentrated under reduced pressure. The thus obtained residue was purified by silica gel column chromatography (Kanto, trade name: Silica Gel 60N, 40-100 µm; heptane: ethyl acetate = 5:1) to obtain the title compound (1.69 g, 90% de) as a colorless oil. The measurement of optical purity was performed by converting the title compound to {(2R,3R)-3-[(2E,4S,5S)-5-hydroxy-4-methylhept-2en-1-yl]-3-methyloxiran-2-yl}methyl 4-methylbenzenesulfonate (Q7) and determining by HPLC using a chiral column (DAICEL, trade name: CHIRALPAK AD-H, hexane : isopropyl alcohol = 90:10).
(ii) Distilled anhydrous dichloromethane (12 ml) was added to activated molecular sieves 4A powder (500 mg) under argon atmosphere and the solution was cooled to -30°C. Subsequently, after adding isopropyl (-)-tartrate (0.202 ml, 0.96 mmol) and titanium tetraisopropoxide (0.189 ml, 0.640 mmol) to the reaction solution while stirring, the mixture was stirred for 5 minutes, and then a dichloromethane solution (3 ml) of (2E,5E,7S,8S)-3,7-dimethyl-8-[(triethylsilyl)oxy]deca-2,5-dien-l-ol (1.0 g, 3.20 mmol) was added dropwise. After stirring the reaction solution for 30 minutes, a 5M tert-butyl hydroperoxide decane solution (1.28 ml, 6.36 mmol) was added dropwise to the reaction solution over 15 minutes. Then the reaction solution was further stirred at the same temperature for 2 hours. Distilled water (5 ml) was added to the reaction solution and it was filtered through celite. The filtrate was diluted with ethyl acetate and washed with distilled water and saturated brine. The organic layer was dried over anhydrous sodium sulfate and concentrated under reduced pressure. The thus obtained residue was purified by silica gel column chromatography (Kanto, trade name: Silica Gel 60N, 40-100 µm; heptane : ethyl acetate = 5:1) to obtain the title compound (925 mg, 82% de) as a colorless oil. The measurement of optical purity was performed by converting the title compound to {(2R,3R)-3-[(2E,4S,5S)-5-hydroxy-4-methylhept-2en-1-yl]-3-methyloxiran-2-yl}methyl 4-methylbenzenesulfonate (Q7) and determining by HPLC using a chiral column (DAICEL, trade name: CHIRALPAK AD-H, hexane : isopropyl alcohol = 90:10).

400MHz ¹H-NMR (CD₃OD) δ (ppm) 0.67 (q, J = 8.0 Hz, 6H), 0.93 (t, J = 7.6 Hz, 3H), 1.02 (d, J = 6.8 Hz, 3H), 1.03 (t, J = 8.0 Hz, 9H), 1.29 (s, 3H), 1.45-1.58 (m, 2H), 2.23-2.35 (m, 3H), 2.97 (dd, J = 4.8, 6.4 Hz, 1H), 3.56 (dt, J = 5.6, 6.0 Hz, 1H), 3.63 (dd, J = 6.0, 12.0 Hz, 1H), 3.75 (dd, J = 4.8, 12.0 Hz, 1H), 5.13 (dt, J = 7.2, 15.6 Hz, 1H), 5.57 (dd, J = 7.6, 15.6 Hz, 1H); 100 MHz ¹³C-NMR (CD₃OD) δ (ppm) 6.14, 7.43, 9.85, 16.39, 17.05, 27.91, 42.79, 42.99, 61.68, 61.72, 63.68, 78.70, 125.23, 138.18; IR (neat) = 3422, 2959, 2877, 1459, 1239, 1103, 1016, 740 cm⁻¹; HRMS C₁₈H₃₆AgO₃Si (M +Ag⁺)
Calculated: 435.1485, Found: 435.1492; [α]_{D}²¹ -15.1 (c2.14, MeOH) (90% de)

### Process (1-19-2): Synthesis of (2R,3R)-3-methyl-3-{(2E,4S,5S)-4-methyl-5-[(triethylsilyl)oxy]hept-2-en-1-yl} oxiran-2-yl)methyl 4-methylbenzenesulfonate (Q6)

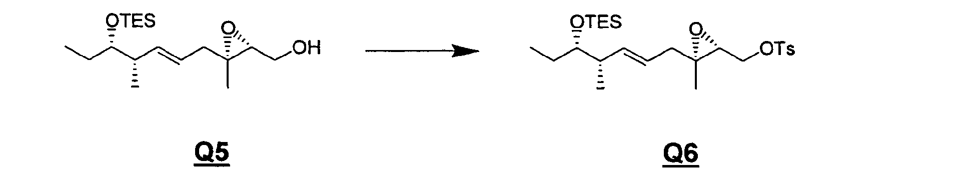

Triethylamine (1.86 ml, 12.90 mmol), 4-dimethylaminopyridine (23.7 mg, 0.19 mmol) and p-toluenesulphonyl chloride (493 mg, 2.59 mmol) were added to a dichloromethane solution (9 ml) of (2R,3R)-3-methyl-3-{(2E,4S,5S)-4-methyl-5-[(triethylsilyl)oxy]hept-2-en-1-yl}oxiran-2-yl)methanol (425 mg, 1.29 mmol, 90% de) at room temperature. The reaction solution was stirred at the same temperature for 2 hours. The reaction solution was diluted with ethyl acetate, and then washed with distilled water and saturated brine. The organic layer was dried over anhydrous sodium sulfate, and concentrated under reduced pressure. The thus obtained residue was purified by silica gel column chromatography (Kanto, trade name: Silica Gel 60N, 40-100 µm; heptane : ethyl acetate = 15:1) to obtain the title compound (641 mg, 90% de) as a colorless oil. The measurement of optical purity was performed by converting the title compound to {(2R,3R)-3-[(2E,4S,5S)-5-hydroxy-4-methylhept-2-en-1-yl]-3-methyloxiran-2-yl}methyl 4-methylbenzenesulfonate (Q7) and determining by HPLC using a chiral column (DAICEL, trade name: CHIRALPAK AD-H, hexane : isopropyl alcohol = 90:10).

400MHz ¹H-NMR (CD₃OD) δ (ppm) 0.66 (q, J = 8.0 Hz, 6H), 0.91 (t, J = 7.6 Hz, 3H), 0.99 (d, J = 7.2 Hz, 3H), 1.02 (t, J = 8.0 Hz, 9H), 1.21 (s, 3H), 1.40-1.56 (m, 2H), 2.15-2.35 (m, 3H), 2.50 (s, 3H), 3.02 (dd, J = 4.8, 6.8 Hz, 1H), 3.54 (dt, J = 5.2, 6.0 Hz, 1H), 4.10 (dd, J = 6.8, 11.2 Hz, 1H), 4.27 (dd, J = 4.8, 11.2 Hz, 1H), 5.37 (dt, J = 8.0, 15.6 Hz, 1H), 5.53 (dd, J = 8.0, 15.6 Hz, 1H), 7.50 (ddd, J = 1.0, 2.0, 6.8 Hz, 2H), 7.85 (dd, J = 1.6, 6.8 Hz, 2H); 100 MHz ¹³C-NMR (CD₃OD) δ (ppm) 6.11, 7.51, 9.90, 16.44, 17.20, 21.73, 27.87, 42.11, 42.92, 59.45, 61.85, 70.33, 78.54, 124.60, 128.97, 131.07, 134.19, 138.53, 146.44; [α]_{D}²¹ +2.70(c2.10, MeOH).

### Process (1-19-3): Synthesis of {(2R,3R)-3-[(2E,4S,5S)-5-hydroxy-4-methylhept-2-en-1-yl]-3-methyloxiran-2-yl}methyl 4-methylbenzenesulfonate (Q7)

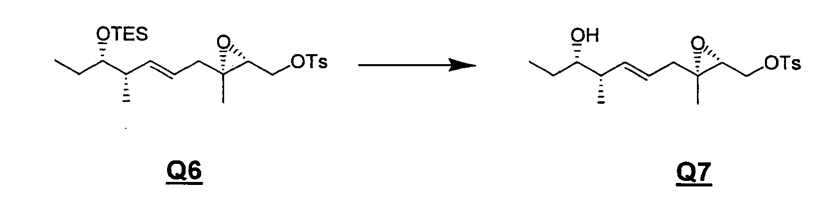

(i) To a THF solution (2 ml) of (2R,3R)-3-methyl-3-{(2E,4S,5S)-4-methyl-5-[(triethylsilyl)oxy]hept-2-en-1-yl}oxiran-2-yl)methyl 4-methylbenzenesulfonate (440 mg, 0.91 mmol, 90% de), 1M hydrochloric acid (0.1 ml) was added at room temperature, and the mixture was stirred at the same temperature for 1 hour. The reaction solution was diluted with ethyl acetate, and then washed with distilled water and saturated brine. The organic layer was dried over anhydrous sodium sulfate, and concentrated under reduced pressure. The thus obtained residue was purified by silica gel column chromatography (Kanto, trade name: Silica Gel 60N, 40-100 µm; heptane : ethyl acetate = 2:1) to obtain the title compound (334 mg, 90% de) as a colorless oil. The optical purity was determined by HPLC using a chiral column (DAICEL, trade name: CHIRALPAK AD-H, hexane : isopropyl alcohol = 90:10).
(ii) The title compound ({(2R,3R)-3-[(2E,45,5S)-5-hydroxy-4-methylhept-2-en-1-yl]-3-methyloxiran-2-yl}meth yl 4-methylbenzenesulfonate) (850 mg, 82% de) was purified by HPLC using a preparative chiral column (DAICEL, trade name: CHIRALPAK AD, 20x250 mm, hexane : isopropyl alcohol = 90:10, flow rate: 10 mL/min) and the title compound (634 mg, >99% de) was obtained as a colorless oil. In addition, β-epoxy isomer thereof (38 mg, >99% de) was obtained as a colorless oil.

Q7: 400MHz ¹H-NMR (CD₃OD) δ (ppm) 0.97 (t, J = 7.6 Hz, 3H), 1.04 (d, J = 6.8 Hz, 3H), 1.21 (s, 3H), 1.29-1.38 (m, 1H), 1.53-1.63 (m, 1H), 2.14-2.30 (m, 3H), 2.50 (s, 3H), 3.03 (dd, J = 4.4, 6.8 Hz, 1H), 3.25-3.29 (m, 1H), 4.10 (dd, J = 6.8, 11.2 Hz, 1H), 4.28 (dd, J = 4.4, 11.2 Hz, 1H), 5.38 (dt, J = 6.8, 15.6 Hz, 1H), 5.49 (dd, J = 8.0, 15.6 Hz, 1H), 7.50 (dd, J = 1.0, 8.4 Hz, 2H), 7.85 (ddd, J = 2.0, 2.0, 8.4 Hz, 2H); 100 MHz ¹³C-NMR (CD₃OD) δ (ppm) 10.64, 16.58, 17.10, 21.62, 28.31, 41.96, 44.21, 59.51, 62.01, 70.43, 77.63, 124.85, 129.00, 131.13, 134.15, 138.80, 146.65; IR (neat) = 3386, 2976, 2958, 2924, 2502, 1933, 1599, 1454, 1359, 1173, 1095, 967, 866, 782, 667, 554 cm⁻¹;HRMS C₁₉H₂₈NaO₅S (M + Na⁺); Calculated: 391.1555, Found: 391.1550; [α]_{D}²⁰ +2.82 (c1.04, MeOH)

β-epoxy isomer (isomer of Q7): 400MHz ¹H-NMR (CD₃OD) δ (ppm) 0.98 (t, J = 7.6 Hz, 3H), 1.04 (d, J = 6.8 Hz, 3H), 1.21 (s, 3H), 1.27-1.42 (m, 1H), 1.53-1.65 (m, 1H), 2.13-2.31 (m, 3H), 2.50 (s, 3H), 3.03 (dd, J = 4.8, 6.8 Hz, 1H), 3.23-3.31 (m, 1H), 4.10 (dd, J = 6.8, 11.6 Hz, 1H), 4.28 (dd, J = 4.8, 11.6 Hz, 1H), 5.38 (dt, J = 7.2, 15.6 Hz, 1H), 5.49 (dd, J = 8.0, 15.6 Hz, 1H), 7.50 (dd, J = 0.8, 8.0 Hz, 2H), 7.85 (m, 2H)

### Process (1-20): Synthesis of (1R)-1,2-anhydro-3,5-dideoxy-1-[(1R,2S)-2-hydroxy-1-methylbutyl]-4-C-vinyl-D-erythr o-pentitol (Q9)

### Process (1-20-1): Synthesis of 5,6:8,9-dianhydro-1,2,4,7-tetradeoxy-4,8-dimethyl-10-O-[(4-methylphenyl)sulfonyl]-D-t hreo-D-galacto-decitol (Q8)

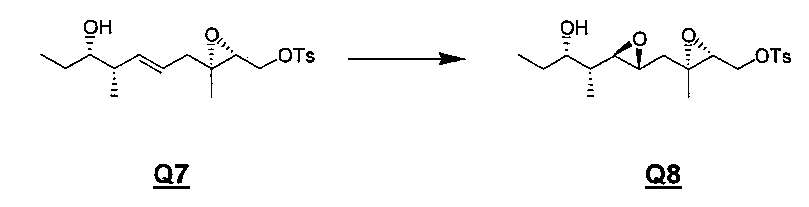

This reaction was carried out with reference to literatures, Wang, Z., -X. and 4 others, Journal of the American Chemical Society, 1997, Vol. 119, p 11224-11235; Wang, Z., -X. and 4 others, Journal of Organic Chemistry, 1997, Vol. 62, p 2328-2329.
(i) {(2R,3R)-3-[(2E,4S,5S)-5-hydroxy-4-methylhept-2-en-1-yl]-3-methyloxiran-2-yl}methyl 4-methylbenzenesulfonate (2.82 g, 7.64 mmol, 89% de) was dissolved in acetonitrile (80.8 ml) and a 0.4 mM ethylenediaminetetraacetic acid disodium salt solution (53.4 ml) of 0.05M sodium tetraborate decahydrate. 1,2:4,5-di-O-isopropylidene-D-erythro-2,3-hexodiuro-2,6-pyranose (1.97 g, 7.64 mmol) was added while ice cooling. Subsequently, a mixed powder of potassium carbonate (12.70 g, 91.80 mmol) and oxone (14.10 g, 22.93 mmol) was added at the same temperature over 4 hours. The reaction solution was stirred at the same temperature for another hour. The reaction solution was diluted with ethyl acetate and washed with distilled water and saturated brine. The organic layer was dried over anhydrous sodium sulfate and concentrated under reduced pressure. The thus obtained residue was purified by silica gel column chromatography (Kanto, trade name: Silica Gel 60N, 40-100 µm; heptane : ethyl acetate = 3:1 → 2:1) to obtain the title compound (2.57 g, 81% de) as a colorless oil. The optical purity of the title compound was determined by HPLC using a chiral column (DAICEL, trade name: CHIRALPAK AD-H, hexane : isopropyl alcohol = 80:20). Subsequently, it was recrystallized with a mixed solvent of hexane-diethyl ether and the title compound (1.52 g) was obtained as a colorless prism crystal of a single diastereomer (>99% de).
(ii) {(2R,3R)-3-[(2E,4S,5S)-5-hydroxy-4-methylhept-2-en-1-yl]-3-methyloxiran-2-yl}methyl 4-methylbenzenesulfonate (700 mg, 1.90 mmol, >99% de) was dissolved in acetonitrile (20.1 ml) and a 0.4 mM ethylenediaminetetraacetic acid disodium salt solution (13.3 ml) of 0.05M sodium tetraborate decahydrate. 1,2:4,5-di-O-isopropylidene-D-erythro-2,3-hexodiuro-2,6-pyranose (489 mg, 1.90 mmol) was added while ice cooling. Subsequently, a mixed powder of potassium carbonate (3.15 g, 22.8 mmol) and oxone (3.50 g, 5.71 mmol) was added at the same temperature over 4 hours. The reaction solution was further stirred at the same temperature for 1 hour. The reaction solution was diluted with ethyl acetate and washed with distilled water and saturated brine. The organic layer was dried over anhydrous sodium sulfate and concentrated under reduced pressure. The thus obtained residue was purified by silica gel column chromatography (Kanto, trade name Silica Gel 60N, 40-100 µm; heptane : ethyl acetate = 3:1 → 2:1) to obtain the title compound (331 mg, 88% de) as a colorless oil. The optical purity of the title compound was determined by HPLC using a chiral column (DAICEL, trade name: CHIRALPAK AD-H, hexane : isopropyl alcohol = 80:20).

400MHz ¹H-NMR (CD₃OD) δ (ppm) 0.98 (d, J = 7.2 Hz, 3H), 0.99 (t, J = 7.6 Hz, 3H), 1.27-1.36 (m, 1H), 1.32 (s, 3H), 1.48-1.61 (m, 2H), 1.67 (dd, J = 6.8, 14.4 Hz, 1H), 1.80 (dd, J = 4.8, 14.4 Hz, 1H), 2.50 (s, 3H), 2.71 (dd, J = 6.4, 8.0 Hz, 1H), 2.85 - 2.89 (m, 1H), 3.10 (dd, J = 4.4, 6.4 Hz, 1H), 3.57 (dt, J = 4.8, 8.0 Hz, 1H), 4.12 (dd, J = 6.4, 11.2 Hz, 1H), 4.31 (dd, J = 4.4, 11.6 Hz, 1H), 7.44 (dd, J = 1.0, 8.0 Hz, 2H), 7.86 (dd, J = 2.0, 8.4 Hz, 1H), 7.87 (dd, J = 2.0, 8.4 Hz, 1H); 100 MHz ¹³C-NMR (CD₃OD) δ (ppm) 10.55, 10.85, 17.41, 21.62, 28.55, 41.82, 42.36, 55.43, 60.18, 60.63, 61.63, 70.17, 75.15, 129.00, 131.13, 134.04, 146.66; IR (KBr) = 3389, 2976, 2957, 2873, 1923, 1598, 1454, 1359, 1188, 1173, 1099, 969, 866, 815, 669, 557, 507 cm⁻¹;HRMS C₁₉H₂₈AgO₆S (M + Ag⁺)
Calculated: 491.0658, Found: 491.0638; [α]_{D}²³ +42.7 (c1.00, MeOH) (> 99% de)

### Process (1-20-2): Synthesis of (1R)-1,2-anhydro-3,5-dideoxy-1-[(1R,2S)-2-hydroxy-1-methylbutyl]-4-C-vinyl-D-erythr o-pentitol (Q9)

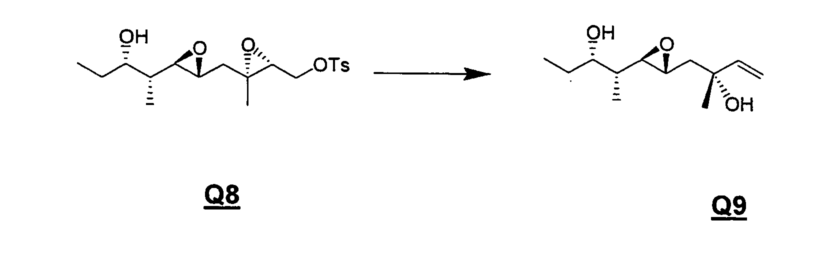

This reaction was carried out with reference to Fujii, N., and 7 others, Journal of the Chemical Society Perkin Transactions 1, 1996, p 865-866.

5,6:8,9-dianhydro-1,2,4,7-tetradeoxy-4,8-dimethyl-10-O-[(4-methylphenyl)sulfo nyl]-D-threo-D-galacto-decitol (350 mg, 0.91 mmol) was dissolved in a mixed solution of acetone (17.5 ml) and DMF (3.5 ml), and potassium iodide (528 mg, 3.18 mmol) was added thereto. The mixture was heated to reflux for 2 hours. The reaction was cooled to 0°C, and 4-(dimethylamino)phenyldiphenylphosphine (420 mg, 1.37 mmol) and iodine (100 mg, 0.45 mmol) were added to the reaction solution. The reaction solution was stirred for 15 minutes at the same temperature. A 5% aqueous solution of sodium bicarbonate (3 ml) and a 5% aqueous solution of sodium thiosulfate (2 ml) were added to the reaction solution, and then the reaction solution was further stirred for 10 minutes at the same temperature. The reaction solution was diluted with ethyl acetate, and washed with distilled water and saturated brine. The organic layer was dried over sodium sulfate anhydrous and concentrated under reduced pressure. The thus obtained residue was purified by silica gel column chromatography (Kanto Chemical, trade name: Silica gel 60N, granular, 40 to 100 µm; heptane : ethyl acetate = 2.5:1) to obtain the title compound (186 mg) as a colorless oil.

400MHz ¹H-NMR (CD₃OD) δ (ppm) 0.98 (d, J = 7.2 Hz, 3H), 0.99 (t, J = 7.6 Hz, 3H), 1.29-1.37 (m, 1H), 1.36 (s, 3H), 1.48-1.61 (m, 2H), 1.73 (dd, J = 6.0, 14.0 Hz, 1H), 1.80 (dd, J = 6.0, 14.0 Hz, 1H), 2.72 (dd, J = 2.4, 7.6 Hz, 1H), 2.95 (dt, J = 2.4, 6.0 Hz, 1H), 3.38 (s, 1H), 3.58 (dt, J = 4.8, 8.0 Hz, 1H), 5.08 (dd, J = 1.6, 10.8 Hz, 1H), 5.29 (dd, J = 1.6, 17.6 Hz, 1H), 6.02 (dd, J = 10.8, 17.6 Hz, 1H); 100 MHz ¹³C-NMR (CD₃OD) δ (ppm) 10.28, 10.92, 27.88, 28.54, 42.39, 45.54, 55.74, 62.10, 73.17, 75.18, 112.21, 146.27; IR (neat) = 3418, 3088, 2970, 2935, 2879, 1647, 1455, 1416, 925 cm⁻¹; HRMS C₁₂H₂₂O₃Ag (M + Ag⁺); Calculated: 321.0620, Found: 321.0667; [α]_{D}²⁴ +13.5 (c1.67, MeOH) (> 99% de).

### Process (1-20-3): Alternative Method for Synthesis of (1R)-1,2-anhydro-3,5-dideoxy-1-[(1R,2S)-2-hydroxy-1-methylbutyl]-4-C-vinyl-D-erythr o-pentitol (Q9)

This reaction was carried out with reference to Sarandeses, L.A. and 2 others, Journal of the Chemical Society Chemical Communication, 1991, p 818-820.

5,6:8,9-dianhydro-1,2,4,7-tetradeoxy-4,8-dimethyl-10-O-[(4-methylphenyl)sulfo nyl]-D-threo-D-galacto-decitol (200 mg, 0.52 mmol) was dissolved in a mixed solution of acetone (5 ml) and DMF (1 ml), and potassium iodide (303 mg, 1.83 mmol) was added thereto. The mixture was heated to reflux for 2 hours. The reaction was cooled to room temperature, and a 5% aqueous solution of sodium bicarbonate (3 ml) and a 5% aqueous solution of sodium thiosulfate (2 ml) were added to the reaction solution. The reaction solution was stirred for 10 minutes at the same temperature. Then, the reaction solution was diluted with ethyl acetate, and washed with distilled water and saturated brine. The organic layer was dried over anhydrous sodium sulfate and concentrated under reduced pressure. The obtained crude product was used in the next reaction.

Zinc powder (102 mg, 1.56 mmol) was added to a mixed solution of ethanol (0.5 ml) and water (0.75 ml), and copper iodide (99 mg, 0.52 mmol) was added thereto. The mixture was stirred under sonication for 5 minutes at room temperature. An ethanol solution (0.5 ml) of above crude product was added to the reaction solution, and the reaction solution was stirred under sonication for 1.5 hours at the same temperature. Then, the reaction solution was diluted with ethyl acetate, filtered through celite, and washed with distilled water and saturated brine. The organic layer was dried over anhydrous sodium sulfate and concentrated under reduced pressure. The thus obtained residue was purified by silica gel column chromatography (Kanto Chemical, trade name: Silica gel 60N, granular, 40 to 100 µm; heptane : ethyl acetate = 2.5:1) to obtain the title compound (110 mg) as a colorless oil. ¹H-NMR data of title compound obtained by this alternative method are completely identical to those of the title compound obtained by the above (20-2).

### Process (1-21): Synthesis of (2S, 3S, 4E)-2-((1E, 3E, 5R)-5-hydroxy-6-{(2R, 3R)-3-[(1R,2S)-2-hydroxy-1-methylbutyl]oxiran-2-yl}-1,5-dimethylhexa-1,3-dien-1-yl)-3-methyl-12-oxoazacyclododec-4-en-6-yl acetate (P15)

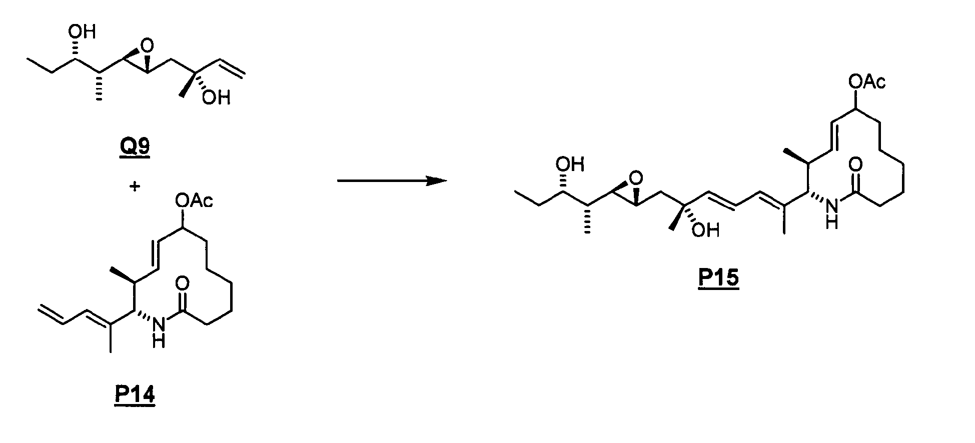

This reaction was carried out with reference to Grubbs, R.H., Handbook of Metathesis, Wiley-VCH, 2003, Vol. 2, p 246-292.

To a solution prepared by dissolving (2S, 3S, 4E)-3-methyl-2-[(1E)-1-methylbuta-1,3-dien-1-yl]-12-oxaazacyclododec-4-en-6-yl acetate (10.0 mg, 31.3 µmol) and (1R)-1,2-anhydro-3,5-dideoxy-1-[(1R,2S)-2-hydroxy-1-methylbutyl]-4-C-vinyl-D-erythr o-pentitol (9.4 mg, 43.9 µmol) in anhydrous dichloromethane (2.5 ml), the second generation Grubbs' catalyst, that is, [1,3-bis-(2,4,6)trimethylphenyl-2-imidazolidinylidene]dichloro(phenylmethylene)-tricycl ohexylphosphine)ruthenium (2.66 mg, 3.1 µmol), was added under an argon atmosphere. The reaction solution was heated to reflux for 2 hours. The residue obtained by concentrating the reaction solution under reduced pressure was purified by silica gel column chromatography (Merck, trade name: 20 PLC plate 20X20 Silica Gel 60 F₂₅₄, 0.5 mm; dichloromethane : methanol = 15: 1) to obtain the title compound (4.6 mg) as a white solid.

400MHz ¹H-NMR (CDCl₃) δ (ppm) 0.80-1.02 (m, 9H), 1.18-1.67 (m, 8H), 1.37 (s, 1.5H), 1.37 (s, 1.5H), 1.69-1.90 (m, 3H), 1.73 (s, 3H), 1.90-2.16 (m, 2H), 1.99 (s, 1.5H), 2.08 (s, 1.5H), 2.19-2.34 (m, 2H), 2.76 (dd, J = 2.2, 6.2 Hz, 1H), 2.94-3.13 (m, 1H), 3.55-3.72 (m, 3H), 4.18-4.29 (m, 1H), 5.17-5.47 (m, 2.5H), 5.58 (dd, J = 9.9, 14.7 Hz, 0.5H), 5.80 (d, J = 15.1 Hz, 1H), 6.10 (d, J = 10.8 Hz, 1H), 6.50 (dd, J = 10.8, 15.1 Hz, 1H); MS m/z 528.37 (M + Na)⁺.

### Example 2

### Synthesis Example of (2S, 3S, 4E)-2-((1E, 3E, 5S)-6-{(2R, 3R)-3-[(1R,2S)-2-hydroxy-1-methylbutyl]oxiran-2-yl}-1,5-dimethylhexa-1,3-dien-1-yl)-3-methyl-12-oxaazacyclododec-4-en-6-yl acetate (P16)

### Process (2-1): Synthesis of tert-butyl[((1S,2S)-1-ethyl-2-{(2R, 3R)-3-[(2S)-2-methylbut-3-en-1-yl]oxiran-2-yl}propyl)oxy]dimethylsilane (Q16)

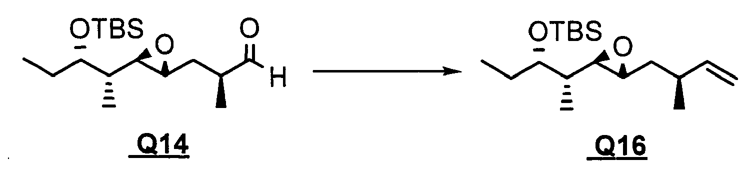

This process was carried out with reference to literatures, Matsubara, S., and 2 others, Synlett, 1998, Vol. 97, p 313-315.

To a THF solution (6 ml) of 20 wt% suspension of Nysted reagent ([cyclo-dibromo-di-µ-methylene(µ-tetrahydrofuran)trizinc]) in tetrahydrofuran (4.56 g, 2.0 mmol), a trifluoroborane dimethylether complex (71 mg, 0.5 mmol) was added at 0°C. The mixture was stirred for 5 minutes at the same temperature. A solution (2ml) of ((5R)-4,5-anhydro-5-((1S, 2S)-2-{[tert-butyl(dimethyl)silyl]oxy}-1-methylbutyl)-2,3-dideoxy-2-methyl-L-erythro-p entose (315 mg, 0.10 mmol) in THF was added to the reaction solution. The reaction solution was warmed up to a room temperature, and stirred for 3 hours. 1 M hydrochloric acid (3 ml) was added to the reaction solution. The reaction solution was diluted with hexane, washed sequentially with water and saturated brine, dried over anhydrous magnesium sulfate, and concentrated under reduced pressure. The thus obtained residue was purified by silica gel column chromatography (Kanto Chemical, trade name: Silica gel 60N, 0.040 to 0.100 mm; heptane : ethyl acetate = 50:1 → 5:1) to obtain the title compound (77 mg) as a colorless oil.

400MHz ¹H-NMR (CD₃OD) δ (ppm) 0.13 (s, 6H), 0. 88 (t, J = 7.6 Hz, 3H), 0.94 (d, J = 7.6 Hz, 3H), 0.96 (s, 9H), 1.10 (d, J = 6.8 Hz, 3H), 1.30-1.42 (m, 1H), 1.48-1.64 (m, 4H), 2.34-2.50 (m, 1H), 2.72 (dd, J = 2.4, 8.0 Hz, 1H), 2.75-2.85 (m, 1H), 3.69-3.82 (m, 1H), 5.01 (brd, J = 10.8 Hz, 1H), 5.08 (brd, J = 17.2 Hz, 1H), 5.72-5.86 (m, 1H); MS m/z 335.05 (M + Na)⁺.

### Process (2-2): Synthesis of (2R,3S)-2-{(2R,3R)-3-[(2S)-2-methylbut-3-en-1-yl]oxiran-2-yl}pentane-3-ol (R1)

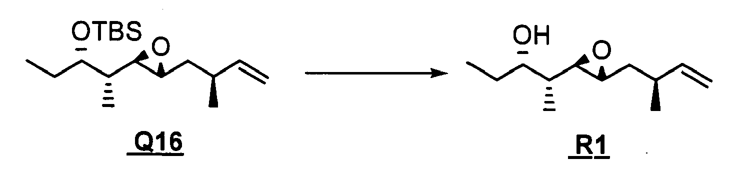

To a THF solution (10ml) of tert-butyl[((1S,2S)-1-ethyl-2-{(2R,3R)-3-[(2S)-2-methylbut-3-en-1-yl]oxiran-2-yl}propyl )oxy]dimethylsilane (400 mg, 1.28 mmol), a 1M THF solution of tetra-n-butylammoniumfluoride (5.12 ml) was added. The reaction solution was stirred for 13 hours at room temperature. The reaction solution was diluted by adding water, and then extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 3: 1) to obtain the title compound (254 mg) as a colorless oil.

400MHz ¹H-NMR (CDCl₃) δ (ppm) 0.96 (t, J = 8.4 Hz, 3H), 0.97 (d, J = 7.1 Hz, 3H), 1.07 (d, J = 6.8 Hz, 3H), 1.41-1.5 (m, 4H), 1.81-1.92 (brs, 1H), 2.33-2.46 (m, 1H), 2.69 (dd, J = 2.2, 7.1 Hz, 1H), 2.79-2.83 (m, 1H), 3.56-3.66 (brs, 1H), 4.99 (d, J = 10.3 Hz, 1H), 5.05 (d, J =17.2 Hz, 1H), 5.74 (ddd, J = 7.7,10.3,17.2 Hz, 1H); MS m/z 221.15 (M + Na)⁺.

### Process (2-3): Synthesis of (2S, 3S, 4E)-2-((1E, 3E, 5S)-6-{(2R, 3R)-3-[(1R,2S)-2-hydroxy-1-methylbutyl]oxiran-2-yl}-1,5-dimethylhexa-1,3-dien-1-yl)-3-methyl-12-oxaazacyclododec-4-en-6-yl acetate (P16)

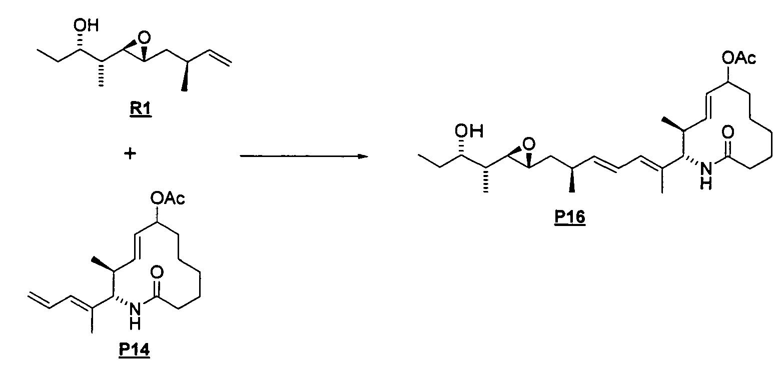

This reaction was carried out with reference to Grubbs, R.H., Handbook of Metathesis, Wiley-VCH, 2003, Vol. 2, p 246-292.

To a solution prepared by dissolving (2S, 3S, 4E)-3-methyl-2-[(1E)-1-methylbuta-1,3-dien-1-yl]-12-oxaazacyclododec-4-en-6-yl acetate (4.2 mg, 13.1 µmol) and (2R,3S)-2-{(2R,3R)-3-[(2S)-2-methylbut-3-en-1-yl]oxiran-2-yl}pentane-3-ol (11.9 mg, 60 µmol) in anhydrous dichloromethane (2 ml), the second generation Grubbs' catalyst, that is, [1,3-bis-(2,4,6)trimethylphenyl-2-imidazolidinylidene]dichloro(phenylmethylene)-tricycl ohexylphosphine)ruthenium (1.11 mg, 1.3 µmol), was added under a nitrogen atmosphere. The reaction solution was heated to reflux for 5 hours. The residue obtained by concentrating the reaction solution under reduced pressure was purified by silica gel column chromatography (Merck, trade name: 20 PLC plate 20X20 Silica Gel 60 F₂₅₄, 0.5 mm; n-heptane : ethyl acetate = 1:6) to obtain the title compound (2.8 mg) as a colorless oil.

400MHz ¹H-NMR (CDCl₃) δ (ppm) 0.90-1.00 (m, 9H), 1.02-1.12 (m, 3H), 1.17-1.88 (m, 13H), 1.70 (s, 3H), 1.89-2.06 (m, 1H), 2.01 (s, 3H), 2.20-2.34 (m, 2H), 2.38-2.52 (m, 1H), 2.64-2.72 (m, 1H), 2.74-2.81 (m, 1H), 3.53-3.64 (m, 1H), 4.16-4.34 (m,1H), 5.17-5,64 (m, 5H), 5.55-5.62 (m, 1.5H), 6.06 (d, J = 10.6 Hz, 1H), 6.24 (dd, J = 10.6, 15.2 Hz, 1H); MS m/z 512.35 (M + Na)⁺.

### Example 3

Synthesis of Example (2S, 3S, 4E)-2-((1E, 3E, 5S)-6-{(2R, 3R)-3-[(1R,2S)-2-hydroxy-1-methylbutyl]oxiran-2-yl}-1,5-dimethylhexa-1,3-dien-1-yl)-1,3-dimethyl-12-oxaazacyclododec-4-en-6-yl acetate (P24)

### Process (3-1): Synthesis of N-{(1S,2E)-4-(benzyloxy)-1-[(1S, 2E)-3-iodo-1-methylprop-2-en-1-yl]-2-methylbut-2-en-1-yl}-2-nitroaniline (P17)

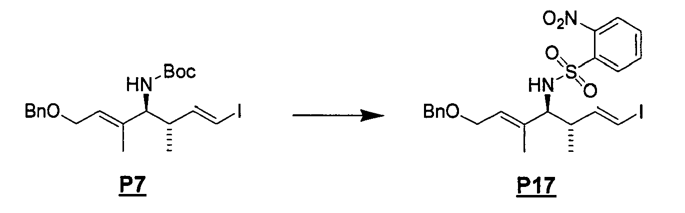

To tert-butyl{(1S, 2E)-4-(benzyloxy)-1-[(1S, 2E)-3-iodo-1-methylprop-2-en-1-yl]-2-methylbut-2-en-1-yl}carbamate (910 mg, 1.93 mmol), hydrogen chloride (4N in ethyl acetate, 20ml) was added. The reaction solution was stirred for 1 hour at room temperature, and concentrated under reduced pressure. The thus obtained residue was diluted with ethyl acetate, and washed sequentially with saturated aqueous solution of sodium bicarbonate, water and saturated brine. The organic layer was dried over anhydrous magnesium sulfate, and concentrated under reduced pressure.

The thus obtained residue was dissolved in dichloromethane (10 ml), and triethylamine (808 µl, 5.79 mmol) and 2-nitrobenzenesulfonylchloride (4.28 mg, 1.93 mmol) were added thereto. The reaction solution was stirred for 3 hours at room temperature. To the reaction solution, saturated aqueous solution of ammonium chloride was added, and extracted with chloroform. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 6:1 → 2:1) to obtain the title compound (935 mg) as a colorless oil.

400MHz ¹H-NMR (CDCl₃) δ (ppm) 0.93(d, J = 6.8 Hz, 3H), 1.41 (s, 3H), 2.32-2.44 (m, 1H), 3.62-3.70 (m, 1H), 3.76-3.94 (m, 2H), 4.41(s, 1H), 4.42 (s, 1H), 5.46 (d, J = 7.9 Hz, 1H), 5.49 (t, J = 5.9 Hz, 1H), 6.22 (d, J = 14.5 Hz, 1H), 6.27 (dd, J = 7.7, 14.5 Hz, 1H), 7.27-7.39 (m, 5H), 7.54-7.63 (m, 2H), 7.77-7.82 (m, 1H), 7.98-8.04 (m, 1H); MS m/z 579.10 (M + Na)⁺.

### Process (3-2): Synthesis of N-{(1S, 2E)-4-(benzyloxy)-1-[(1S, 2E)-3-iodo-1-methylprop-2-en-1-yl]-2-methylbut-2-en-1-yl} -N-methyl-2-nitroaniline (P 18)

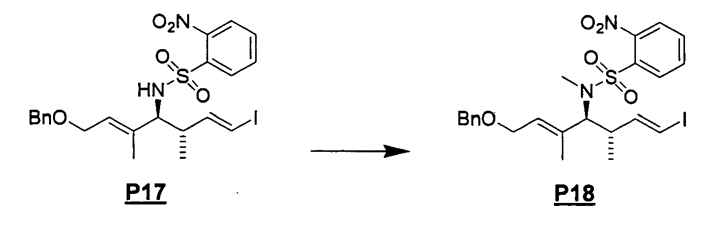

To a DMF solution (16 ml) of N-{(1S, 2E)-4-(benzyloxy)-1-[(1S, 2E)-3-iodo-1-methylprop-2-en-1-yl]-2-methylbut-2-en-1-yl}-2-nitroaniline (935 mg, 1.68 mmol), potassium carbonate (1.86 g, 13.5 mmol) and methyl iodide (418 µl, 6.73 mmol) were added under a nitrogen atmosphere. The reaction solution was stirred for 68 hours at room temperature. Water was added to the reaction solution, and then extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 2:1) to obtain the title compound (912 mg) as a colorless oil.

400MHz ¹H-NMR (CDCl₃) δ (ppm) 1.00 (d, J = 6.8 Hz, 3H), 1.66 (s, 3H), 2.65-2.76 (m, 1H), 2.83 (s, 3H), 4.07 (d, J = 6.0 Hz, 1H), 4.08 (d, J = 6.0 Hz, 1H), 4.18 (d, J = 11.0 Hz, 1H), 4.49 (s, 2H), 5.58 (t, J = 6.0 Hz, 1H), 6.08 (d, J = 14.3 Hz, 1H), 6.24 (dd, J = 9.2, 14.3 Hz, 1H), 7.27-7.39 (m, 5H), 7.55-7.62 (m, 1H), 7.62-7.71 (m, 2H), 7.93-7.98 (m, 1H); MS m/z 609.06 (M + Na)⁺.

### Process (3-3): Synthesis of N-{(1S, 2E)-4-(benzyloxy)-1-[(1S, 2E)-3-iodo-1-methylprop-2-en-1-yl]-2-methylbut-2-en-1-yl}-7-hydroxy-N-methylheptan amide (P 19)

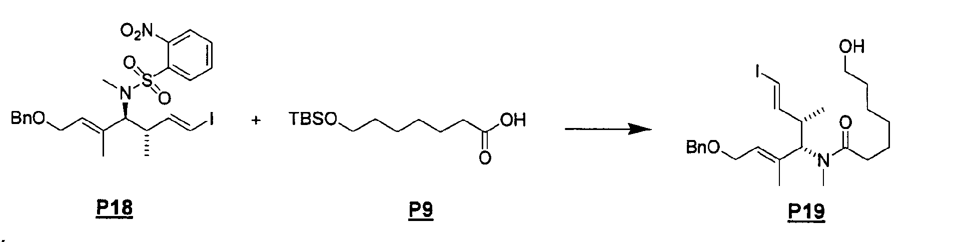

To a DMF solution (16 ml) of N-{(1S, 2E)-4-(benzyloxy)-1-[(1S, 2E)-3-iodo-1-methylprop-2-en-1-yl]-2-methylbut-2-en-1-yl}-N-methyl-2-nitroaniline (912 mg, 1.60mmol), lithium hydroxide (153 mg, 6.38 mmol) and thioglycolic acid (222 µl, 3.19 mmol) were added under a nitrogen atmosphere. The reaction solution was stirred for 2.5 hours at room temperature. Saturated aqueous solution of sodium bicarbonate was added to the reaction solution, and then extraction with diethyl ether was performed. The organic layer was washed with saturated aqueous solution of sodium bicarbonate, water and saturated brine, dried over anhydrous magnesium sulfate, and concentrated under reduced pressure.

The obtained residue and 7-{[tert-butyl(dimethyl)silyl]oxy}heptanoate (658 mg, 2.54 mmol) were dissolved in DMF (30ml). To this solution, diisopropylamine (1.17 ml, 6.73 mmol) and PyBOP, that is, benzotriazole-1-yloxytris(pyrrolidine)phosphonium hexafluorophosphate (1.31 g, 2.54 mmol) were added sequentially. The mixture was stirred for 1 day at room temperature. The reaction solution was diluted with saturated aqueous solution of ammonium chloride, and then extracted with ethyl acetate. The organic layer was washed with saturated brine, dried over anhydrous magnesium sulfate, and concentrated under reduced pressure.

To a solution obtained by dissolving the thus obtained residue in THF (16 ml), a 1M THF solution (8.45 ml) of tetra-n-butylammonium fluoride was added. The mixture was stirred for 19.5 hours at room temperature. The reaction solution was diluted with water, and then extracted with ethyl acetate. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 2:1) to obtain the title compound (550 mg) as a colorless oil.

400MHz ¹H-NMR (CDCl₃) δ (ppm) 1.06 (d, J = 6.6 Hz, 3H), 1.34-1.47 (m, 2H), 1.55(s, 3H), 1.57-1.72 (m, 6H), 2.22-2.35 (m, 2H), 2.63 (s, 3H), 2.64-2.72 (m, 1H), 3.65 (t, J = 6.6 Hz, 2H), 4.13 (d, J = 6.2 Hz, 2H), 4.51 (d, J = 12.3 Hz, 1H), 4.53 (d, J = 12.3 Hz, 1H), 5.01 (d, J = 10.8 Hz, 1H), 5.54 (t, J = 6.2 Hz, 1H), 6.03 (d, J = 14.3 Hz, 1H), 6.36 (dd, J = 9.7, 14.3Hz, 1H), 7.26-7.39 (m, 5H); MS m/z 536.20 (M + Na)⁺.

### Process (3-4): Synthesis of (9E, 11S, 12S)-12-[(1E)-3-benzyloxy-1-methylprop-1-en-1-yl]-8-[tert-butyl(dimethyl)silyl]oxy}-1,11-dimethylazacyclododec-9-en-2-one (P20)

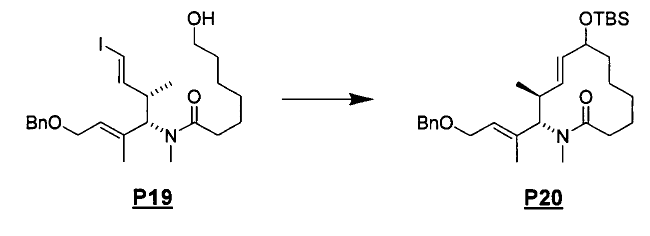

This process was carried out with reference to literatures, Furstner, A., Chemical Reviews, 1999, Vol. 99, p 991-1045; Stamos, D.P. and 3 others, Tetrahedron Letters, 1997, Vol. 38, Issue No. 36, p 6355-6358; Pilli, R.A. and 3 others, The Journal of Organic Chemistry, 1998, Vol. 63, p 7811-7819.

In the process, DMF was subjected to freezing degasification just before it was used. Also, chromium (II) chloride and nickel (II) chloride were dried for 5 hours at 200°C under reduced pressure using a vacuum pump just before they were used.

To a dichloromethane solution (2 ml) of N-{(1S, 2E)-4-(benzyloxy)-1-[(1S, 2E)-3-iodo-1-methylprop-2-en-1-yl]-2-methylbut-2-en-1-yl}-7-hydroxy-N-methylheptan amide (100 mg, 0.195 mmol), a Dess-Martin reagent (165 mg, 0.39 mmol) was added at room temperature, and the reaction solution was stirred at room temperature for 2 hours. The reaction solution was diluted with ethyl acetate, and washed with saturated aqueous solution of sodium bicarbonate containing sodium sulfite and saturated brine in said order. The organic layer was dried over anhydrous magnesium sulfate, and concentrated under reduced pressure.

The solution prepared by dissolving the obtained residue in DMF (4 ml) was added dropwise to a DMF solution (26 ml) of chromium (II) chloride (144 mg, 1.17 mmol) and nickel (II) chloride (1.52 mg, 11.7 µmol) at 0°C under an argon atmosphere. The reaction solution was stirred at room temperature for 13 hours. To this reaction solution, 1M serine sodium salt aqueous solution (20 ml) prepared by dissolving in saturated aqueous solution of sodium bicarbonate was added, and then the reaction solution was stirred until it gave blackish-purple color. After diluting the reaction solution by adding water, extraction with ethyl acetate was performed. The organic layer was washed with saturated brine, dried over anhydrous magnesium sulfate, and concentrated under reduced pressure.

The thus obtained residue was dissolved in DMF (3ml), and imidazole (79.7 mg, 1.17 mmol) and tert-butyldimethylsilylchloride (90.9 mg, 0.585 mmol) were added sequentially thereto. The mixture was stirred at room temperature for 1 day. After diluting the reaction solution by adding water, extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 3:1) to obtain the title compound (57 mg) as a colorless oil.

400MHz ¹H-NMR (CDCl₃) δ (ppm) 0.00 (s, 1.5H), 0.01 (s, 1.5H), 0.01 (s, 1.5H), 0.02 (s, 1.5H), 0.86 (s, 4.5H), 0.89 (s, 4.5H), 1.06 (d, J = 6.2 Hz, 1.5H), 1.07 (d, J = 6.2 Hz, 1.5H), 1.12-1.56 (m, 4H), 1.59 (s, 1.5H), 1.60 (s, 1.5H), 1.63-1.72 (m, 2H), 1.89-2.12 (m, 2H), 2.53-2.67 (m, 2H), 2.76 (s, 1.5H), 2.77(s, 1.5H), 4.06-4.15 (m, 1H), 4.18 (d, J = 13.7 Hz, 2H), 4.53 (d, J = 12.1 Hz, 1H), 4.54 (d, J = 12.1 Hz, 1H), 5.05 (d, J = 11.4 Hz, 1H), 5.22 (dd, J = 8.8, 15.4 Hz, 0.5H), 5.34 (dd, J = 9.7, 15.4 Hz, 0.5H), 5.40 (dd, J = 3.8, 15.4 Hz, 0.5H), 5.44 (dd, J = 3.5, 9.7 Hz, 0.5H), 5.48-5.60 (m, 1.5H), 5.64-5.70 (m, 0.5H), 7.28-7.39 (m, 5H); MS m/z 522.39 (M + Na)⁺.

### Process (3-5): Synthesis of (9E, 11S, 12S)-8-[tert-butyl(dimethyl)silyl]oxy-12-[(1E)-3-hydroxy-1-methylprop-1-en-1-yl]-1,11-dimethylazacyclododec-9-en-2-one (P21)

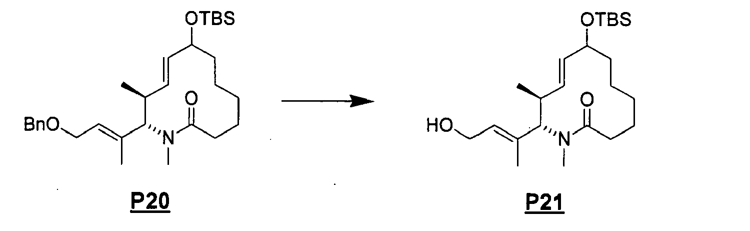

This process was carried out with reference to Ireland, R.E. and 3 others, Journal of the American Chemical Society, 1985, Vol. 107, p 3285-3294.

In the process, anhydrous THF distilled from benzophenone ketyl just before it was used. Also, lithium-4,4'-di-tert-butylbiphenyl to be used in the process was prepared just before its use in accordance with an common method using lithium (15.8 mg, 2.28 mmol) and 4,4'-di-tert-butylbiphenyl (304 mg, 1.14 mmol).

To an anhydrous THF solution (2 ml) of (9E, 11 S, 12S)-12-[(1E)-3-benzyloxy-1-methylprop-1-en-1-yl]-8-{[tert-butyl(dimethyl)silyl]oxy}-1,11-dimethylazacyclododec-9-en-2-one (57 mg, 0.114 mmol), an anhydrous THF solution (10 ml) of lithium-4,4'-di-tert-butylbiphenyl was added dropwise at -78°C under a nitrogen atmosphere until the reaction solution gives dark blue color. After stirring the reaction solution at -78°C for 30 minutes, it was diluted by adding saturated aqueous solution of ammonium chloride, and then extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 1:2) to obtain the title compound (33.6 mg) as a colorless oil.

400MHz ¹H-NMR (CDCl₃) δ (ppm) 0.02 (s, 1.5H), 0.02 (s, 1.5H), 0.03 (s, 1.5H), 0.04 (s, 1.5H), 0.88 (s, 4.5H), 0.90 (s, 4.5H), 1.06 (d, J = 7.2 Hz, 1.5H), 1.08 (d, J = 7.2 Hz, 1.5H), 1.13-1.83 (m, 6H), 1.63 (s, 1.5H), 1.65 (s, 1.5H), 1.88-2.14 (m, 2H), 2.53-2.71 (m, 2H), 2.79 (s, 1.5H), 2.79 (s, 1.5H), 4.06-4.18 (m, 1H), 4.31 (s, 1H), 4.32 (s, 1H), 5.06 (d, J = 11.4 Hz, 1H), 5.24 (dd, J = 8.8, 15.2 Hz, 0.5H), 5.35 (dd, J = 9.7, 15.2 Hz, 0.5H), 5.42 (dd, J = 3.7, 15.2 Hz, 0.5H), 5.45 (dd, J = 3.7, 9.7 Hz, 0.5H), 5.49-5.63 (m, 1.5H), 5.64-5.70 (m, 0.5H); MS m/z 432.25 (M + Na)⁺.

### Process (3-6): Synthesis of (9E, 11 S, 12S)-8-[tert-butyl(dimethyl)silyl]oxy-1,11-dimethyl-12-[(1E)-1-methylbuta-1,3-dien-1-yl ]azacyclododec-9-en-2-one (P22)

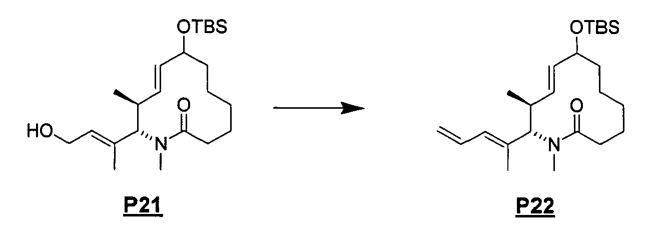

To a dichloromethane solution (2 ml) of (9E, 11 S, 12S)-8-[tert-butyl(dimethyl)silyl]oxy-12-[(1E)-3-hydroxy-1-methylprop-1-en-1-yl]-1,11-dimethylazacyclododec-9-en-2-one (33.6 mg, 82 µmol), a Dess-Martin reagent (52.2 mg, 0.123 mmol) was added at room temperature, and the reaction solution was stirred at room temperature for 30 minutes. The reaction solution was diluted with ethyl acetate, and washed sequentially with a saturated aqueous solution of sodium bicarbonate containing sodium sulfite and saturated brine. The organic layer was dried over anhydrous magnesium sulfate, and concentrated under reduced pressure.

The solution prepared by dissolving the obtained residue in anhydrous THF (1ml) was added dropwise to an anhydrous THF solution (1 ml) of methylenetriphenylphosphorane prepared by a common method from a solution of methyltriphenylphosphonium iodide (49.7 mg, 0.123 mmol) and n-butylithium (2.77M, n-hexane, 44.4 µl, 0.123 mmol) under a nitrogen atmosphere, at 0°C. The mixture was stirred at the same temperature for 30 minutes. After diluting the reaction solution by adding a saturated aqueous solution of ammonium chloride, extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 2: 1) to obtain the title compound (24.1 mg) as a white solid.

400MHz ¹H-NMR (CDCl₃) δ (ppm) 0.01 (s, 1.5H), 0.02 (s, 1.5H), 0.02 (s, 1.5H), 0.03 (s, 1.5H), 0.87 (s, 4.5H), 0.87 (s, 4.5H), 1.04 (d, J = 6.4 Hz, 1.5H), 1.06 (d, J = 6.4 Hz, 1.5H), 1.12-1.70 (m, 6H), 1.72 (s, 1.5H), 1.74 (s, 1.5H), 1.88-2.12 (m, 2H), 2.53-2.69 (m, 2H), 2.75 (s, 1.5H), 2.77 (s, 1.5H), 4.06-4.18 (m, 1H), 5.07-5.31 (m, 3.5H), 5.36 (dd, J = 9.7, 15.2 Hz, 0.5H), 5.38-5.45 (m, 0.5H), 5.41 (dd, J = 3.7, 15.4 Hz, 0.5H), 5.48 (dd, J = 3.8, 15.8 Hz, 0.5H), 5.57 (ddd, J = 1.7, 9.5, 15.2Hz, 0.5H), 6.02 (dd, J = 10.6, 27.8 Hz, 1H), 6.58-6.69 (m, 1H); MS m/z 428.28 (M + Na)⁺.

### Process (3-7): Synthesis of (2S, 3S, 4E)-1,3-dimethyl-2-[(1E)-1-methylbuta-1,3-dien-1-yl]-12-oxoazacylododec-4-en-6-yl acetate (P23)

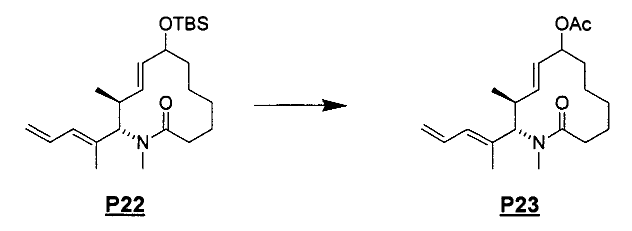

To a solution (2 ml) of (9E, 11 S, 12S)-8-[tert-butyl(dimethyl)silyl]oxy-1,11-dimethyl-12-[(1E)-1-methylbuta-1,3-dien-1-yl ] azacyclododec-9-en-2-one (24.1 mg, 59.4 µl) in THF, a 1M THF solution (267 µl) of tetra-n-butylammonium fluoride was added. The mixture was stirred at room temperature for 23 hours. After diluting the reaction solution by adding water, extraction with ethyl acetate was performed. The organic layer was washed with saturated brine, dried over anhydrous magnesium sulfate, and concentrated under reduced pressure.

The thus obtained residue was dissolved in dichloromethane (1 ml), and triethylamine (41.5 µl, 0.297 mmol), 4-dimethylaminopyridine (3.63 mg, 29.7 µmol) and acetic anhydride (16.8 µl, 0.178 mmol) were added sequentially thereto. The mixture was stirred at room temperature for 1 hour. After diluting the reaction solution by adding water, extraction with ethyl acetate was performed. The organic layer was washed with saturated brine, dried over anhydrous magnesium sulfate, and concentrated under reduced pressure. The thus obtained residue was purified by silica gel column chromatography (Kanto Chemical, trade name: Silica gel 60N, granular, 0.040 to 0.100 mm; n-heptane : ethyl acetate = 2:1) to obtain the title compound (19.5 mg) as a colorless oil.

400MHz ¹H-NMR (CDCl₃) δ (ppm) 1.04 (d, J = 4.9 Hz, 1.5H), 1.05 (d, J = 4.9 Hz, 1.5H), 1.18-1.84 (m, 6H), 1.72 (s, 1.5H), 1.73 (s, 1.5H), 1.87-2.16 (m, 2H), 2.01 (s, 1.5H), 2.01 (s, 1.5H), 2.53-2.69 (m, 2H), 2.76 (s, 1.5H), 2.77 (s, 1.5H), 5.19-5.32 (m, 4.5H), 5.38 (ddd, J = 1.7, 9.5, 15.4 Hz, 0.5H), 5.45 (dd, J = 4.0, 15.6 Hz, 0.5H), 5.54 (ddd, J = 1.1, 9.2, 15.6 Hz, 0.5H), 5.59 (dd, J = 10.1, 14.7 Hz, 0.5H), 5.78 (dd, J = 4.4, 15.4 Hz, 0.5H), 5.94-6.45 (m, 1H), 6.57-6.68 (m, 1H); MS m/z 356.14 (M + Na)⁺.

### Process (3-8): Synthesis of (2S, 3S, 4E)-2-((1E, 3E, 5S)-6-{(2R, 3R)-3-[(1R,2S)-2-hydroxy-1-methylbutyl]oxiran-2-yl}-1,5-dimethylhexa-1,3-dien-1-yl)-1,3-dimethyl-12-oxaazacyclododec-4-en-6-yl acetate (P24)

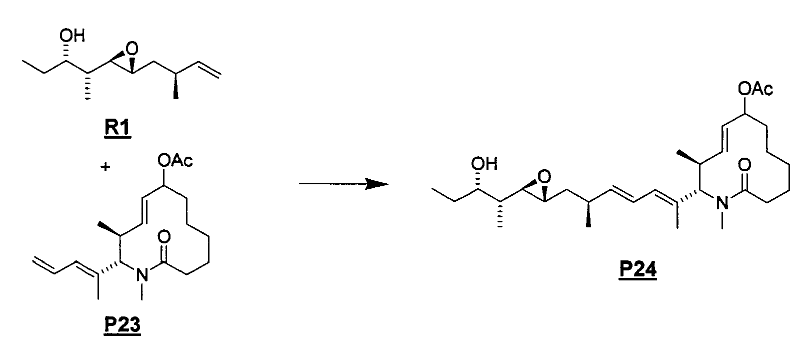

This reaction was carried out with reference to Grubbs, R.H., Handbook of Metathesis, Wiley-VCH, 2003, Vol. 2, p 246-292.

To a solution prepared by dissolving (2S, 3S, 4E)-1,3-dimethyl-2-[(1E)-1-methylbuta-1,3-dien-1-yl]-12-oxoazacylododec-4-en-6-yl acetate (19.5 mg, 58.5 µmol) and (2R,3S)-2-{(2R,3R)-3-[(2S)-2-methylbut-3-en-1-yl]oxiran-2-yl}pentane-3-ol (34.8 mg, 0.176 mmol) in anhydrous dichloromethane (1 ml), the second generation Grubbs' catalyst, that is, [1,3-bis-(2,4,6trimethylphenyl)-2-imidazolidinylidene]dichloro(phenylmethylene)-tricycl ohexylphosphine)ruthenium (4.97 mg, 5.9 µmol) was added under a nitrogen atmosphere. The reaction solution was heated to reflux for 3 hours. The residue obtained by concentrating the reaction solution under reduced pressure was purified by silica gel column chromatography (Merck, trade name: 20 PLC plate 20X20 Silica Gel 60 F₂₅₄, 0.5 mm; n-heptane: ethyl acetate = 1:3) to obtain the title compound (0.6 mg; 0.5 of Rf value; developing solvent, n-heptane : ethyl acetate = 1:3) as a white solid.

MS m/z 526.40 (M + Na)⁺.

### Example 4

### Synthesis Example of (1R)-4-C-{(1E, 3E)-4-[(2S, 4E, 6S, 7R, 10R)-6-(acetyloxy)-7,10-dihydroxy-7-methyl-12-oxoazacyclododec-4-en-2-yl]penta-1,3-dien-1-yl}-1,2-anhydro-3,5-dideoxy-1-[(1R, 2S)-2-hydroxy-1-methylbutyl]-D-erythro-pentitol (P36)

### Process (4-1): Synthesis of tert-butyl(4R, 5S)-4-[1-(acetyloxy)prop-2-en-1-yl]-2,2,5-trimethyl-1,3-oxazolidine-3-carboxylate (P25)

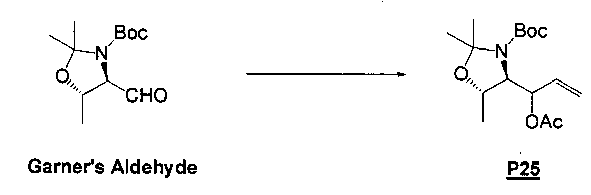

To a solution (40.0 ml) of Garner's aldehyde (2.00 g, 8.22mmol) prepared from D-threonine (reference literature: Organic Synthesis, Coll. Vo1.9, p 300 (1992); Koskinen, A.M.P; Otsomaa, L.A; Tetrahedron, 1997, 53 (18), p 6473-6484) in anhydrous THF, a 1.44M THF solution (6.85 ml, 9.86mmol) of vinylmagnesium chloride was added dropwise under a nitrogen atmosphere. The reaction solution was stirred for 1 hour at -78°C, and warmed up to a room temperature by adding saturated aqueous solution of ammonium chloride. The reaction solution was extracted with ethyl acetate. The organic layer was washed with water and saturated brine, dried over anhydrous magnesium sulfate, and concentrated under reduced pressure.

The thus obtained residue was dissolved in anhydrous dichloromethane (22.0 ml), pyridine (1.95 ml, 24.1 mmol), acetic anhydride (2.28 ml, 24.1 mmol) and 4-dimethylaminopyridine (98.1 mg, 803 µmol) were added thereto at room temperature. The reaction solution was stirred for 5 hours at room temperature, and concentrated under reduced pressure. The thus obtained residue was purified by silica gel column chromatography (Yamazen, trade name: Hi-Flash Column, Silica gel, 40 µm, 60Å; n-heptane : ethyl acetate = 2:1, Rf = 0.65) to obtain the title compound (1.84 g) as a colorless oil.

MS m/z 335.90 (M + Na)⁺.

### Process (4-2): Synthesis of tert-butyl(4S, 5S)-4-allyl-2,2,5-trimethyl-1,3-oxazolidine-3-carboxylate (P26)

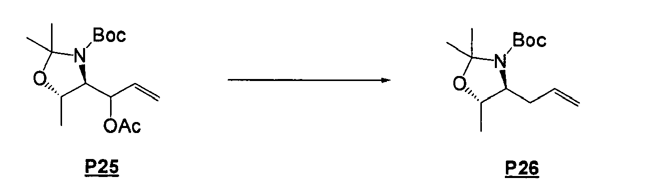

To a DMF solution (72.0 ml) of tert-butyl(4R, 5S)-4-[1-(acetyloxy)prop-2-en-1-yl]-2,2,5-trimethyl-1,3-oxazolidine-3-carboxylate (1.44 g, 4.59 mmol), tetrakistriphenylphosphinepalladium (531 mg, 459 µmol), sodium formate (3.75 g, 55.2 mmol) and triethylamine (10.9 ml, 77.8 mmol) were added under a nitrogen atmosphere. The reaction solution was stirred for 8 hours at 65°C, cooled to room temperature, diluted with diethyl ether, and washed with water and saturated brine. The organic layer was dried over anhydrous magnesium sulfate, and concentrated under reduced pressure. The thus obtained residue was purified by silica gel column chromatography (Yamazen, trade name: Hi-Flash Column, Silica gel, 40 µm, 60Å; n-heptane : ethyl acetate = 4:1, Rf = 0.7) to obtain the title compound (800 mg) as a colorless oil.

400MHz ¹H-NMR (CDCl₃) δ (ppm) 1.27-1.30 (m, 3H), 1.45 (brs, 3H), 1.48 (s, 9H), 1.58 (brs, 3H), 2.51 (brs, 2H), 3.47 (brs, 1H), 3.97 (m, 1H), 5.08 (brs, 1H), 5.11 (brs, 1H), 5.73 (m, 1H).

### Process (4-3): Synthesis of ethyl (2E, 4S)-4-[(tert-butoxycarbonyl)amino]-3-methylhepta-2,6-dienoate (P27)

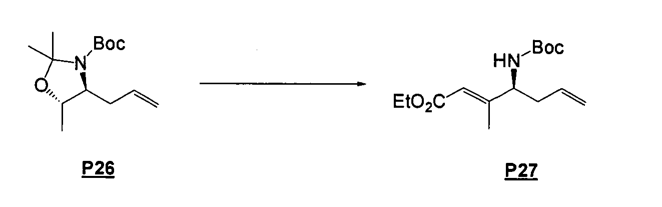

To a methanol solution (36.0 ml) of tert-butyl(4S, 5S)-4-allyl-2,2,5-trimethyl-1,3-oxazolidine-3-carboxylate (888 mg, 3.47 mmol), camphorsulfonic acid (40.3 mg, 174 µmol) was added. The mixture was stirred at room temperature for 4 hours. The reaction solution was diluted with ethyl acetate, and washed with water and saturated brine. The organic layer was dried over anhydrous magnesium sulfate, and concentrated under reduced pressure.

The thus obtained residue was dissolved in dichloromethane (21.0 ml), a Dess-Martin reagent (1.74 g, 4.10 mmol) was added thereto. The mixture was stirred at room temperature for 3 and a half hours. The reaction solution was diluted with ethyl acetate, and washed with saturated aqueous solution of sodium bicarbonate, aqueous solution of sodium sulfite, water and saturated brine. The organic layer was concentrated under reduced pressure.

The thus obtained residue was dissolved in anhydrous THF (10.0 ml), and added dropwise to a solution separately prepared by adding sodium hydride (60%, 342 mg, 8.55 mmol) to a solution (20.0 ml) of triethylphosphonoacetate (2.05 ml, 10.3 mmol) in anhydrous THF and stirring for 20 minutes at 0°C. The reaction solution was stirred for 1 and a half hours at 0°C, and further stirred for 40 minutes at room temperature. The reaction solution was poured into saturated aqueous solution of ammonium chloride, and then extraction with ethyl acetate was performed. The organic layer was washed with water and saturated brine, dried over anhydrous magnesium sulfate, and concentrated under reduced pressure. The thus obtained residue was purified by silica gel column chromatography (Yamazen, trade name: Hi-Flash Column, Silica gel, 40 µm, 60Å; n-heptane : ethyl acetate = 2:1, Rf = 0.6) to obtain the title compound (600 mg) as a colorless oil.

400MHz ¹H-NMR (CDCl₃) δ (ppm) 1.27 (t, J = 7.0 Hz, 3H), 1.43 (s, 9H), 2.15 (s, 3H), 2.31 (m, 1H), 2.35 (m, 1H), 4.13 (m, 1H), 4.15 (q, J = 7.0 Hz, 2H), 4.66 (brs, 1H), 5.11 (m, 1H), 5.15 (m, 1H), 5.66 (m, 1H), 5.78 (m, 1H).

### Process (4-4): Synthesis of tert-butyl[(1S, 2E)-1-allyl-4-hydroxy-2-methylbut-2-en-1-yl]carbamate (P28)

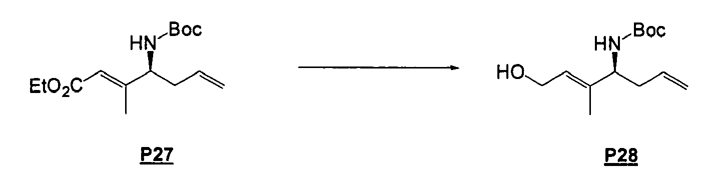

To a solution of ethyl (2E, 4S)-4-[(tert-butoxycarbonyl)amino]-3-methyhepta-2,6-dienoate (100 mg, 353 µmol) in anhydrous dichloromethane (3.0 ml), trifluoroborane diethylether complex (44.7 µl, 353 µmol) was added at -78°C under a nitrogen atmosphere. The mixture was stirred for 30 minutes at the same temperature. Diisobutylaluminum hydride (0.98 M, an n-hexane solution, 1.08 ml, 1.06 mmol) was added dropwise to the reaction solution. The reaction solution was stirred for 1 hour at the same temperature, and further stirred for 1 hour at room temperature after adding an aqueous solution of Rochelle salt, and then, extraction with ethyl acetate was performed. The organic layer was washed with saturated brine, dried over anhydrous magnesium sulfate, and concentrated under reduced pressure. The thus obtained residue was purified by silica gel column chromatography (Yamazen, trade name: Hi-Flash Column, Silica gel, 40 µm, 60Å; n-heptane : ethyl acetate = 2:1, Rf = 0.15) to obtain the title compound (72.3 mg) as a colorless oil.

400MHz ¹H-NMR (CDCl₃) δ (ppm) 1.28 (brs, 1H), 1.43 (s, 9H), 1.67 (s, 3H), 2.23-2.47 (m, 2H), 4.06 (brs, 1H), 4.20 (dd, J = 5.4, 5.4 Hz, 2H), 4.59 (brs, 1H), 5.08 (d, J = 1.2 Hz, 1H), 5.11 (dd, J = 1.2, 9.2 Hz, 1H), 5.57 (brt, J = 6.8 Hz, 1H), 5.69(m, 1H).

### Process (4-5): Synthesis of (2E, 4S)-4-[(tert-butoxycarbonyl)amino]-3-methylhepta-2,6-dien-1-yl benzoate (P29)

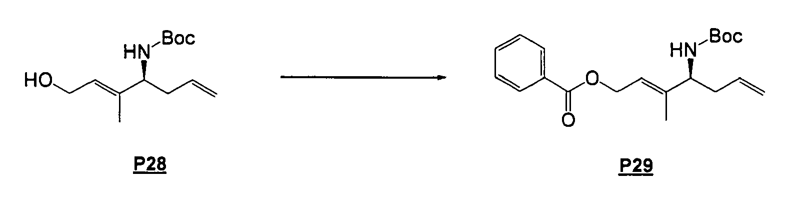

To a solution (33.0 ml) of tert-butyl[(1S, 2E)-1-allyl-4-hydroxy-2-methylbut-2-en-1-yl]carbamate (1.62 g, 6.71 mmol) in anhydrous dichloromethane, benzoic anhydride (1.82 g, 8.05 mmol), pyridine (1.30 ml, 16.1 mmol), and 4-dimethylaminopyridine (82.0 mg, 671 µmol) were added. The mixture was stirred for 2 and a half hours at room temperature, and further stirred for 6 hours after adding benzoic anhydride (1.82 g, 8.05 mmol) thereto. The reaction solution was poured into water, and then extraction with ethyl acetate was performed. The organic layer was washed with 2N hydrochloric acid, saturated aqueous solution of sodium bicarbonate, water and saturated brine, dried over anhydrous magnesium sulfate, and concentrated under reduced pressure. The thus obtained residue was purified by silica gel column chromatography (Yamazen, trade name: Hi-Flash Column, Amino, 40 µm, 60Å; n-heptane : ethyl acetate = 1:1, Rf = 0.5) to obtain the title compound (2.17 g) as a colorless oil.

400MHz ¹H-NMR (CDCl₃) δ (ppm) 1.24 (s, 9H), 1.77 (s, 3H), 2.28-2.35 (m, 2H), 4.11 (brs, 1H), 4.60 (brs, 1H), 4.88 (d, J = 6.8 Hz, 2H), 5.07-5.12 (m, 2H), 5.61-5.75 (m, 2H), 7.41-7.45 (m, 2H), 7.56 (m, 1H), 8.03-8.04 (m, 2H).

### Process (4-6): Synthesis of (2E, 4S)-4-amino-3-methylhepta-2,6-dien-1-yl benzoate hydrochloride (P30)

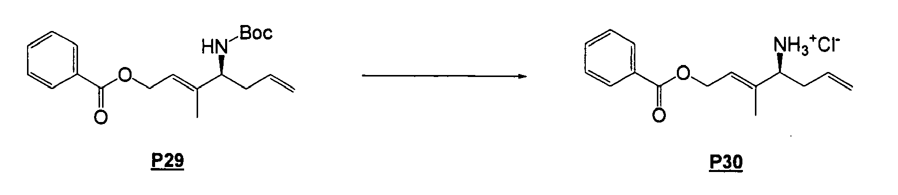

(2E, 4S)-4-[(tert-butoxycarbonyl)amino]-3-methylhepta-2,6-dien-1-yl benzoate (2.17 g, 6.28 mmol) was added to hydrogen chloride (4N in ethylacetate, 40.0ml), and the mixture was stirred for 40 minutes at 0°C. The reaction solution was concentrated under reduced pressure, thereby obtaining the title compound (1.66 g) as a white solid. This title compound was used in the subsequent process, without subjecting any more purification.

400MHz ¹H-NMR (DMSO-d₆) δ (ppm) 1.91 (s,3H), 2.50-2.62 (m, 2H), 3.85 (t, J = 7.2 Hz, 1H), 4.99 (d, J = 6.8 Hz, 2H), 5.21-5.30 (m, 2H), 5.72-5.82 (m, 1H), 5.87 (t, J = 6.8 Hz, 1H), 7.51-7.55 (m, 2H), 7.66 (m, 1H), 8.05-8.07 (m, 2H).

### Process (4-7): Synthesis of (2E, 4S)-4-({(3R)-3-{[tert-butyl(dimethyl)silyl]oxy}-5-[(2S,4R, 5S)-4-methyl-2-phenyl-5-vinyl-1,3-dioxolan-4-yl]pentanoyl}amino)-3-methylhepta-2,6-dien-1-yl benzoate (P31)

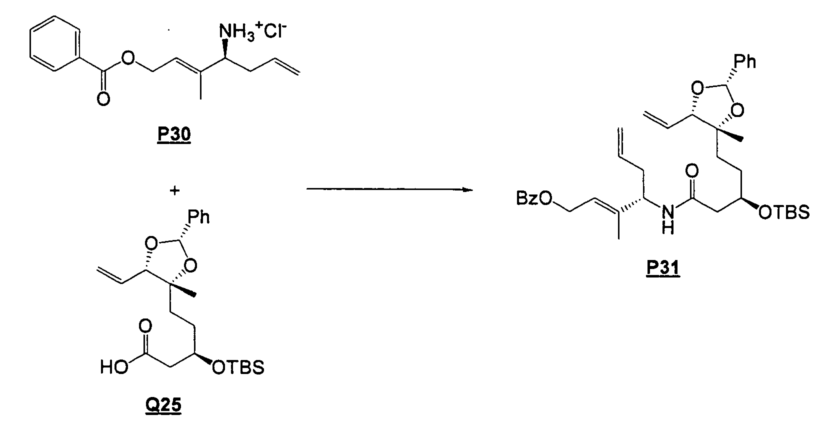

To a DMF solution (6.20 ml) of (2E, 4S)-4-amino-3-methylhepta-2,6-dien-1-yl benzoate hydrochloride (257 mg, 911 µmol), (3R)-3-{[tert-butyl(dimethyl)silyl]oxy}-5-[(2S, 4R, 5S)-4-methyl-2-phenyl-5-vinyl-1,3-dioxolan-4-yl]pentanoic acid (319 mg, 759 µmol), 1-hydroxybenzotriazole (205 mg, 1.52 mmol), 1-ethyl-3-(3-dimethylaminopropyl) carbodiimide hydrochloride (291 mg, 1.52 mmol), and triethylamine (741 µl, 5.31 mmol) were added. The mixture was stirred for 16 hours at room temperature, and further stirred for 1 and a half hours after adding 1-hydroxybenzotriazole (20.0 mg, 104 µmol), 1-ethyl-3-(3-dimethylaminopropyl) carbodiimide hydrochloride (14.0 mg, 104 µmol), and triethylamine (90.0 µl, 644 µmol) thereto. The reaction solution was diluted with ethyl acetate, and washed with water and saturated brine. The organic layer was dried over anhydrous magnesium sulfate, and concentrated under reduced pressure. The thus obtained residue was purified by silica gel column chromatography (Yamazen, trade name: Hi-Flash Column, Silica gel, 40 µm, 60Å; n-heptane : ethyl acetate = 1:1, Rf = 0.55) to obtain the title compound (412 mg) as a yellow oil.

400MHz ¹H-NMR (CDCl₃) δ (ppm) 0.03 (s, 3H), 0.04 (s, 3H), 0.88 (s, 9H), 1.28 (s, 3H), 1.28-1.77 (m, 4H), 1.70 (s, 3H), 2.25-2.30 (m, 2H), 2.42 (dd, J = 4.6, 14.8 Hz, 1H), 3.94 (m, 1H), 4.24 (d, J = 6.8 Hz, 1H), 4.49 (dt, J = 7.2, 7.2 Hz, 1H), 4.79 (d, J = 6.8 Hz, 2H), 5.03-5.08 (m, 2H), 5.28 (d, J = 10.4 Hz, 1H), 5.40 (d, J = 17.2 Hz, 1H), 5.62-5.70 (m, 2H), 5.84 (m, 1H), 5.88 (s, 1H), 6.41 (d, J = 8.8 Hz, 1H), 7.34-7.36 (m, 3H), 7.39-7.43 (m, 2H), 7.46-7.48 (m, 2H), 7.52-7.56 (m, 1H), 8.09 (dd, J = 1.2, 7.2 Hz, 2H); MS m/z 670.41 (M + Na)⁺.

### Process (4-8): Synthesis of (2E)-3-[(2S, 3aS, 4E, 7S, 11R, 13aR)-11-{[tert-butyl(dimethyl)silyl]oxy}-13a-methyl-9-oxo-2-phenyl-3a,6,7,8,9,10,11,1 2,13,13a-decahydro[1,3]dioxolo[4,5-f]azacyclododecene-7-yl]-but-2-en-1-yl benzoate (P32)

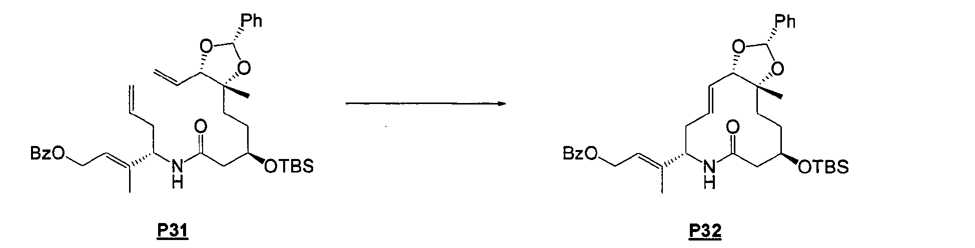

To a solution (1.03 L) of ((2E, 4S)-4-({(3R)-3-{[tert-butyl(dimethyl)silyl]oxy}-5-[(2S, 4R, 5S)-4-methyl-2-phenyl-5-vinyl-1,3-dioxolan-4-yl]pentanoy}amino)-3-methylhepta-2,6-dien-1-yl benzoate (333 mg, 514 µmol) in anhydrous dichloromethane, the second generation Grubbs' catalyst, that is, [1,3-bis-(2,4,6)trimethylphenyl-2-imidazolidinylidene]dichloro(phenylmethylene)-tricycl ohexylphosphine)ruthenium (87.2 mg, 103 µmol), was added under a nitrogen atmosphere. The reaction solution was heated to reflux for 9 and a half hours. The residue obtained by concentrating the reaction solution under reduced pressure was purified by silica gel column chromatography (Yamazen, trade name: Hi-Flash Column, Amino, 40 µm, 60Å; n-heptane : ethyl acetate = 1:1, Rf = 0.7) to obtain the title compound (135 mg) as a white amorphous.

400MHz ¹H-NMR (CDCl₃) δ (ppm) 0.10 (s, 6H), 0.89 (s, 9H), 1.41 (s, 3H), 1.47-1.82 (m, 3H), 1.82 (s, 3H), 2.01 (m, 1H), 2.35-2.45 (m, 2H), 2.50 (dd, J = 4.4, 14.4 Hz, 1H), 2.58 (brd, J = 13.2 Hz, 1H), 3.77 (m, 1H), 4.24 (d, J = 8.8 Hz, 1H), 4.39 (brt, J = 9.6 Hz, 1H), 4.88 (d, J = 6.8 Hz, 2H), 5.45 (brd, J = 9.2 Hz, 1H), 5.57-5.72 (m, 3H), 5.94 (s, 1H), 7.34-7.50 (m, 7H), 7.56 (m, 1H), 8.02-8.06 (m, 2H); MS m/z 642.40 (M + Na)⁺.

### Process (4-9): Synthesis of (2S, 3aS, 4E, 7S, 11R, 13aR)-11-{[(1,1-dimethylethyl)dimethylsilyl]oxy}-3a,7,8,10,11,12,13,13a-otcahydro-13a -methyl-7-[(1E)-1-methyl-1,3-butadienyl]-2-phenyl-1,3-dioxolo[4,5-f]azacyclododecene-9(6H)-one (P33)

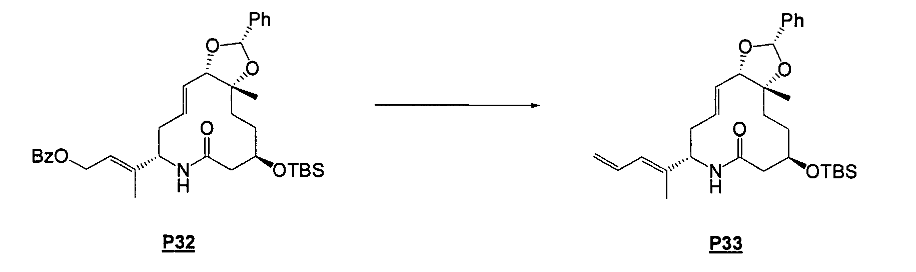

To a solution of (2E)-3-[(2S, 3aS, 4E, 7S, 11R, 13aR)-11-{[tert-butyl(dimethyl)silyl]oxy}-13a-methyl-9-oxo-2-phenyl-3a,6,7,8,9,10,11,1 2,13,13a-decahydro[1,3]dioxolo[4,5-f]azacyclododecene-7-yl]-but-2-en-1-yl benzoate (135 mg, 218µmol) in THF-methanol-water (2.0 ml, 2.0 ml, 1.0 ml, respectively), lithium hydroxide-monohydrate (27.4 mg, 654 µmol) was added. The mixture was stirred for 40 minutes at room temperature. The reaction solution was poured into water, and then extraction with ethyl acetate was performed. The obtained organic layer was washed with water and saturated brine, dried over anhydrous magnesium sulfate, and concentrated under reduced pressure.

The thus obtained residue was dissolved in dichloromethane (5.0 ml), manganese dioxide (1.10 g, 12.7 mmol) was added thereto. The mixture was stirred for 45 minutes at room temperature. The reaction solution was diluted with ethyl acetate, and filtered through celite. The filtrate was concentrated under reduced pressure.

The thus obtained residue was dissolved in anhydrous THF (1.0 ml), and added dropwise at -15°C to a reaction solution separately prepared by dropwisely adding a solution of 2.66M n-butyllithium in n-hexane (162 µl, 432 µmol) to a suspension (3.0 ml) of methyltriphenylphosphonium iodide (175 mg, 432 µmol) in anhydrous THF at 0°C under a nitrogen atmosphere and stirring at the same temperature for 10 minutes. After stirring this reaction solution at the same temperature for 10 minutes, saturated aqueous solution of ammonium chloride and ethyl acetate were added thereto. The organic layer was isolated, washed with water and saturated brine, dried over anhydrous magnesium sulfate, and concentrated under reduced pressure. The thus obtained residue was purified by silica gel column chromatography (Yamazen, trade name: Hi-Flash Column, Silica gel, 40 µm, 60Å; n-heptane : ethyl acetate = 1:1, Rf = 0.63) to obtain the title compound (15.0 mg) as a colorless oil.

400MHz ¹H-NMR (CDCl₃) δ (ppm) 0.10 (s,6H), 0.89 (s, 9H), 1.41(s, 3H), 1.43-1.82 (m, 3H), 1.81 (s, 3H), 2.02 (m, 1H), 2.29-2.57 (m, 4H), 3.75 (m, 1H), 4.24 (d, J = 9.2 Hz, 1H), 4.42 (brt, J = 10.2 Hz, 1H), 5.13 (d, J = 10 Hz, 2H), 5.22 (d, J = 17.2 Hz, 1H), 5.37 (brd, J = 8.0 Hz, 1H), 5.57-5.73 (m, 3H), 5.94 (s, 1H), 6.01 (d, J = 10.8 Hz, 1H), 6.56 (dt, J = 10.4, 16.8 Hz, 1H), 7.35-7.41 (m, 3H), 7.48-7.50 (m, 2H); MS m/z 534.33 (M + Na)⁺.

### Process (4-10): Synthesis of (4R, 7R, 8S, 9E, 12S)-4,7,8-trihydroxy-7-methyl-12-[(1E)-1-methylbuta-1,3-dien-1-yl]azacyclododec-9-e n-2-one (P34)

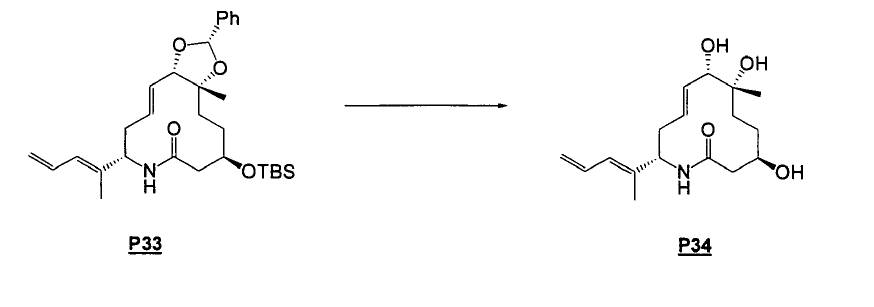

To a methanol solution (0.5 ml) of (2S, 3aS, 4E, 7S, 11R, 13aR)-11-{[(1,1-dimethylethyl)dimethylsilyl]oxy}-3a,7,8,10,11,12,13,13a-otcahydro-13a -methyl-7-[(1E)-1-methyl-1,3-butadienyl]-2-phenyl-1,3-dioxolo[4,5-f]azacyclododecene-9(6H)-one (15.0 mg, 29.3 µmol), pyridinium para-toluene sulfonate (29.5 mg, 117 µmol) was added at room temperature. The reaction solution was stirred for 4 days at the same temperature, diluted with ethyl acetate, and washed with saturated aqueous solution of sodium bicarbonate. The aqueous layer was further extracted with ethyl acetate. The combined organic layer was dried over anhydrous magnesium sulfate, and concentrated under reduced pressure. The thus obtained residue was purified by silica gel column chromatography (Yamazen, trade name: Hi-Flash Column, Silica gel, 40 µm, 60Å; methanol : ethyl acetate = 0:1 → 1:19 → 1:9) to obtain the title compound (4.2 mg) as a colorless oil. At the same time, (2S, 3aS, 4E, 7S, 11R, 13aR)-11-{[(1,1-dimethylethyl)dimethylsilyl]oxy}-3a,7,8,10,11,12,13,13a-otcahydro-13a -methyl-7-[(1E)-1-methyl-1,3-butadienyl]-2-phenyl-1,3-dioxolo[4,5-f]azacyclododecene-9(6H)-one (P33) (4.3 mg) was recovered.

MS m/z 332.20 (M + Na)⁺.

### Process (4-11): Synthesis of (2S, 4E, 6S, 7R, 10R)-7,10-dihydroxy-7-methyl-2-[(1E)-1-methylbuta-1,3-dien-1-yl]-12-oxoazacyclodod ec-4-en-6-yl acetate (P35)

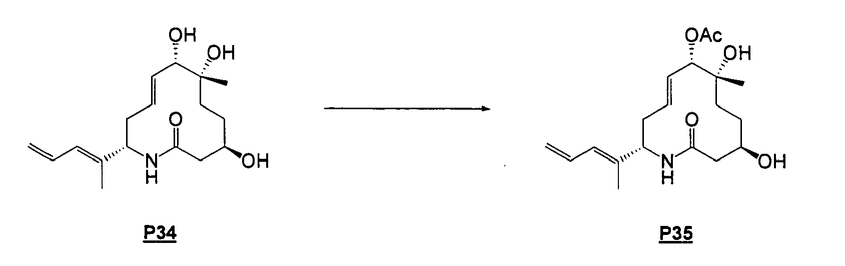

To a solution (2.0 ml) of (4R, 7R, 8S, 9E, 12S)-4,7,8-trihydroxy-7-methyl-12-[(1E)-1-methylbuta-1,3-dien-1-yl]azacyclododec-9-e n-2-one (4.2 mg, 13.6 µmol) in anhydrous dichloromethane, acetic anhydride (1.29 µml, 13.6 µmol), triethylamine (3.79 µl, 27.2 µmol) and 4-dimethylaminopyridine (0.17 mg, 1.4 µmol) were added. The reaction solution was stirred for 1 hour at room temperature, and further stirred for 1 hour after adding acetic anhydride (0.6 µml, 6.33 µmol) thereto. The reaction solution was diluted with ethyl acetate, washed with water and saturated brine, and dried over anhydrous magnesium sulfate. The organic layer was concentrated under reduced pressure. The thus obtained residue was purified by thin-layer chromatography (Merck, trade name: PLC plate, Silica Gel 60 F₂₅₄, 0.5 mm; methanol : ethyl acetate = 1:9) to obtain the title compound (4.8 mg) as a colorless oil.

MS m/z 374.50 (M + Na)⁺.

### Process (4-12): Synthesis of (1R)-4-C-{(1E, 3E)-4-[(2S, 4E, 6S, 7R, 10R)-6-(acetyloxy)-7,10-dihydroxy-7-methyl-12-oxoazacyclododec-4-en-2-yl]penta-1,3-dien-1-yl}-1,2-anhydro-3,5-dideoxy-1-[(1R, 2S)-2-hydroxy-1-methylbutyl]-D-erythro-pentitol (P36)

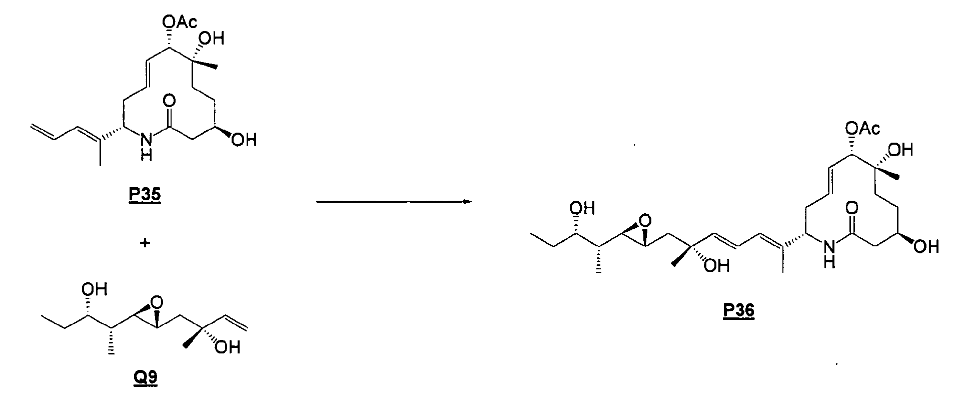

To a solution (2.0 ml) of (2S, 4E, 6S, 7R, 10R)-7,10-dihydroxy-7-methyl-2-[(1E)-1-methylbuta-1,3-dien-1-yl]-12-oxoazacyclodod ec-4-en-6-yl acetate (4.8 mg, 13.6 µmol) in anhydrous dichloromethane, the second generation Grubbs' catalyst, that is, (1R)-1,2-anhydro-3,5-dideoxy-1-[(1R,2S)-2-hydroxy-1-methylbutyl]-4-C-vinyl-D-erythr o-pentitol (14.6 mg, 68.0 mmol), was added under a nitrogen atmosphere. The mixture was heated to reflux for 1 and a half hours. After adding the second generation Grubbs' catalyst thereto (1.2 mg, 1.40 µmol) and stirring the mixture for 1 hour, the reaction solution was filtered by silica gel (Fuji Silysica, trade name: Chromatorex, NH, 200-350 mesh), and then the filtrate was concentrated under reduced pressure. The thus obtained residue was purified by thin-layer chromatography (Merck, trade name: PLC plate, Silica Gel 60 F₂₅₄, 0.5 mm; methanol : ethyl acetate = 1:9) to obtain the title compound (1.5 mg) as a colorless oil.

400MHz ¹H-NMR (CD₃OD) δ (ppm) 0.95 (d, J = 7.2 Hz, 3H), 0.99 (t, J = 7.4 Hz, 3H), 1.24 (s, 3H), 1.28-1.62 (m, 7H), 1.34 (s, 3H), 1.74 (dd, J = 6.0, 14.0 Hz, 1H), 1.85 (s, 3H), 1.86 (dd, J = 6.0, 14.0 Hz, 1H), 2.09 (s, 3H), 2.17 (dd, J = 12.6, 23.8 Hz, 1H), 2.49-2.59 (m, 3H), 2.72 (m, 1H), 2.93 (m, 1H), 3.57 (dt, J = 4.4, 8.0 Hz, 1H), 3.75 (m, 1H), 4.64 (m, 1H), 5.10 (d, J = 9.2 Hz, 1H), 5.66-5.81 (m, 2H), 5.85 (d, J = 15.2 Hz, 1H), 6.08 (d, J = 10.8Hz, 1H), 6.56 (dd, J = 10.6, 15.4 Hz,1H); MS m/z 560.30 (M + Na)⁺.

### Test Example 1

Proliferation inhibitory effect of (2S, 3S, 4E)-2-((1E, 3E, 5R)-5-hydroxy-6-{(2R, 3R)-3-[(1R,2S)-2-hydroxy-1-methylbutyl]oxiran-2-yl}-1,5-dimethylhexa-1,3-dien-1-yl)-3-methyl-12-oxaazacyclododec-4-en-6-yl acetate (P15) against WiDr human colon cancer cell and the like.

WiDr human colon cancer cells cultured in RPMI1640 medium containing 10% fetal bovine serum, penicillin (100 unit/ml) and streptomycin (100 µg/ml) (SIGMA product) were seeded into a 96-well plate by 2×10³ cells/90 µl/well. After culturing the cells overnight in a CO₂ incubator, 10 µl of the compound (P15) which is diluted in series in 3-folds was added in each well above, and cultured for another 3 days. Then, 50 µl of Cell Titer-Glo Luminescent Cell Viability Assay (Promega product) was added thereto, cells were shaken for 2 minutes and allowed to stand for 15 minutes to allow reaction. After completing the reaction, luciferase activity was measured using the luminometer. The thus obtained measurement value was given as an index for the number of living cells in each well.

On the basis of these results, the concentration (IC50 value) of the compound (P15) for 50% inhibition of WiDr human colon cancer cell growth was determined. The IC50 value was 0.57 µM, and it was found that the compound (P15) has the proliferation inhibitory effect against WiDr human colon cancer cell.

The same experiment as above was carried out, except that WiDr human colon cancer cell was changed to U937 human leukemia cell, KP4 human pancreatic cancer cell, MIApaca2 human pancreatic cancer cell, MDA-MB231 human breast cancer cell, MDA-MB435 human breast cancer cell, DU145 human prostate cancer cell, PC9 human lung cancer cell and OVCAR3 human ovarian cancer, respectively. The concentration (IC50 values) of the compound (P15) required for 50% inhibition of each cancer cell was determined. The results are shown in Table 3. It was found that the compound (P15) has the proliferation inhibitory effect against the above-described cancer cells.

### Test Example 2

Proliferation inhibitory effect of (2S, 3S, 4E)-2-((1E, 3E, 5S)-6-{(2R, 3R)-3-[(1R,2S)-2-hydroxy-1-methylbutyl]oxiran-2-yl}-1,5-dimethylhexa-1,3-dien-1-yl)-3-methyl-12-oxaazacyclododec-4-en-6-yl acetate (P16) against WiDr human colon cancer cell and the like.

The same experiment as in Test Example 1 was carried out, except that the compound (P15) was changed to the compound (P16). The concentration (IC50 value) of the compound (P16) required for 50% inhibition of WiDr human colon cancer cell growth was determined. The IC50 value was 0.29 µM, and it was found that the compound (P16) has the proliferation inhibitory effect against WiDr human colon cancer cell.

The same experiment as above was carried out, except that WiDr human colon cancer cell was changed to U937 human leukemia cell, KP4 human pancreatic cancer cell, MIApaca2 human pancreatic cancer cell, MDA-MB231 human breast cancer cell, MDA-MB4365 human breast cancer cell, DU145 human prostate cancer cell, PC9 human lung cancer cell and OVCAR3 human ovarian cancer, respectively. The concentrations (IC50 values) of the compound (P16) required for 50% inhibition of each cancer cell was determined. The results are shown in Table 3. It was found that the compound (P16) has the proliferation inhibitory effect against the above-described cancer cells.

**TABLE 3**

| | | IC50 (µM) | |
|---|---|---|---|
| Test Examples | | Test Example 1 | Test Example 2 |
| Compounds | | P15 | P16 |
| Human colon cancer cell | WiDr | 0.57 | 0.29 |
| Human leukemia cell | U937 | 0.55 | 0.14 |
| Human pancreatic cancer cell | KP4 | 1.3 | 0.30 |
| Human pancreatic cancer cell | MIApaca2 | 0.50 | 0.14 |
| Human breast cancer cell | MDA-MB231 | 0.47 | 0.13 |
| Human breast cancer cell | MDA-MB435 | 1.7 | 0.38 |
| Human prostate cancer cell | DU145 | 2.3 | 0.51 |
| Human lung cancer cell | PC9 | 1.4 | 0.24 |
| Human ovarian cancer | OVCAR3 | 0.43 | 0.097 |

## Claims

1. A compound represented by the following Formula (1) or a salt thereof: 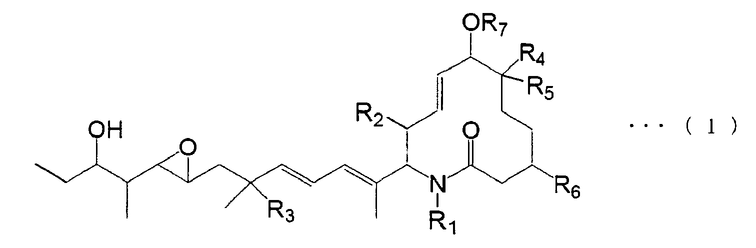 wherein R₁ is a hydrogen atom, a C₁₋₆ alkyl group, a C₁₋₆ alkylcarbonyl group or a C₆₋₁₄ arylcarbonyl group; R₂ is a hydrogen atom or a C₁₋₆ alkyl group; R₃ is a hydrogen atom or a hydroxyl group; R₄ is a hydrogen atom or a hydroxyl group; R₅ is a hydrogen atom or a C₁₋₆ alkyl group; R₆ is a hydrogen atom or a hydroxyl group; and R₇ is R^{a}C(=Y)-
wherein Y is an oxygen atom or a sulfur atom, and R^{a} is:
a) a C₁₋₂₂ alkyl group optionally having a substituent(s);
b) an unsaturated C₂₋₂₂ alkyl group optionally having a substituent(s);
c) a C₆₋₁₄ aryl group optionally having a substituent(s);
d) a 5 to 14-membered ring heteroaryl group optionally having a substituent(s);
e) a C₇₋₂₂ aralkyl group optionally having a substituent(s);
f) a 5 to 14-membered ring heteroaralkyl group optionally having a substituent(s);
g) a C₁₋₂₂ alkoxy group optionally having a substituent(s);
h) an unsaturated C₂₋₂₂ alkoxy group optionally having a substituent(s);
i) a C₆₋₁₄ aryloxy group optionally having a substituent(s);
j) a 5 to 14-membered ring heteroaryloxy group optionally having a substituent(s); or
k) R^{N1}R^{N2}N- optionally having a substituent(s), wherein R^{N1} and R^{N2} may be the same or different from each other and are each:
1) a hydrogen atom;
2) a C₁₋₂₂ alkyl group optionally having a substituent(s);
3) an unsaturated C₂₋₂₂ alkyl group optionally having a substituent(s);
4) an aliphatic C₂₋₂₂ acyl group optionally having a substituent(s);
5) an aromatic C₇₋₁₅ acyl group optionally having a substituent(s);
6) a C₆₋₁₄ aryl group optionally having a substituent(s);
7) a 5 to 14-membered ring heteroaryl group optionally having a substituent(s);
8) a C₇₋₂₂ aralkyl group optionally having a substituent(s);
9) a 3 to 14-membered ring non-aromatic heterocyclic group formed by R^{N1} and R^{N2} and the nitrogen atom to which R^{N1} and R^{N2} are bonded, wherein the non-aromatic heterocyclic group optionally has a substituent(s);
10) a 5 to 14-membered ring heteroaralkyl group optionally having a substituent(s);
11) a C₃₋₁₄ cycloalkyl group optionally having a substituent(s); or
12) a 3 to 14-membered ring non-aromatic heterocyclic group optionally having a substituent(s).

2. A compound represented by the following Formula (2) or a salt thereof: 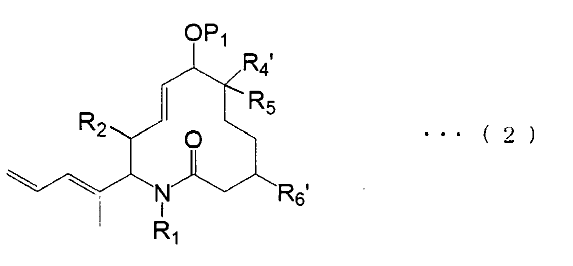 wherein R₁ is a hydrogen atom, a C₁₋₆ alkyl group, a C₁₋₆ alkylcarbonyl group or a C₆₋₁₄ arylcarbonyl group; R₂ is a hydrogen atom or a C₁₋₆ alkyl group; R₄' is a hydrogen atom, a hydroxyl group or an O-protecting group; R₅ is a hydrogen atom or a C₁₋₆ alkyl group; R₆' is a hydrogen atom, a hydroxyl group or an O-protecting group; and P₁ is a hydrogen atom or a protecting group: or R₄' and OP₁ may together represent the following formula: 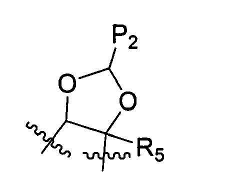 wherein P₂ is a phenyl group or a C₁₋₆ alkyl group.

3. A compound represented by the following Formula (3) or a salt thereof: 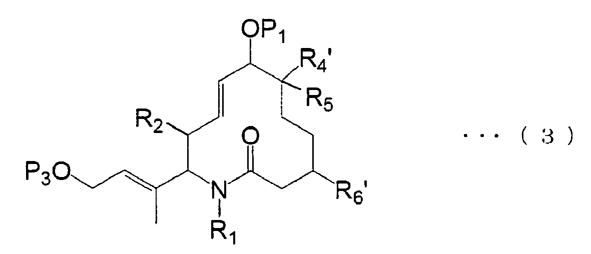 wherein R₁ is a hydrogen atom, a C₁₋₆ alkyl group, a C₁₋₆ alkylcarbonyl group, or a C₆₋₁₄ arylcarbonyl group; R₂ is a hydrogen atom or a C₁₋₆ alkyl group; R₄' is a hydrogen atom, a hydroxyl group, or an O-protecting group; R₅ is a hydrogen atom or a C₁₋₆ alkyl group; R₆' is a hydrogen atom, a hydroxyl group, or an O-protecting group; P₁ is a hydrogen atom or a protecting group; and P₃ is a hydrogen atom or a protecting group: or R₄' and OP₁ may together represent the following formula: 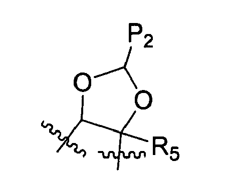 wherein P₂ is a phenyl group or a C₁₋₆ alkyl group.

4. A compound represented by the following Formula (4-1) or a salt thereof: 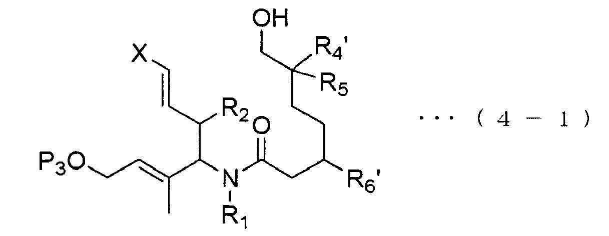 wherein R₁ is a hydrogen atom, a C₁₋₆ alkyl group, a C₁₋₆ alkylcarbonyl group or a C₆₋₁₄ arylcarbonyl group; R₂ is a hydrogen atom or a C₁₋₆ alkyl group; R₄' is a hydrogen atom, a hydroxyl group or an O-protecting group; R₅ is a hydrogen atom or a C₁₋₆ alkyl group; R₆' is a hydrogen atom, a hydroxyl group or an O-protecting group; P₃ is a hydrogen atom or a protecting group; and X is halogen.

5. A compound represented by the following Formula (4-2) or a salt thereof: 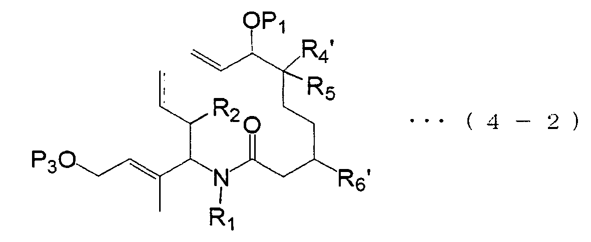 wherein R₁ is a hydrogen atom, a C₁₋₆ alkyl group, a C₁₋₆ alkylcarbonyl group or a C₆₋₁₄ arylcarbonyl group; R₂ is a hydrogen atom or a C₁₋₆ alkyl group; R₄' is a hydrogen atom, a hydroxyl group or an O-protecting group; a R₅ is a hydrogen atom or a C₁₋₆ alkyl group; R₆' is a hydrogen atom, a hydroxyl group or an O-protecting group; P₁ is a hydrogen atom or a protecting group; and P₃ is a hydrogen atom or a protecting group: or R₄' and OP₁ may together represent the following formula: 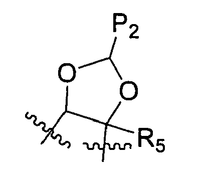 wherein P₂ is a phenyl group or a C₁₋₆ alkyl group.

6. A medicament comprising at least one compound selected from a compound according to claim 1 or a salt thereof as an active component.

7. The medicament according to Claim 6, which is an antitumor agent.

8. The medicament according to Claim 7, which is an agent for treating solid tumor.

9. The medicament according to Claim 8, wherein the agent for treating solid tumor is an agent for treating a lung cancer, a brain tumor, a breast cancer, a prostate cancer, an ovarian cancer, a colon cancer or a skin cancer.

10. The medicament according to Claim 7, which is an agent for treating leukemia.

11. A use of the compound according to claim 1 or salts, thereof, for production of a medicament.

12. The use according to Caim 11, wherein the medicament is an antitumor agent.

13. The use according to Caim 12, wherein the antitumor agent is an agent for treating solid tumor.

14. The use according to Caim 13, wherein the agent for treating solid tumor is an agent for treating lung cancer, brain tumor, breast cancer, prostate cancer, ovarian cancer, colon cancer or skin cancer.

15. The use according to Caim 14, wherein the medicament is an agent for treating leukemia.

16. The compound according to Caim1 or a salt thereof, for treating a tumor.

17. The compound according to Claim 16 or a salt thereof, wherein the tumor is a solid tumor.

18. The compound according to Claim 17 or a salt thereof, wherein the solid tumor is lung cancer, brain tumor, breast cancer, prostate cancer, ovarian cancer, colon cancer or skin cancer.

19. The compound according to Claim 16 or a salt thereof, wherein the tumor is leukemia.
